# EUROPEAN PATENT APPLICATION

(11) **EP 3 473 232 A1**
(43) Date of publication of application: **24.04.2019**
(21) Application number: 17813262.7
(22) Date of filing: 12.06.2017
(51) Int. Cl.: A61J 3/00, B65B 1/30

(54) **INSPECTION ASSISTANCE SYSTEM AND DRUG DISPENSER**

(30) Priority: 17.06.2016 JP 2016121290
(71) Applicant: Yuyama Mfg. Co., Ltd., Toyonaka-shi Osaka 561-0841 (JP)
(72) Inventor: KOIKE, Naoki, Toyonaka-shi Osaka 561-0841 (JP); TANAKA, Toru, Toyonaka-shi Osaka 561-0841 (JP)
(74) Representative: Patentanwälte Bauer Vorberg Kayser
(86) International application number: PCT/JP2017/021645
(87) International publication number: WO 2017/217366

(57) **Abstract**

[Problem] To provide a judgement supporting system being able to support the judgement work of the pharmacist who judges the results of the package processing and a medicine dispensing apparatus.

[Solution Means] A judgement supporting system 1 comprises a packaging unit 504 performing a package processing for packaging one or a plurality of tablets in a wrapping material for every administration timing based on formulation data; and a shifted-back detection part 510 for determining occasion of a shifted-back defect when at least a part of the tablets to be charged in a first wrapping material in the package processings is wrapped in a second wrapping material being next to the first wrapping material.

## Description

### FIELD OF INVENTION

The present invention relates to a medicine dispensing apparatus for performing a package processing with packaging a medicine every administration timing and a judgement supporting system being able to judge whether or not results of the package processing are proper.

### BACKGROUND

Popularly, a medicine dispensing apparatus, which comprises a plurality of medicine cassettes in which various medicines are contained and dispenses medicines from each medicine cassette based on formulation data while being capable of packaging the medicines every administration timing, is known (for example, refer to Patent Literature 1).

### PRIOR ART LITERATURE

### PATENT LITERATURE

Patent Literature 1: Japan Patent (Laid-Open) Publication No. 2011-104077

### SUMMARY OF INVENTION

### PROBLEM TO BE SOLVED BY INVENTION

Here, in a medical institute such as a hospital or a pharmacy, judgement work for confirming is performed by a pharmacist whether or not results of a package processing by a medicine dispensing apparatus is proper corresponding to formulation data.

An object of the present invention is to provide a judgement supporting system being able to support the judgement work of the pharmacist who judges the results of the package processing and a medicine dispensing apparatus.

### MEANS FOR SOLVING PROBLEM

A judgment supporting system of the present invention comprises a packaging unit performing a package processing for packaging one or a plurality of tablets in a wrapping material for every administration timing based on formulation data; and a shifted-back detection part for determining occasion of a shifted-back defect in a case that at least a part of the tablets to be charged in a first wrapping material in the package processing is wrapped in a second wrapping material being next to the first wrapping material.

The judgement supporting system may further comprises an operation display processing part displaying a first re-execution operation part for receiving an operation to re-execute the package processing with respect to at least one of the first wrapping material and the second wrapping material determined that the shifted-back detect has been occurred by the shifted-back detection part; and a re-execution part executing the package processing with respect to at least one of the first wrapping material and the second wrapping material when the first re-execution operation part is operated.

Here, the first re-execution part may receive an operation for executing the package processing with respect to the first wrapping material and the second wrapping material determined that the shifted-back detect has been occurred by the shifted-back detection part. In addition, the re-execution processing part may execute the package processing with respect to the first wrapping material and the second wrapping material when the re-execution operation part is operated.

Besides, the judgement supporting system may further comprises a judgement processing part for determining propriety of a result of the package processing based on the formulation data. Here, the operation display processing part displays the first re-execution operation part and a second re-execution operation part receiving an operation for executing the package processing with respect to a non-proper wrapping material of which result by the judgement processing part is not proper. In addition, the re-execution processing part executes the package processing with respect to the first wrapping material and the second wrapping material when the first re-execution operation part is operated and executes the package processing with respect to the non-proper wrapping material when the second operation part is operated.

Here, the judgement processing part may execute at least one of an image judgement processing for determining propriety of a result of the package processing based on identification information of the tablet included in a photographed image photographing the tablet; a shape judgement processing for determining propriety of a result of the package processing based on an appearance of the tablet included in a photographed image photographing the tablet; and a counting judgement processing or determining propriety of a result of the package processing based on packaging amounts of the tablets. In addition, the operation display processing part displays one or a plurality of the second re-execution operation part for receiving the operation to execute the package processing with respect to the non-proper wrapping material of which determination result in any one of the image judgement processing, the shape judgement processing and the counting judgement processing is not proper.

It is contemplated that a medicine dispensing apparatus comprises a medicine supply unit for dispensing one or a plurality of medicines based on formulation data; a packaging unit for performing a package processing to package the tablet dispensed by the medicine supply unit for every administration timing into a wrapping material; a printer unit for printing information on the wrapping material; and a controller part being able to execute with switching between a first printing mode and a second printing mode, the first printing mode printing the information on the wrapping material by the printer unit upon executing the package processing and the second printing mode printing the information on the wrapping material by the printer unit without accompanied with the dispensation of the medicine by the medicine supply unit to form the wrapping material in an empty state.

Here, it is contemplated that the controller part, when the first printing mode is selected and also when a start operation for packaging is done without input of medicine designation information for identifying a medicine acknowledges an error, and the controller part when the second printing mode is selected and even when a start operation for packaging is done without input of medicine designation information of identifying a medicine, does not acknowledge an error.

It is contemplated that a medicine dispensing apparatus od the present invention comprises a plurality of medicine cassette being able to dispense a predetermined medicine for every unit amount; a detection processing part being able to detect removal of each of the medicine cassettes; a specification processing part for specifying the medicine cassette corresponding to the subjected medicine when inputted information of a subjected medicine to be replenished to the medicine cassette; a determination processing part for determining whether or not the removed medicine cassette and the medicine cassette specified with the specification processing part is identical each other after inputting information of the subjected medicine and when detecting the removal of the medicine cassette by the detection processing part; and a report processing part for acknowledging a determination result by the determination processing part.

It is contemplated that a medicine dispensing apparatus comprises a plurality of medicine cassettes being able to dispense a predetermined medicine for every unit amount; a cassette lock part being able to lock removal of each of the medicine cassettes; a specification processing part for specifying the medicine cassette corresponding to the subjected medicine when inputted information of a subjected medicine to be replenished to the medicine cassette; and a lock processing part for locking the removal of the medicine cassette by the cassette lock part upon mounting the medicine cassette and for releasing the lock by the cassette lock part with respect to the medicine cassette specified by the specification processing part.

### ADVANTAGE OF INVENTION

According to the present invention, a judgement supporting system being able to support the judgement work of the pharmacist who judges the results of the package processing and a medicine dispensing apparatus may be provided.

### BRIEF DESCRIPTION OF INVENTION

Fig. is a drawing illustrating a construction of a judgement supporting system of an embodiment of the present invention.
Fig. 2 is a drawing for an appearance of a medicine dispensing apparatus of an embodiment of the present invention.
Fig. 3 is a drawing of a construction for a medicine dispensing apparatus of an embodiment of an embodiment of the present invention.
Fig. 4A is a schematic drawing for illustrating an inside construction of a medicine dispensing apparatus of an embodiment of the present invention.
Fig. 4B is a schematic drawing for illustrating a rotation unit of a medicine dispensing apparatus of an embodiment of the present invention.
Fig. 5 is a drawing of one example of a fixed cassette of a medicine dispensing apparatus of an embodiment of the present invention.
Fig. 6 is a drawing of one example of a variable cassette of a medicine dispensing apparatus of an embodiment of the present invention.
Fig. 7 is a drawing of one example of a variable cassette of a medicine dispensing apparatus of an embodiment of the present invention.
Fig. 8 is a drawing of one example of a variable cassette of a medicine dispensing apparatus of an embodiment of the present invention.
Fig. 9 is a drawing of one example of a mounting part of a variable cassette of a medicine dispensing apparatus of an embodiment of the present invention.
Fig. 10 is a drawing of one example of a packaging result in a medicine dispensing apparatus of an embodiment of the present invention.
Fig. 11 is a drawing of one example of allocation information used in a judgement supporting system of an embodiment of the present invention.
Fig. 12 is a drawing of one example of drive correspondence information used in a judgement supporting system of an embodiment of the present invention.
Fig. 13 is a drawing of one example of a rotation unit of a medicine dispensing apparatus of the present invention of an embodiment of the present invention.
Fig. 14 is a drawing of one example of a rotation unit of a medicine dispensing apparatus of the present invention of an embodiment of the present invention.
Fig. 15 is a flowchart of one example of a medicine dispensing processing executed in a medicine dispensing apparatus of an embodiment of the present invention.
Fig. 16 is a flowchart of one example of a judgement supporting processing executed in a judgement supporting system of an embodiment of the present invention.
Fig. 17 is a drawing of one example of a display screen displayed in a judgement supporting system of an embodiment of the present invention.
Fig. 18 is a drawing of one example of a display screen displayed in a judgement supporting system of an embodiment of the present invention.
Fig. 19 is a drawing of one example of a display screen displayed in a judgement supporting system of an embodiment of the present invention.
Fig. 20A is a drawing of one example of a display screen displayed in a judgement supporting system of an embodiment of the present invention.
Fig. 20B is a drawing of one example of a display screen displayed in a judgement supporting system of an embodiment of the present invention.
Fig. 21 is a drawing of one example of a display screen displayed in a judgement supporting system of an embodiment of the present invention.
Fig. 22A is a drawing of one example of a display screen displayed in a judgement supporting system of an embodiment of the present invention.
Fig. 22B is a drawing of one example of a display screen displayed in a judgement supporting system of an embodiment of the present invention.
Fig. 23 is a drawing of one example of a display screen displayed in a judgement supporting system of an embodiment of the present invention.
Fig. 24 is a flowchart of one example of a re-execution control processing executed in a judgement supporting system of an embodiment of the present invention.
Fig. 25 is a drawing for explaining a construction of a packaging unit of a medicine dispensing apparatus of an embodiment of the present invention.
Fig. 26 is a drawing for explaining a construction of a packaging unit of a medicine dispensing apparatus of an embodiment of the present invention.
Fig. 27 is a drawing for explaining a construction of a packaging unit of a medicine dispensing apparatus of an embodiment of the present invention.
Fig. 28 is a drawing for explaining a construction of a packaging unit of a medicine dispensing apparatus of an embodiment of the present invention.
Fig. 29 is a drawing for explaining a construction of a packaging unit of a medicine dispensing apparatus of an embodiment of the present invention.
Fig. 30 is a drawing for explaining a construction of a packaging unit of a medicine dispensing apparatus of an embodiment of the present invention.
Fig. 31 is a drawing for explaining a construction of a packaging unit of a medicine dispensing apparatus of an embodiment of the present invention.
Fig. 32 is a drawing for explaining a shifted-back defect to be occurred in a medicine dispensing apparatus of an embodiment of the present invention.
Fig. 33 is a drawing for explaining a shifted-back defect to be occurred in a medicine dispensing apparatus of an embodiment of the present invention.
Fig. 34 is a flowchart for explaining a method for forming a medicine package executed in a medicine dispensing apparatus of an embodiment of the present invention.
Fig. 35 is a flowchart for explaining a subroutine for forming a second vertical seal executed in a medicine dispensing apparatus of an embodiment of the present invention.
Fig. 36 is one example of a display screen displayed in a judgement supporting system of an embodiment of the present invention.
Fig. 37 is one example of a display screen displayed in a judgement supporting system of an embodiment of the present invention.
Fig. 38 is one example of a display screen displayed in a judgement supporting system of an embodiment of the present invention.
Fig. 39 is one example of a display screen displayed in a judgement supporting system of an embodiment of the present invention.
Fig. 40 is one example of a display screen displayed in a judgement supporting system of an embodiment of the present invention.
Fig. 41 is one example of a display screen displayed in a judgement supporting system of an embodiment of the present invention.
Fig. 42 is one example of an erroneous charging protection processing executed in a medicine dispensing apparatus of an embodiment of the present invention.
Fig. 43 is another example of an erroneous charging protection processing executed in a medicine dispensing apparatus of an embodiment of the present invention.

### MODE FOR PRACTICING INVENTION

Hereinafter, the present invention will be explained with referring to attached drawings provided for understanding the present invention. Here, an embodiment hereinbelow is one example by embodying the present invention and shall not have characteristics for limiting technical scope of the present invention. Besides, constructions and processing functions may be optionally combined while avoiding and/or selecting the constructions and the processing described in the following embodiments.

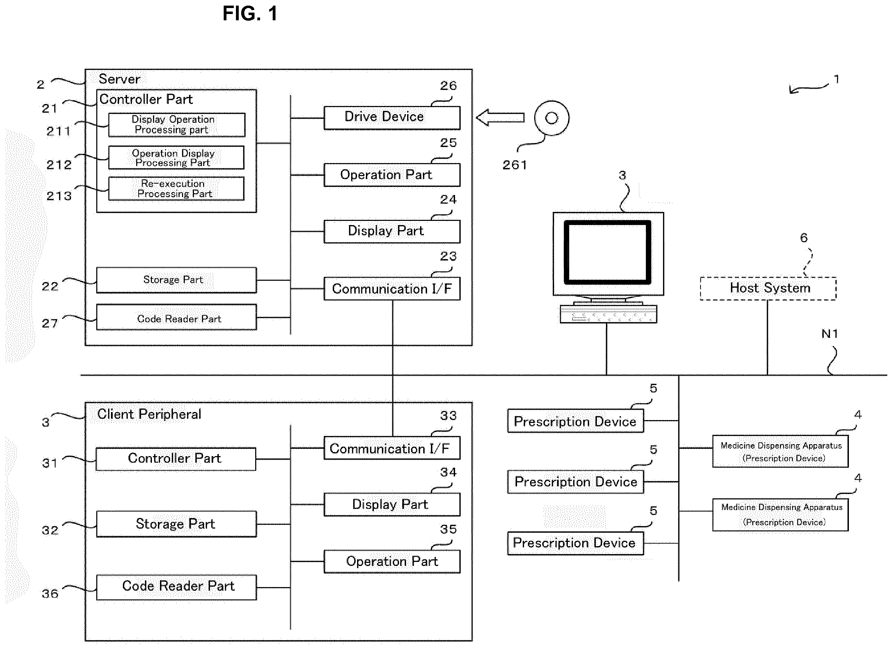

As illustrated in Fig. 1, a judgement supporting system 1 of an embodiment of the present invention comprises a judgement supporting apparatus 2, one or a plurality of client peripherals 3, one or a plurality of medicine dispensing apparatuses 4, and one or a plurality of prescription devices 5. Here, the medicine dispensing apparatus 4 alone may be understood as the judgement supporting system of the present invention.

The judgement supporting apparatus 2, the client peripherals 3, the medicine dispensing apparatus 4, and the prescription device 5 are each connected in wireless and/or wired communication network N1 such as LAN or INTERNET and the like. Besides, to the judgement supporting apparatus 2 a host system 6 such as an electronic clinical recording system or a prescription inputting peripheral is connected through the communication network N1. Now, it may be contemplated that the judgement supporting apparatus 2 may read the formulation data from a medical prescription or that the formulation data may be input by a user operation in the judgement supporting apparatus 2.

### [Judgement supporting apparatus 2]

The judgement supporting apparatus 2 is a personal computer comprising a controller part 21, a storage part 22, a communication I/F 23, and display part 24, an operation part 25, a drive device 26 and a code reader part 27 and the like. The judgement supporting apparatus 2 may be placed the inside or the outside of the medical institute where the judgement supporting system 1 is utilized.

The controller part 21 may comprises controlling devices such as a CPU, a ROM, a RAM and an EEPROM (Registered Trademark, the same shall be applied hereinafter.) and the like. The CPU is a processor for executing various computing processings. The ROM is a non-volatile storage part for storing beforehand information such as a control program and the like for making the CPU execute processings of various kinds. The RAM is a volatile storage part, and the EEPROM is a non-volatile storage part. The RAM and the EEPROM may be used as temporal storage memories (working region) for various processings executed by the CPU. Besides, the controller part 21, using the CPU, executes various processings according to the various control programs stored in the storage part 22 beforehand.

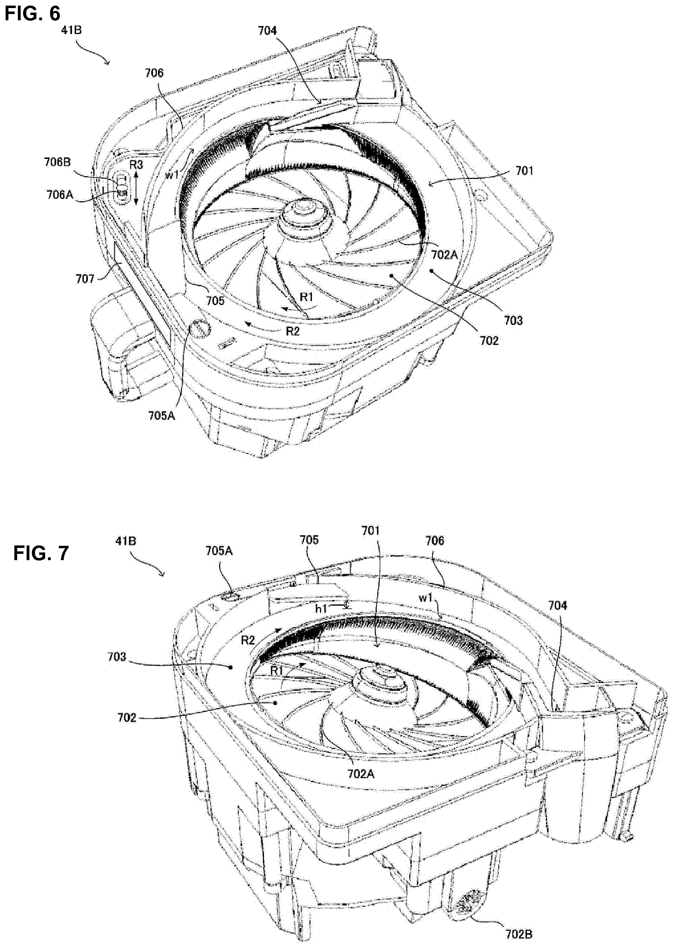

The storage part 22 is a storage part such as an HDD (Hard Disk Drive) and/or an SSD (Solid State Drive) storing various data. Particularly, in the storage part 22, a judgement supporting program, which makes the computer such as the controller part 21 and the like execute a judgement supporting processing described later (Fig. 7), is stored beforehand. In addition, in the storage part 22, various databases such as, for example, a medicament master, a patient master, and a user master and the like are also stored. Furthermore, in the storage part 22, a medicine database is stored separately from the medicament master.

In the medicament master, information relating to each of medicines may be included such as medicine IDs, medicine codes, medicine names, YJ codes, JAN codes (or RSS codes), medicine bottle codes, categories (dosage forms: powders, tablets, liquid agents, ointments and the like), shapes of tablets (capsules, spherical tablets, plane tablets (disk shaped tablets and the like), colors of tablets, specific gravities, families of medicines (common drugs, poisons, narcotic drugs, dangerous drugs, psychotropic drugs or therapeutic drugs and the like), formulation variations, diluted drugs, notice items, normal images of tablets (appearance images of front side and back side of tablets) and the like. For example, the normal image may be recorded by retrieving the normal image recorded beforehand in the medicine database described hereinafter and the like.

In the user master, information about users may be included such as pharmacy names, names of pharmacists, IDs of pharmacists, passwords, user groups and/or processing authorities may be included. In the patient master, information about the patients such as patient IDs, names, sexes, ages, medical histories, prescribed medicine histories, family information, diagnosis and treatment departments, hospital wards, and sickrooms and the like.

In the medicine database, information for every medicine is stored correspondingly such as medicine codes, medicine names, JAN codes, RSS codes, medicine bottle codes, medicine forms, units, specific gravities, medicine families, formulation variations, diluted medicines, notice items, allergy information and information for attachment documents. Particularly, in the medicine database, with respect to the tablets, information about an identification information formed to the tablet and a shape of the tablet are stored. The medicine database may be retrieved, for example, from a recording medium such as the CD and/or the DVD by the drive device 26, or may be received from an external apparatus through the communication network N1, and thereafter, may be stored in the data storage part 22. Besides, the medicine database may be used in the judgement supporting system 1 when the information is read in various masters such as the medicament master and the like or when the information of the attachment documents is referred and the like. Furthermore, the controller part 21 may be constructed so as to be able to retrieve the medicine database depending on its necessity from an external apparatus and/or a website through the communication networks N1. Here, the medicine database may also be used upon updating the medicament master and the like.

The communication I/F 23 is an interface including a networking card etc. for executing data communications through the communication network N1 between external devices such as the client peripherals 2, the medicine dispensing apparatus 4, and the prescription device 5 in accordance with predetermined communication protocols.

The display part 24 is a display part such as a liquid crystal monitor etc. for displaying various information and operation screens in accordance with control instructions from the controller part 21. The operation part 25 is the operation part such as a keyboard, a mouse, and a touch panel for receiving user operations and may input operation signals corresponding to the user operations to the controller part 21. The operation part 25 may receive various operation inputs such as a selection operation of the formulation data on a display screen displayed on the display part 24 and an issuing operation for the formulation data for requesting a prescription start of the formulation data.

The drive device 26 may read the judgement supporting program from a computer readable medium 261 in which the judgement supporting program is recorded. The recording medium 261 may be a CD, a DVD, a BD, or a USB memory and the like and the drive device 26 may be a CD drive, a DVD drive, a BD drive, or a USB port and the like. In the judgement supporting apparatus 2, by the controller part 21 using the drive device 26, the judgement supporting program read from the recording medium 261 is stored in the storage part 22.

The code reader part 27 is a barcode reader being able to read code information (barcode or two-dimensional code). For example, the code reader part 27 is used for retrieving the formulation data from the code information described in the medical prescription. The formulation data read from the medical prescription are stored in the storage part 22 by the controller part 21.

In the judgement supporting apparatus 2 constructed as described above, the controller part 21 comprises a display processing part 211, an operation display processing part 212, and a re-execution processing part 213. Particularly, the controller part 21 functions as the display processing part 211, the operation display processing part 212 and the re-execution processing part 213 by executing various processings according to the judgement supporting program. Here, the controlling part 21 also comprises a function for generating the formulation data (prescription data) for the prescription for allowing to perform the prescription processing such as the package processing of the medicine dispensing apparatus 4 and the prescription device 5 based on the formulation data and also inputting the formulation data to the medicine dispensing apparatus 4 and the prescription device 5. Thereby, in the medicine dispensing apparatus 4 and the prescription device 5, the prescription processing such as the package processing may be performed based on the formulation data.

The display processing part 211 displays on the client peripheral 3 and the like a photographed image of each of the tablets taken during the package processing in a packaging unit (one package unit) in which one or a plurality of tablets dispensed from any one or both of the medicine cassette 41 and a hand distribution unit 45 based on the formulation data in the medicine dispensing apparatus 4 are wrapped with a wrapping material such as a dispensing paper for every administration timing. Here, in the present embodiment, the administration timing may be used as the term including administration days and administration periods (after morning, after lunch, or after dinner and the le like); however, the administration timing of the present invention may merely mean the administration periods.

Besides, the photographed image may be taken before or after each of the tablets is wrapped by the dispensing paper in each of the package processing. Furthermore, the display processing part 211 displays on the client peripheral 3 judgement results of an automatic judgement based on the identification information (letters or characters) of the tablets included in the photographed image of the tablet and the formulation data and the like. More particularly, the display processing part 211 may display the photographed images together with the judgement results by the automatic judgement processing when the photographed image has been taken.

The operation display processing part 212 displays an operation part for re-executing individually a part or the the whole of the prescription processing based on the formulation data performed in the medicine dispensing apparatus 4 and the prescription device 5 on a screen on which the judgement results of the medicine prescribed in the medicine dispensing apparatus 4 and the prescription device 5 are displayed.

The re-execution processing part 213 makes the medicine dispensing apparatus 4 or the prescription device 5 re-execute a part or the whole of the prescription processing based on the formulation data performed in the medicine dispensing apparatus 4 and the prescription device 5. For example, the re-execution processing part 213 may generates re-execution data for executing a part or the whole of the prescription processing based on the formulation data and may send the re-execution data to the medicine dispensing apparatus 4 or the prescription device 5 together with a re-execution instruction. Besides, the re-execution data is the data for re-execution the prescription processing corresponding to one or plural administration timing.

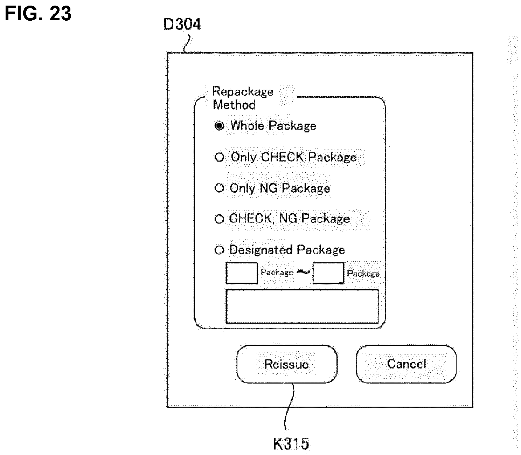

Particularly, according to the present embodiment, the operation display processing part 212 may be able to display a reissuing operation screen D304 (refer to Fig. 23) for re-execution of a part or the whole of the package processing performed in the medicine dispensing apparatus 4. Here, a part or the whole of the package processing means a package processing with respect to one or a plurality of administration timings. Furthermore, the re-execution processing part 213, in response to the user operation to the reissuing operation screen D304, may make the medicine dispensing apparatus 4 which performed the package processing or the medicine dispensing apparatus 4 different from the medicine dispensing apparatus 4 which performed a part or the whole of the package processing re-execute a part or the whole of the package processing performed in the medicine dispensing apparatus 4.

### [Client peripheral 3]

The client peripheral 3 is a personal computer comprising a controller part 31, a storage part 32, a communication I/F 33, a display part 34, an operation part 35 and a code reader part 36 and the like. The client peripheral 3 is an operation peripheral each of which is placed at the medical institutes where the judgement supporting system 1 is utilized and is operated by a user such as a pharmacist.

The controller part 31 comprises controller devices such as a CPU, a ROM, a RAM and an EEPROM and the like. The CPU is a processor for executing various computing processing. The ROM is a non-volatile storage part for storing beforehand information such as a control program and the like for making the CPU execute processing of various kinds. The RAM is a volatile storage part, and the EEPROM is a non-volatile storage part. The RAM and the EEPROM may be used as temporal storage memories (working region) for various processing executed by the CPU. Besides, the controller part 31, using the CPU, executes various processing according to the various control programs stored in the storage part 22 beforehand.

The storage part 22 is a storage part such as an HDD (Hard Disk Drive) and/or an SSD (Solid State Drive) storing various application programs executed by the controller part 31 and various data Particularly, in the storage part 32, various application programs such as an operating system (OS) and a browser software may be stored. The browser software is an application software which may make the display part 34 display various operation screens by accessing to the judgement supporting apparatus 2 through the communication network N1 and may transfer input operations to the operation screens using the operation part 35 to the judgement supporting apparatus 2. Particularly, when address information such as a URL (Universal Resource Locator) corresponding to the judgement supporting apparatus 2 is input to a predetermined position of the operation screen displayed by the browser software, the controller part 31 may accesses to the judgement supporting apparatus 2 based on the address information.

The communication I/F 33 is an interface including a networking card etc. for executing data communications through the communication network N1 between external devices such as the client peripherals 2, the medicine dispensing apparatus 4, and the prescription device 5 in accordance with predetermined communication protocols.

The display part 34 is a display part such as a liquid crystal monitor or an organic EL display and the like for displaying various information in accordance with control instructions from the controller part 31. The operation part 35 is the operation part operated by a user for inputting various information to the client peripheral 3. Particularly, the operation part 35 may comprises a keyboard, a mouse (pointing device) and a touch panel and the like for receiving the input operation to various operation screen displayed on the display part 34.

The code reader part 36 is a barcode reader being able to read the code information (barcode or two-dimensional code). For example, the code reader part 36 is used for retrieving medicine data from the code information printed on a medicine bottle or a medicine box. Furthermore, the code reader part 36 may be used to read formulation identification information such as formulation ID etc. for identifying the formulation data from the code information printed on a medicine package 451 described later.

Besides, in the judgement system 1, a server-client system is constructed by the judgement supporting apparatus 2 and the client peripheral 3 and the case will be explained in that the judgement supporting apparatus 2 performs various processings in response to the user operation at the client peripheral 3. For example, the control part 21 of the judgement supporting apparatus 2 makes the display part 31 of the client peripheral 3 display various screens by sending data described in a page description language such as HTML to the client peripheral 3. Besides, the controller part 31 of the client peripheral 3 sends operation signals to the judgement supporting system 2 depending on the operation input to the operation part 35.

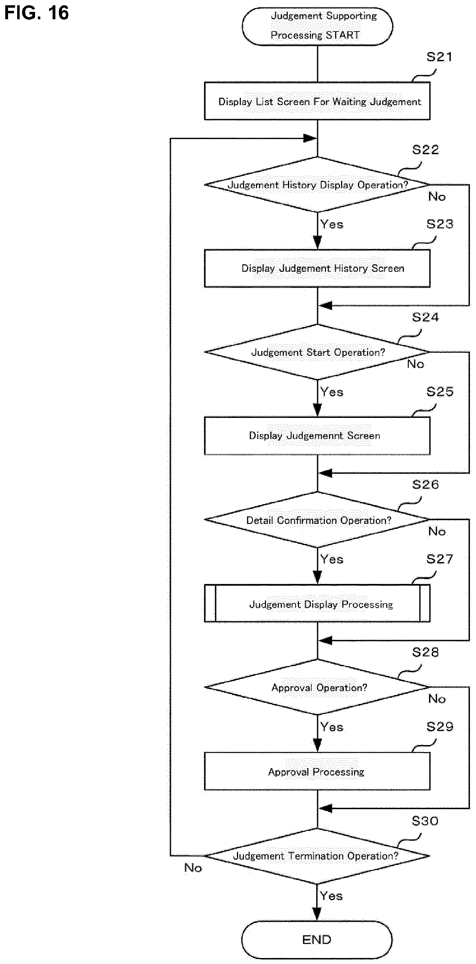

Now, a part or the whole of the judgement supporting program is installed in any one or a plurality of the judgement supporting apparatus 2, the client peripheral 3, and the medicine dispensing device 4 and it is contemplated that judgement supporting processings described later (refer to Fig. 16) are cooperatively performed by the judgement supporting apparatus 2, the client peripheral 3, and the medicine dispensing device 4 and the like.

### [Prescription device 5]

The prescription device 5 is a device used upon prescribing the medicine based on the formulation data. To the prescription device 5, for example, a powder packaging apparatus, a liquid agent distributing apparatus, a sheet dispensing apparatus, and a picking assistance apparatus and the like may be included as well as a tablet packaging apparatus for packaging the tablets as the medicine dispensing device 4. The powder packaging apparatus comprises a plurality of powder cassettes containing a plurality of kinds of powders and may dispense the powder contained in the powder cassette automatically for every predetermined amount. Besides, the liquid agent distributing apparatus comprises a plurality of medicine bottles each of which a plurality of kinds of liquid agent is reserved and may dispense the liquid agent of a required amount from the medicine bottle according to the formulation data. The sheet dispensing apparatus dispenses from a plurality of sheet cassettes each reserving PTP sheets or a heat seal wrapping the tablets beforehand. The picking assistance apparatus is one that is used when a pharmacist prescribes manually and reads the medicine name from the identification information (barcodes) attached to a medicine shelf or a medicine bottle and that performs verification of the read medicine name with the medicine name included in the formulation data.

### [Medicine dispensing apparatus 4]

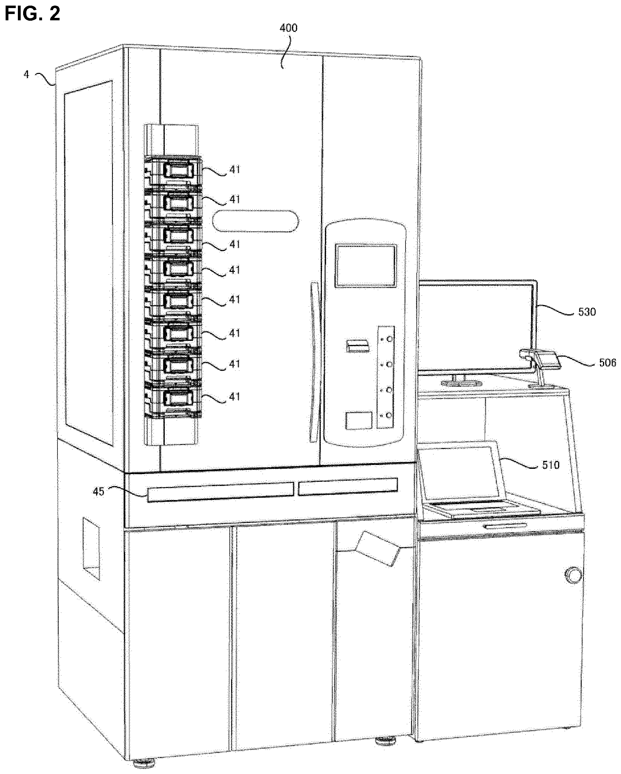

Now, with referring to Fig. 2-Fig. 15, the medicine dispensing apparatus 4 will be explained.

As shown in Fig. 2, Fig. 3 and Fig. 4A, the medicine dispensing apparatus 4 comprises a formulation control unit 501, a tablet supply unit 502 (one example of a medicine supply unit) and a packaging unit 504, a packaging control unit 505, and a barcode reader 506 and the like. The medicine dispensing apparatus 4 is a prescribing device used for the prescription of the medicine. Here, long dashed and short dashed line illustrates a transferring path of the tablet.

The formulation unit 501, the tablet supply unit 502, the packaging unit 504, and the packaging control unit 505 are connected by an internal bus N2. The formulation control unit 501 and the barcode reader 506 may perform wireless communications according to a communication regulation such as a wireless LAN or Bluetooth (Registered Trademark) and the like. Besides, the medicine dispensing apparatus 4 is controlled by the formulation control unit 501 and the packaging control unit 505 to dispense the tablet supplied from the tablet supply unit 502 with dispensing the packaging unit 504 in the packaging unit such as the administration period and the like.

### [Formulation control unit 501]

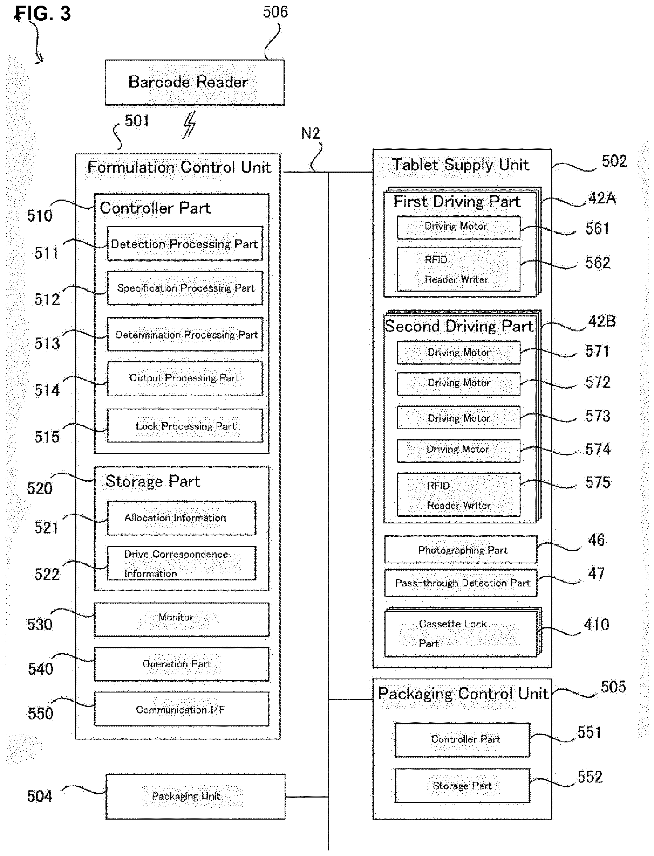

The formulation control unit 501 is a computer for totally controlling the medicine dispensing apparatus 4. As shown in Fig. 2 and Fig. 3, the formulation control unit 501 comprises a controller part 510, a storage part 520, a monitor 530, an operation part 540, and a communication IF 550 and the like.

The controller part 510 is a control means comprising a CPU, a RAM, a ROM, and an EEPROM. The controller part 510 executes various processing by the CPU according to various programs stored beforehand in a storage means such as the ROM, the EEPROM, and the storage part 520, Here, the CPU is a processor for executing various processing and the RAM and the EEPROM may be used as temporal storage memories (working area) for the processing executed by the CPU. Here, the controller part 510 may be an integrated circuit such as ASIC or DSP.

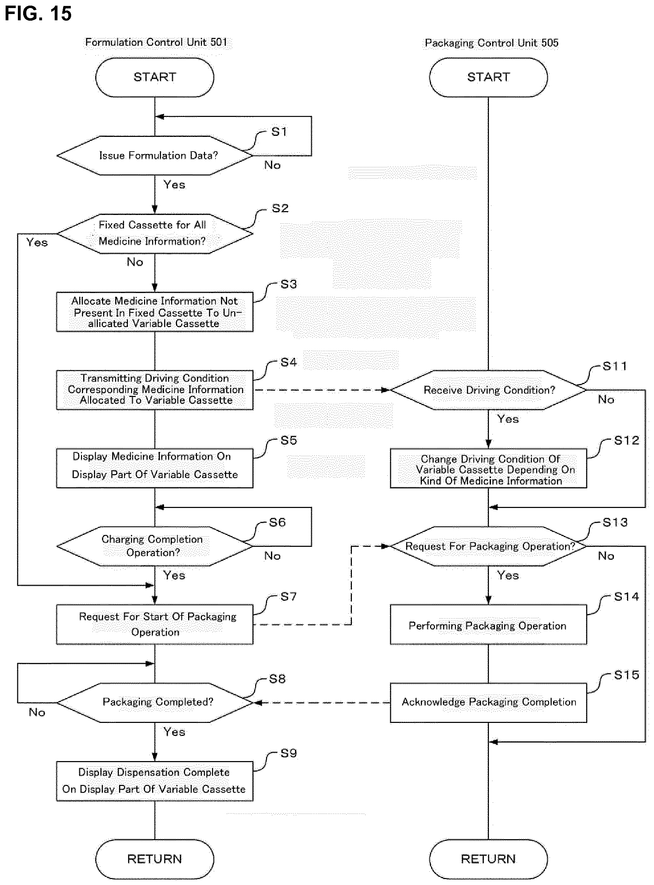

The storage part 520 is is a storage part such as an HDD (Hard Disk Drive) and/or an SSD (Solid State Drive) storing various data. Particularly, in the storage part 520, the medicine dispensing program for making the computer such as the controller part 510 execute medicine dispensing processing described later (refer to Fig. 15) is stored.

Besides, the medicine dispensing program is stored in, for example, a computer readable recording medium such as a CD, a DVD, and a semiconductor memory, and is installed by retrieved from the recording medium by a reader device such as a disc drive not shown in the figure. The present invention may be understood as the invention for the computer readable recording medium in which the medicine dispensing program is recorded.

Furthermore, in the storage part 520, for example, various databases are stored such as the medicament master, the patient master, the cassette master, and the pharmacy master and the like. Here, the controller part 510 may update the various database stored in the storage part 520 based on the read data from the CD, the DVD, or the semiconductor memory and the like with the reader device not shown in the figure. Furthermore, the controller part 510 may also change contents of the various database depending on the user operation to the operation part 540.

In the medicament master, information relating to each of medicines may be included such as medicine IDs, medicine codes, medicine names, YJ codes, JAN codes (or RSS codes), medicine bottle codes, categories (dosage forms: powders, tablets, liquid agents, ointments, and the like), sizes of the tablets (height and width), specific gravities, families of medicines (common drugs, poisons, narcotic drugs, dangerous drugs, psychotropic drugs or therapeutic drugs and the like), formulation variations, diluted drugs, notice items, a normal image of tablets (appearance images of front side and back side of tablets) and the like. In the patient master, information about the patients may be included such as patient IDs, names, sexualities, ages, medical histories, prescribed medicine histories, family information, diagnosis and treatment departments, hospital wards, and sickrooms, and the like. In the pharmacy master, information about the pharmacy such as pharmacy names, names of pharmacists, and IDs of pharmacists may be included.

Furthermore, the cassette master is information indicating corresponding relations of the cassette identification information for each of the fixed cassettes 41A and the medicine information allocated to each of the fixed cassettes 41A. The cassette master may be registered by the controller part 510 depending on the user operation to the operation part 540, for example, at an initial setting of the medicine dispensing apparatus 4.

The monitor 530 is a display means for displaying various information and operation screens according to instructions from the controller part 510. For example, in the monitor 530, the various information such as an input screen of the formulation data and a selection screen of the formulation data may be displayed.

The operation part 504 is an operation means such as an operation button, a keyboard, a mouse and a touch panel and the like and allows to input operation signals corresponding to the user operation to the controller part 510. The operation part 540 receives various inputs such as, for example, an input operation of the formulation data displayed on the monitor 530, a selection operation of the formulation data in the selection screen, and the issuing operation for the formulation data requesting start of dispensing for the formulation data.

The communication IF 550 is a communication interface for connecting the medicine dispensing apparatus 4 to the communication network N1 such as LAN and the like and executes data communications between the judgement supporting apparatus 2 connected through the communication network N1. Furthermore, the communication IF 550 also comprises a wireless communication interface such as a wireless communication card for performing wireless data communication between various wireless communication devices such as barcode reader 506 and the like.

The communication IF 550 receives the formulation data from the judgement supporting apparatus 2 and stores the formulation data on the storage part 520. For example, the communication IF 550 monitors whether or not the formulation data are newly stored in a predetermined storage region of the storage part 22 disposed at the judgement supporting apparatus 2, and when the formulation data are newly stored in the predetermined storage region, retrieves the formulation data from the predetermined storage region. Of course, the communication IF 550 may be one that receives the formulation data sent from the judgement supporting apparatus 2.

### [Tablet supply unit 502]

The tablet supply unit 502 comprises a plurality of medicine cassettes 41, an individual dispensing part 43, a rotation unit 44, a hand distribution unit 45, a photographing part 46, a pass-through detection part 47, a printer unit 48, and a stamping unit 49 and the like. In a plurality of the medicine cassettes 41, a plurality of the fixing cassettes 41A which may dispense predetermined and specified kinds of tablets for every one tablet (unit amount) and a plurality of the variable cassettes 41B which may dispense optional kinds of tablets for every one tablet (unit amount) by changing the driving conditions may be included. The tablets being able to dispense from the fixed cassettes 41A and the variable cassettes 41B may be a solid medicine with various forms such as a disc shape, a spherical shape, a capsule shape and the like. Here, it is contemplated as another embodiment that the case that the tablet supply unit 502 does not have the fixed cassettes 41A and has only a plurality of the variable cassettes.

Each fixed cassettes 41A is constructed detachably to a mounting part 411 disposed in the tablet supply unit 502. To each of the mounting part 411, a first driving part 42A for driving the fixed cassette 41A individually is disposed. Each first driving part 42A comprises a driving motor 561 and a RFID reader writer 562. The driving motor 561 supplies driving force to a driving mechanism of the fixed cassettes 41A. The RFID reader writer 562 may read the information from a RFID tag (not shown in the figure) disposed to the fixed cassettes 41A or write the information to the RFID tag using the wireless communication technology of the RFID (Radio Frequency Identification).

Now, positions for placing the RFID tag (not shown) and the RFID reader writer 562 may be determined relatively in a range so far as reading and writing of the information of the RFID tag (not shown) by the RFID reader writer 562 may be possible. The RFID tag (not shown in the figure) is a non-volatile memory medium storing the cassette identification information for identifying each of the fixed cassettes 41A and the cassette identification information is written by the formulation control unit 501 in the initial setting of the medicine dispensing apparatus 4 and the like.

Each variable cassettes 41B is constructed detachably to a mounting part 412 disposed to the tablet supply unit 502. To each of the mounting part 412, a second driving part 42B for individually driving the fixed cassette 41B is disposed. Each second driving part 42B comprises driving motors 571- 574 and a RFID reader writer 575. The driving motors 571-574 supply driving force to driving mechanisms of the fixed cassettes 41B. The RFID reader writer 562 may read the information from a RFID tag 575A disposed to the fixed cassettes 41A or write the information to the RFID. The RFID reader writer 562 may read information from a RFID tag 575A disposed to the fixed cassettes 41B or write the information to the RFID tag 575A using the wireless communication technology of the RFID (Radio Frequency Identification).

Now, positions for placing the RFID tag 575A (not shown) and the RFID reader writer 575 may be determined relatively in a range so far as reading and writing of the information of the RFID tag 575A (not shown) by the RFID reader writer 575 may be possible.

The RFID tag 575A is a non-volatile memory medium storing cassette identification information for identifying each of the variable cassettes 41B and the medicine information of the tablets and the like allocated to the variable cassettes 41B in a medicine dispensing processing described later (refer to Fig. 15 left side). The medicine information is the information being able to identify kinds of the tablets (medicine) and may include such as, for example, the medicine names, the medicine IDs, the medicine codes, the JAN codes, the RSS codes, the QR codes (Registered Trademark, the same is applied hereunder.) and the like. Here, the JAN codes and the RSS codes are the information in numerals or letters expressed by a one-dimensional code (barcode, GS1 code) and the QR codes are the information in numerals or letters expressed by a two-dimensional code.

### [Fixed cassettes 41A]

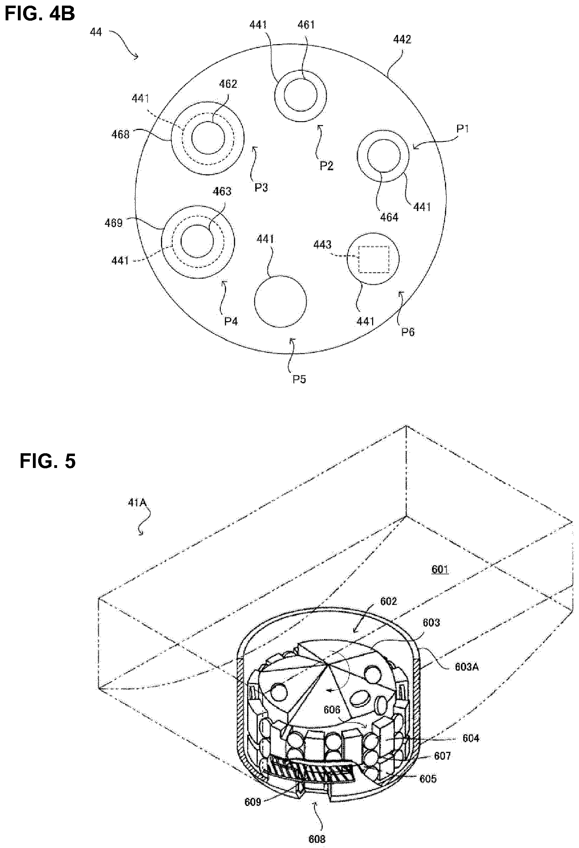

Now, with referring to Fig. 5, one example of the fixed cassette 41A will be explained. Here, the construction of the fixed cassette 41A explained herein is mere one example and the other construction may be allowed so far as it has the same function. Furthermore, Fig 5 is a drawing in that a cover member covering over an upper part of the fixed cassette 41A is omitted.

Because in each of the fixed cassette 41A the tablets reserved in the fixed cassette 41A is predetermined, the medicine information reserved in the fixed cassette 41A is described beforehand, for example, at a front face of each fixed cassette 41A.

As shown in Fig. 5, the fixed cassette 41A comprises a tablet container part 601 in which many tablets are contained and a tablet discharging part 602 for discharging the tablet contained in the tablet container part 601 one by one. The tablet discharging part 602 is disposed at a concave part formed at almost the center of the tablet container part 601 and the tablets in the tablet container part 601 falls down to the tablet discharging part 602 in turn.

The tablet discharging part 602 comprises a rotor 603 rotatably supported by a case of the fixed cassette 41A and an inner wall 603A enclosing an outer periphery of the rotor 603. The rotor 603 is connected to the driving motor 561 of the first driving part 42A through a drive transferring system (not shown in the figure) when the fixed cassette 41A is mounted to the mounting part. Furthermore, at the outer peripheral face of the rotor 603, ribs 604, ribs 605 and gaps 606 are formed intermittently at a predetermined arrangement spacing. Thereby, at the outer periphery of the rotor 603, the libs 604, the libs 605, the gaps 606 enclosed by the inner wall 603A are formed. A width of the gap 606 may be determined depending on predetermined kinds of tablets as the tablets to be contained in the fixed cassette 41A and corresponds to a width of one tablet of the tablet agent.

Furthermore, between the ribs 604, the ribs 605, the gaps 607 extending around the whole peripheral face of the rotor 603 is formed. Here, heights of top ends of the ribs 604 and the ribs 605 are determined depending on the kinds of tablets determined beforehand as the tablets to be contained in the fixed cassette 41A. Particularly, the top end height of the ribs 604 corresponds to the height of 3 tablets of the tablet agent and in each of the gaps 606 of the rotor 603, each of 3 tablets is inserted. The top end height of the ribs 605 corresponds to the height of one tablet of the tablet agent.

On the other hand, to the inner wall 603A, a discharge port 608 for discharging the tablets from the rotor 603 is formed and to the discharge port 608, a separation plate 609 being inserted to the gap 607 is disposed. Thereby, at the discharge port 608, among 3 tablets inserted in the gap 606, the upper 2 tablets are regulated not to fall by the separating plate 609 and only the lowest one tablet may be discharged. Therefore, in the fixed cassette 41A, by driving the rotor 603 with the driving motor 561 the tablets contained in the tablet container part 601 may be dispensed for every one tablet unit.

### [Variable cassette 41B]

Next, with referring to Fig. 6-Fig. 9, one example of the variable cassette will be described. Here, the variable cassette 41B and the fixed cassette 41A are also disclosed, for example, in International Publication No. 2014/112221 and the like. In addition, the construction of the variable cassette 41B explained herein is mere one example, the other construction may be possible so far as it may dispense optional kinds of tablets for every one tablet. For example, Japan Patent Publication (laid-Open) No. 2010-535683 or Japan Patent Publication (Laid-Open) No. 2010-115493 disclose other examples of the variable cassette 41B.

As shown in Fig. 6-Fig. 8, the variable cassette 41B comprises a tablet container part 701 in which many tablets are contained, and a first rotor 702 and a second rotor 703 for dispensing the tablet from the tablet container part 701. Now, Fig. 6-Fig. 8 are drawings in that a cover member covering over an upper part of the variable cassette 41B is omitted. Furthermore, the variable cassette 41B may dispense the tablets for every predetermined unit amount, and may have the construction that it dispenses for each of plural tablets.

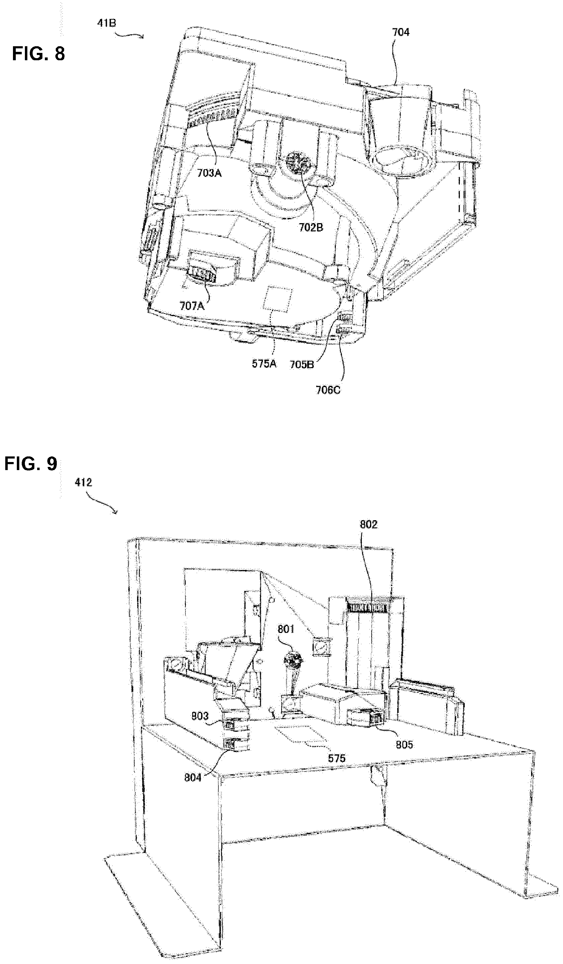

The first rotor 702 is a member having a disc shape and constructs a bottom face of the tablet container part 701. A rotation axis of the first rotor 702 is inclined with a predetermined angle with respect to the vertical direction and a top face of the first rotor 702 is inclined to the horizontal face with the predetermined angle. Furthermore, at the top face of the first rotor 702, radial ribs 702A are formed for every predetermined spacing. In addition, the first rotor 702 is supported rotatably by a case of the variable cassette 41B and is engaged to a driving gear 792B shown in Fig. 7 and Fig. 8.

The second rotor 703 is an annular hollow member disposed around the first rotor 702 in a plane view and is one example of a transfer member for transferring the tablet in the tablet container part 701 to the dispensing port 704 and for dispensing from the dispensing port 704. Furthermore, a top end part of the first rotor 702 is placed at the same horizontal plane level with the second rotor 703. In addition, the second rotor 703 is supported rotatably by the case of the variable cassette 41B and a driving gear 703A shown in Fig. 8 is formed at an outer peripheral face thereof.

On the other hand, as shown in Fig, 9, to the mounting part 412, a driving gear 801 which is engaged to the driving gear 702B of the first rotor 702 when the variable cassette 41B is mounted and a driving gear 802 which is engaged to the driving gear 703A of the second rotor 703 are disposed. The driving gear 801 is engaged to the driving motor 571 of the second driving part 42B and the driving gear 802 is engaged to the driving motor 572 of the second driving part 42B.

Furthermore, as shown in Fig. 6 and Fig. 7, the variable cassette 41B comprises a height regulation member 705 and a width regulation member 706 disposed over a dispensing path of the tablets conveyed to the dispensing port 704 by the second rotor 703.

The height regulation member 705 regulates a size in a height direction of the tablets which may be transferred to the dispensing port 704 by the second rotor 703 and the width regulating member 706 regulates a size in a width direction of the tablets which may be transferred to the dispensing port 704 by the second rotor 703. Thereby, in the variable cassette 41B, only the tablets within the height h1 regulated by the height regulation member 705 and the width w1 regulated by the width regulating member 706 among the tablets placed on the second rotor 703 may be dispensed from the dispensing port 704. Therefore, in the variable cassette 41B, the tablets may be dispensed for every one tablet unit win the case that the height h1 and the width w1 are more than the height and the width of one tablet and are less than the height and the width of two tablets contained in the tablet container part 701.

Furthermore, the variable cassette 41B comprises a height adjustment part 705A for changing the height h1 by regulated with the height regulating member 705 and a width adjustment part 706A changing the width w1 by regulated with the width regulating member 706. At the outer peripheral face of the width adjustment part 706A, a pinion gear is formed to be engaged by a rack (gear) formed on an inner peripheral face of a slot 706B formed to the width regulating member 706.

The height adjustment part 705A is supported rotatably by the case of the variable cassette 41B and is engaged to a driving gear 706C shown in Fig. 8. The height adjustment part 705A changes the height h1 regulated by the height regulating member 705 with moving upwardly and downwardly the position of a lower end part of the height regulating member 705.

The width adjustment part 706A is supported rotatably by the case of the variable cassette 41B and engaged to a driving gear 706C shown in Fig. 8. The width adjustment part 706A changes a protrusion amount of the width regulating member 706 toward the tablet container part 701 side to change the w1 regulated by the width regulating member 706. Particularly, the protrusion amount of the width regulating member 706 toward the tablet container part 701 side may be changed by each relative movement of the width adjustment part 706A and the slot 706B along to an arrow R3 direction (refer to Fig. 6) with respect to rotation of the width adjustment part 706A.

On the other hand, as shown in Fig. 9, to the mounting part 412, a driving gear 803 to be engaged to the driving gear 705B and a driving gear 804 to be engaged to the driving gear 706C, when each of which is mounted to the variable cassette 41, are disposed B. The driving gear 803 is engaged to the driving motor 573 of the second driving part 42B and the driving gear 804 is engaged to the driving motor 574 of the second driving part 42B.

Now, as shown in Fig. 8 and Fig, 9, the variable cassette 41B and the mounting part 412 comprise a driving gear 707A and a driving gear 805 each of which is connected when the variable cassette 41B is mounted to the mounting part 412. The driving gear 707A is engaged to an up-and-down mechanism not shown in the figure for going up and down in the up-and-down direction the first rotor 702 and the driving gear 805 is engaged to a driving motor not shown in the figure. Thereby, as the driving motor is driven, the driving force is transferred to the driving gear 707A from the driving gear 805, and thereby the first rotor 702 may go up and down by the up-and-down mechanism.

In addition, in the variable cassette 41B, when the first rotor 702 is rotated to a rotational direction R1 (refer to Fig. 6 and Fig. 7), the tablet in the tablet container 701 is discharged to the second rotor 703 from the first rotor 702. Likely, in the variable cassette 41B, when the second rotor 703 is rotated to a rotational direction R2 (refer to Fig. 6 and Fig. 7), the tablet on the second rotor 703 is transferred to the dispensing port 704. Here, the second rotor 703 is one example of the transferring means.

However, the tables stacked in the height direction among the tablets transferred by the second rotor 703 may be returned to the tablet container part 701 by contacting with the height regulating member 705. Besides, the tablets transferred side by side in the width direction among the tablets transferred by the second rotor 703 are returned to the tablet container part 701 by contacting with the width regulating member 706.

Thereby, in the variable cassette 41B, the tablets corresponding to the height h1 regulated by the height regulating member 705 and the width w1 regulated by the width regulating member 706 are transferred to the dispensing port 704 in the state that every tablet is positioned side by side on the second rotor 703 in a circumference direction. Thus, in the variable cassette 41B, the tablets contained in the tablet container part may be dispensed for every one tablet unit so that dispensed amounts of the tablets may be regulated.

As described above, by using the variable cassette 41B, because the height h1 regulated by the height regulating member 705 and the width w1 regulated by the width regulating member 706 may be changed so that the tablets in optional kinds may be dispensed for every one tablet unit.

Furthermore, to each variable cassette 41B, as shown in Fig. 6, a display part 707 capable of changing display contents is disposed. Here, the display part 707 is an electronic paper to which once the display contents are written by turning on electricity, the display of the display contents is kept even if thereafter the electricity is turned off.

Particularly, to each of the variable cassette 41B and the mounting part 412, contact type connectors becoming connected upon mounting the variable cassette 41B to the mounting part 412 (not shown in the figure) are disposed. Here, to the connector at the variable cassette 41B side, the display part 707 is connected and to the connector at the mounting part 412 side, the formulation control unit 501 is connected. Furthermore, when the variable cassette 41B is mounted to the mounting part 412, the display part 707 and the formulation control unit 501 are connected with the connectors. Thereby, the formulation unit 501 becomes possible to change the display of each display part 707. Besides, the display part 707 is not limited to the electronic paper and may be other display means such as a liquid crystal display and the like. Furthermore, it is contemplated that the display part 707 is disposed to the mounting part 412 to which the variable cassette 41B is mounted.

Furthermore, to each of the variable cassette 41B as shown in Fig. 8, an RFID tag 575 A is incorporated therein. The RFID tag 575A is a non-volatile recording medium in which recorded information may be re-writable by the RFID reader writer and may be used to store the identification information for each variable cassette 41B and the medicine information allocated to each variable cassette 41B. The RFID tag 575A is one which is mounted on a controller board disposed to each variable cassette 41B and the controller board has also a function that changes the display on the display part 707 of the variable cassette 41B according to the control signals from the formulation control unit 501.

The hand distribution unit 45 is used for dispensing the tablets being not adequate to dispense from the medicine cassette 41 such as, for example, a one-half tablet or a one-quarter tablet which is less than one tablet, and is disposed to be able to draw out to the medicine dispensing apparatus 4. Here, the hand distribution unit 45 may be called as DTA (Detachable Tablet Adapter). Besides, the hand distribution unit 45 comprises a plurality of DTA measures disposed in a matrix-like (grid-like).

The individual dispensing part 43 comprises a plurality of measures corresponding to the positions of each DTA measures of the hand distribution unit 45 and each measure of the individual dispensing part 43 is placed below each of the DTA measures in the state that the hand distribution unit 45 is received into the medicine dispensing apparatus 4. Besides, when the hand distribution unit 45 is used, the hand distribution unit 45 is drawn out from the front of the medicine dispensing apparatus 4 and the one-half tablets or the one-quarter tablet and the like are charged. Thereafter, the tablets charged to the DTA measures of the hand distribution unit 45 are supplied to each of the measures of the individual dispensing part 43. For example, in the hand distribution unit 45, a bottom face of each DTA measure may be opened and closed and by opening the bottom face the tablets charged in each of the DTA measure fall to each measure of the individual dispensing part 43.

The individual dispensing part 43 may supply the tablets received in each measures of the individual dispensing part 43 to the rotation unit 44 in each of the unit of the measure. Here, the hand distribution unit 45 and a hand dispensing unit being able to dispense the tablets in a measure unit as the individual dispensing part 43 are disclosed in Japan Patent Publication (Laid-Open) No. 2006-110386.

The individual dispensing part 43 comprises an open-and-close mechanism being able to open and to close the bottom face of each measure in turn and by opening in turn the bottom face of each measure with the open-and-close mechanism, the tablets charged to each measure are dispensed in turn. More particularly, it is contemplated that the individual dispensing part 43 supplies the tablets within the measure to the rotation unit 44 from each measure in a particular order determined beforehand.

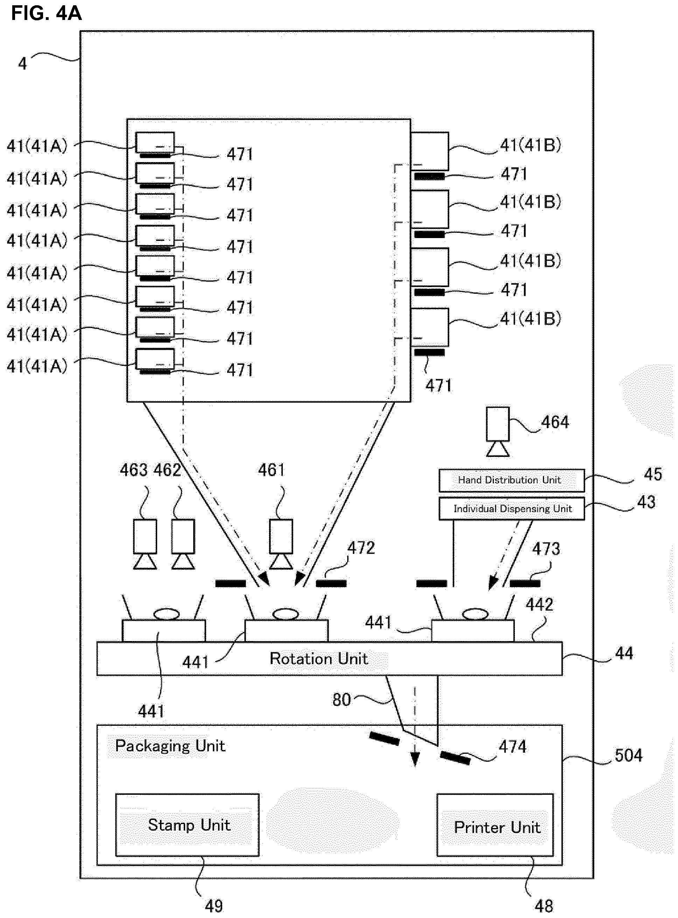

The photographing part 46, as shown in Fig. 4A, comprises cameras 461-464 disposed to a moving path of the tablets from the medicine cassette 41 to the packaging unit 504 and to a moving path of the tablets from the hand distribution unit 45 to the packaging unit 504. Here, images taken by the cameras 461-464 may be color and/or monochrome. The cameras 461-464 are used to take photographs of the tablets for every one tablet or for each of a plurality of tablets before the tablets dispensed from the medicine cassette 41 or the hand distribution unit 45 are dispensed by the dispensing paper in the packaging unit 504. In addition, by the controller part 510, the photographed images of the tablets taken by the cameras 461-464 are stored in the storage part 520 in association to the formulation data as an object of the package processing when the photographed images are taken and are sent to the judgement supporting apparatus 2.

As shown in Fig. 4A, the camera 416 is used to take photograph of the tablets supplied to the rotation unit 44 from the medicine cassette 41. The camera 462 and the camera 463 are used to take the photograph of a plurality of different peripheral regions of the tablet rotated on a tablet rotation part 441 described later and disposed to the rotation unit 44. The camera 464 is used to take photograph of the tablets received in the hand distribution unit 45.

Particularly, the controller part 510 takes a plurality of photographs of every tablet using the camera 462 and the camera 463 when the package processing based on the formulation data is performed, and stores them in the storage part 520 in association to the administration timing of the tablets received. For example, each of the photographed images may be stored in association to the formulation identification information such as the formulation ID.

Furthermore, the controller part 510 stores in the storage part 520 kinds of the tables, the photographed images, and the judgement results of an automatic judgement processing described later in association to the medicine package 451 (administration timing) obtained in the package processing based on the formulation data. That is to say, the kinds of the tablets, the photographed images, and the judgement results are stored in association each other for every administration timing included in the formulation data. Particularly, the controller part 510 stores each of the judgement results for every administration timing in association to each of the administration timing within the judgement results of the package processing based on the formulation data in the automatic judgement processing.

The pass-through detection part 47 comprises pass-through detection sensors 471-474 such as optical sensors for detecting passages of the tablets along to the moving path from the medicine cassette 41 to the packaging unit 504 and in the moving path from the hand distribution unit 45 to the packaging unit 504. In addition, detection signals of the tablets by the pass-through detection sensors 471-474 are input to the controller part 510.

As shown in Fig. 4A, the pass-through sensor 471 detects the tablets dispensed from the medicine cassette 41 and the pass-through sensor 472 detects the tablets falling down to the rotation unit 44 from the medicine cassette 41. In addition, the pass-through sensor 473 detects the tablets falling down to the rotation unit 44 from the individual dispensing part 43. Furthermore, the pass-through sensor 474 detects the tablets falling down to the packaging unit 504 from the rotation unit 44 and is placed at the position capable of detecting the tablets falling down in the medicine package 451 from a medicine introduction part 80 described later and disposed to the rotation unit 44. More particularly, the pass-through sensor 474 is disposed in the state being inserted in the dispensing paper S before the formation of the medicine package 451. Besides, the controller part 510 stores, upon performing the dispensing process based on the formulation data, a tablet number detected by the pass-through sensor 474 as the administration timing unit, i.e., as the dispensed tablet number for every medicine package 451 in the storage part 520 and sends them to the judgement supporting apparatus 2.

In addition, the controller part 510 performs a photograph processing for taking photographs by the photographing part 46, for example, in response to a detection timing of the tablets by the pass-through detection part 47. Particularly, when the tablets falling down to the rotation unit 44 from the medicine cassette 41 are detected by the pass-through detection part 47, the tablets are photographed by the photographing part 46. Taking photographs by the camera 462 and the camera 463 are performed at a photographing interval (several ms) determined beforehand under performing the package processing in the medicine dispensing apparatus 4.

On the other hand, the controller part 510 takes photographs of the hand distribution unit 45 using the camera 464 when the operation for inputting completion of hand distribution work to the hand distribution unit 45 of the tablets is input. Besides, the controller part 510 sends hand distributed images photographed by the camera 464 to the judgement supporting apparatus 2 together with information for identifying the formulation data.

The printer unit 48 may print information on the medicine package 451 before receiving the tablets in the packaging unit 504. Form example, on a surface of each medicine package 451, the information such as a patient name, an administration timing (or administration period), a medicine name, or a prescription dosage may be printed. Furthermore, in the packaging unit 504, at the first or the last of the medicine package 451 continuously obtained by the package processing based on one formulation data, an empty medicine package 451, on which one-dimensional or two-dimensional code indicating the formulation identification information such as the formulation ID for identifying the formulation data is printed may be added. The code information may be readable by the code reader part 27 and the code reader part 36. Here, the code information may be printed on each of the medicine packages 451.

The stamp unit 49 may record particular letters or a drawing pattern determined beforehand by stamping on the medicine package 451 after the tablets are received in the packaging unit 504. Particularly, the stamp unit 49 may record on the medicine package 451 the letters or images and the like indicating judgement results of the automatic judgement processing described later. For example, for every medicine package 451, it is contemplated that a stamp "OK" is stamped when the judgment result of the automatic judgement is proper; a stamp "CH" is stamped when it requires checks and a stamp "NG" is stamped when it is error.

### [Automatic judgement processing]

In the medicine dispensing apparatus 4, the controller part 510 may perform automatic judgement processing for determining propriety of the package processing based on the formulation data. Particularly, in the automatic judgement processing, based on the photographed images of the tablets taken by the camera 462 or the camera 463 and the formulation data, the propriety of the package processing based on the formulation data may be determined in a unit of the formulation data and the propriety of the package processing may be determined for every administration timing. Here, the controller part when executing the related automatic judgement processing is one embodiment of the judgement processing part.

In the automatic judgement processing, it is determined whether or not the identification information included in the photographed images of the tablets is identical with the medicine information included in the formulation data. More particularly, it is contemplated that the controller part 510 performs an image judgement processing for checking the image of the identification information of the tablet included in the photographed images with a normal image of the identification information of the tablet recorded in association to the tablet included in the formulation data. Here, the normal image is registered in the medicament master beforehand in association to every kind of the tablets. Furthermore, in the image judgement processing, it may be possible that the identification information of the tablets is read from the photographed images by a pattern matching processing or a letter recognition processing, and the identification information of the tablets and the identification information of the tablets to be included as prescribed medicines in the formulation data are checked.

Furthermore, it is determined to be "proper (image)" when a matching rate as the check result in the image judgement processing is not less than a first threshold. Besides, the result of the automatic judgement processing is determined to be "check-required (image)" when the matching rate is not less than a second threshold determined beforehand while being lower than the first threshold. Furthermore, the judgement result of the automatic judgement processing is determined to be "error (image)" when the matching rate as the check result is less than the second threshold. That is to say, the controller part 510 evaluates the matching rate by the image judgement processing in three levels.

As described above, the controller part 510 may judge the propriety of the package processing based on the photographed images taken by the camera 462 or the camera 463 and the formulation data before dispensing by the medicine package 451 in the package processing. In addition, the controller part 510 sends the judgement results of the automatic judgement processing to the judgement supporting apparatus 2. Here, when the image judgement processing is performed as the automatic judgement processing, the controller part 510 sends a part or the whole of the photographed images of the tablets to the judgement supporting apparatus 2. For example, only the images used in the check during the image judgement processing or only the images when the identification information of the tablets is read may be sent.

Incidentally, when the tablets included as the prescribed medicine in the formulation data is a non-registered medicine of which normal image of the tablet is not registered in the medicament master, the controller part 510 can not perform the image judgement processing based on the identification information of the tablets included in the photographed images. Thus, when the tablets included in the formulation data to be subjected to the package processing are the non-registered medicine, the controller part 510 does not perform the image judgement processing and determines the judgement result to be "check-required (non-registered)".

Furthermore, also in the case that the tablets included in the formulation data are unfigured medicine on which there are no letters and stamps, the controller part 510 cannot perform the image judgement processing depending on the identification information included in the photographed images. Thus, also when the unfigured medicine is included in the tablets included as the prescribed medicine in the formulation data, it is contemplated that the controller part 510 does not perform the image judgement processing but performs a shape judgement processing that the appearances such as a shape, a color, and a size of the tablet included in the photographed images is checked with the information of the appearances of the tablets stored in the medicament master as the automatic judgement processing. Besides, when the check results of the shape judgement results are proper, the controller 510 determines to be "proper (shape)" and when the check results of the shape judgement processing are not proper, the controller 510 determines to be "error (shape)". Here, also the tablets included in the formulation data to be subjected to the package processing are the unfigured medicines, it is contemplated as another embodiment that the controller 510 does not perform the image judgement processing and determines the judgement results of the automatic judgement processing to be "check-required (unfigured)".

Now, in the case that the un-registered medicine or the unfigured medicine is included as the prescribed medicines in the formulation data and the judgement result is determined to be "check-required (non-registered)" or to be "check-required (unfigured)", it is contemplated that "CH" is always recorded on the medicine package 451 by the stamp unit 49 when the non-registered medicine or the unfigured medicine is included as the prescribed medicine in the formulation data. On the other hand, the controller part 510, when the non-registered medicine or the unfigured medicine is included as the prescribed medicine in the formulation data, it is contemplated that the recordation of "CH" on the medicine package 451 by the stamp unit 49 is not performed. Thereby, "CH" is recorded on the medicine package 451 only in the case when the matching rate in the image judgement processing is less than the first threshold and is not less than the second threshold and further when the judgement results of the image judgement processing is to be check-required.

In addition to the above, the controller part 510, when the non-registered medicine or the unfigured medicine is included as the prescribed medicine in the formulation data, it is contemplated that a counting judgement processing, in which the propriety of tablets numbers charged in the medicine package 451 is determined, is performed as the automatic judgment processing. Particularly, the tablet number falling down to the medicine package 451 from the rotation unit 44 is counted by the pass-through detection sensor 474 and in the counting judgement processing, it is determined whether or not the counted the tablet number and the tablet number in the formulation data are consistent each other. Furthermore, the controller part 510 determines the judgement results of the automatic judgement processing to be "proper (count)" when the check results of the counting judgement processing is proper and also determines the judgement results to be "error (counting)" when the check results of the counting judgement processing is not proper. Here, the controller part 510 may perform the counting judgement processing together with the image judgement processing or the shape judgement processing.

In addition, in the medicine dispensing apparatus 4, the controller part 510 sends kinds of the judgement processing (image judgement processing, shape judgement processing, or counting judgement processing) performed in the automatic judgement processing and the judgement results thereof to the judgement supporting apparatus 2. Thereby, in the judgement supporting apparatus 2, confirmation of the results of the automatic judgement processing may be possible.

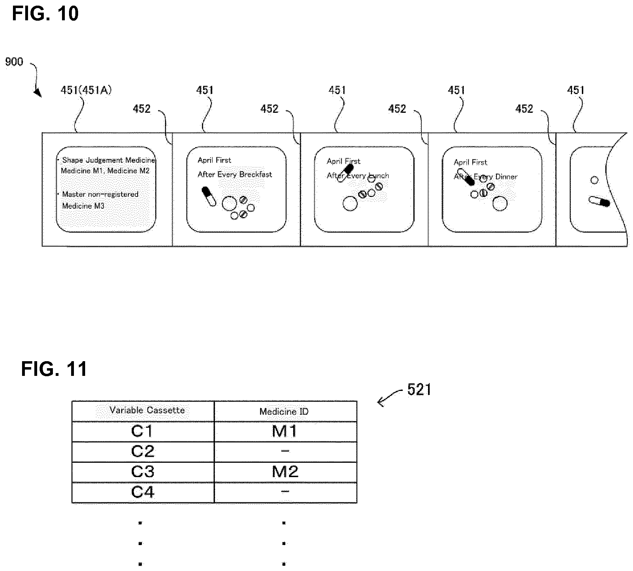

Furthermore, it is contemplated that the controller part 510 prints, when the shape judgement processing or the counting judgement processing and the like are performed, as shown in Fig. 10, an exception judgement information in the automatic judgement processing which indicates that the judgement was performed in the different method from the image judgement processing such as the shape judgement processing or the counting judgement processing on the first or the last medicine package 451A included in the continuous medicine package sheet 900. Here, the medicine package 451A may be the same with the medicine package 451 on which the code information of the formulation identification information is printed.

In the exception judgement information, for example, the kinds of the judgement processing in the automatic judgement processing and the identification information of the tablets subjected to the judgement processing (medicine names, medicine IDs and the like) may be included. Besides, the controller part 510, when the non-registered medicine is included in the formulation data, may print so on the medicine package 451A. Particularly, in the embodiment illustrated in Fig. 10, with respect to the medicine M1 and M2 as the prescribed medicine included in the formulation data, the acknowledgement "the shape judgement processing is performed" is printed on the medicine package 451A. Furthermore, on the medicine package 451A, the acknowledging that the medicine M3 as the prescribed medicine included in the formulation data is the non-registered medicine is printed. Here, when the image judgement processing is performed, the acknowledgement indicating so may be printed on the medicine package 451A.

### [Rotation unit 44]

The rotation unit 44 is, as shown in Fig. 4A and Fig. 4B, comprises six tablet rotation parts 441, a unit rotation part 442, and a medicine introduction part 80. The unit rotation part 442 is supported rotatably by a base stand not shown in the figure. Here, Fig. 4B is a schematic drawing illustrating a state viewed from the upper side.

Each of the tablet rotation parts 441 may displace postures of the tablets by rotating one tablet supplied from the medicine cassette 41 or the hand distribution unit 45. To the unit rotation part 442, six tablet rotation parts 441 are placed in spacings of 60 degrees about a predetermined rotation axis and the unit rotation part 442 may make the tablet rotation part 441 rotate about the predetermined rotation axis. Particularly, the unit rotation part 442 may move in turn each of the tablet rotation part 441 to a falling down position P1 of the tablets from the individual dispensing part 43, to the falling down position P2 of the tablets from the medicine cassette 41, to the photographing allowed position by the camera 462 P3, to the photographing allowed position of camera 463 P4, to a reserving position P5, and to the discharge position to the medicine introduction part 80 P6.

In addition, in the medicine dispensing apparatus, when one tablet dispensed from the medicine cassette 41 falls down to the tablet rotation part 441 at the falling down position P1 or when one tablet dispensed from the hand distribution unit 441 falls down to the tablet rotation part 441 at the falling down position P2, the tablet rotation part 441 is rotated for 60 degrees by the unit rotation part 442. Thereby, each of the tablets dispensed from the medicine cassette 41 or the hand distribution unit 45 is moved in turn to the discharge position P6 from the falling down position P1 or from the falling down position P2.

Furthermore, in the photographing allowed position P3 and the photographing allowed position P4, the tablets are photographed by the camera 462 and the camera 463. Here, as shown in Fig. 4B, above the rotation unit 44, a lighting device 468 and the lighting device 469 are fixed for lighting the tablets at the photographing allowed positions by the camera 462 and the camera 463. The lighting device 468 and the lighting device 469 project the light to the tablet rotation part 441 in different angles and different luminance each other such that images with different lighting environments may be photographed by the camera 462 and the camera 463.

Thereafter, the tablets placed on the tablet rotation part 441 fall down to the packaging unit 504 from the discharge position P6 through the medicine introduction part 80 forming falling down paths of the tablets and then charged to the dispensing paper 451 in the packaging unit 504.

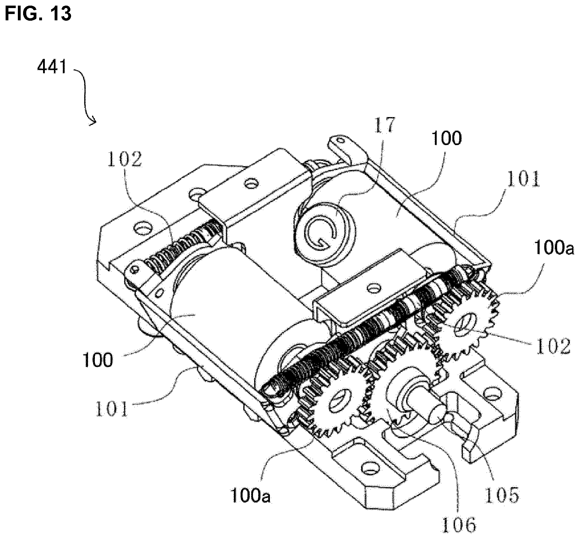

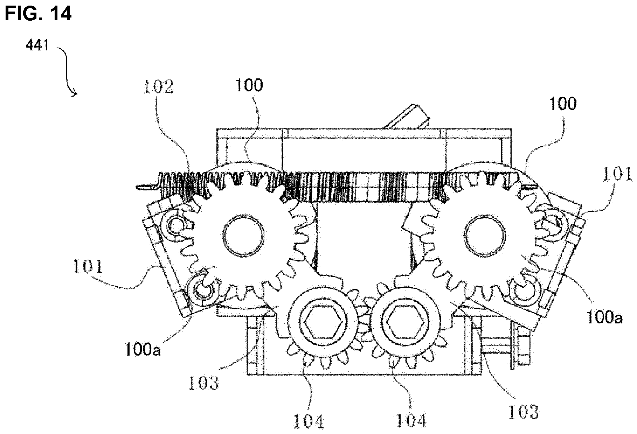

Now, with referring to Fig. 13 and Fig. 14, one example of the tablet rotation part 441 will be explained. As illustrated in Fig. 13 and Fig. 14, the tablet rotation part 441 comprises a pair of rotation rollers 100, a pair of supporting plates 101 supporting each of the rotation roller 100 individually, and a spring 102 urging the pair of supporting plates 101 to a direction becoming near each other. In addition, in the tablet rotation part 441, arms 103 extend from both ends of each supporting plate 101 and at a top part thereof, gears 104 engaging each other are formed. Thereby, in a normal condition, the rotation rollers 100 are each in the state in close proximity and the tablet 17 may be supported by the pair of rotation rollers 100. On the other hand, the pair of rotation rollers 100 become close and apart synchronously with the rotation of the gear 104. In the contact or closely coming state, the medicine 17 is supported rotatably while in the apart state, the medicine 17 falls down toward the medicine introduction part 80 from the pair of the rotation rollers 100.

Incidentally, to each of one end of the rotation axes of rotation roller 100, driven gears 100a are each integrated thereto. To each of the driven gears 100a, a driving gear 106 integrated to one end side of the driving axis 105 engages. At an opposite end of the driving axis 105, a driven roller not shown in the figure is integrated. The driven roller is constructed with a magnet gear. In addition, in the rotation unit 44, a connection magnet gear not shown in the figure connected to a predetermined drive motor is disposed at the position apart from the tablet rotation part 441 and just below the tablet rotation part 441 when the tablet rotation part 441 is moved to the photographing allowed position P3 by the camera 462 or photographing allowed position P4 by the camera 463.

Furthermore, when the tablet rotation part 441 is moved to the photographing allowed position P3 by the camera 462 or photographing allowed position P4 by the camera 463, the driving force from the predetermined drive motor is transferred to the driven roller through the connection magnet gear. Thereby, the driving force of the predetermined drive motor is transferred to the driven roller and the pair of rotation rollers 100 through the driving axis 105 such that the pair of rotation rollers 100 rotates to the same direction synchronously. Therefore, when the tablet 17 is placed on the pair of rotation rollers 100, the tablet 17 is rotated. Therefore, in the tablet rotation part 441, the posture of the tablet 17 photographed by the camera 462 and the camera 463 may be changed such that the outer peripheral face of the tablet 17 may be photographed from the different directions. That is to say, the tablet 17 rotated by the tablet rotation part 441 is photographed intermittently or sequentially by the camera 462 and the camera 463 so that the outer peripheral face including the identification information of the tablet formed by stamping to the tablet 17 may be photographed.

### [Packaging unit 504]

The packaging unit 504 receives the medicine supplied from one or both of the medicine cassette 41 and the hand distribution unit 45 of the tablet supply unit 502 for every packaging unit such as the administration timing. For example, the packaging unit 504 wraps the medicines in the packaging unit by a transparent or opaque dispensing paper S having roll shape and seals thereof to form the medicine package 451 by thermal bonding and the like. Thereby, the medicine package sheet 900 in which each of the medicine package 451 encloses the medicine in the packaging unit is fed out from the dispensing unit.

Now, Fig. 10 shown one example of the medicine package sheet 900 fed out from the packaging unit 504. As shown in Fig. 10, in the medicine package sheet 900, a plurality of the medicine packages 451 wrapping a plurality of the tablets in the packaging unit is continuously formed and between the medicine packages 451, dotted lines 452 (perforation) for easy separation of each medicine package are formed.

### [Packaging control unit 505]

The packaging control unit 505 comprises as shown in Fig. 3, a controller part 551 and a storage part 552 and makes the medicine dispensing apparatus perform the dispensing operation by controlling the tablet supply unit 502 and the packaging unit 504. Now, the packaging control unit 505 is incorporated in the medicine dispensing apparatus 4.

The controller part 551 is a controller means comprising a CPU, a RAM, a ROM and an EEPROM. The controller part 551 executes various processings by the CPU according to various programs stored beforehand in a storage means such as the ROM, the EEPROM or the storage part 552, Here, the RAM and the EEPROM are used as temporal memories (working region) for the various processing executed by the CPU. Here, the controller part 551 may be an integrated circuit such as an ASIC or a DSP.

The storage part 552 is a storage means for storing various data such as an HDD (HARD DISK DRIVE) or an SSD (Solid State Drive). Particularly, in the storage part 552, a packaging control program for making a computer such as the controller part 551 execute packaging control processings (refer to Fig. 15 right side) explained later is stored beforehand. Here, the packaging control program may be recorded in a computer readable recording medium such as a CD, a DVD, or a semiconductor memory and by read with a disc drive not shown in the figure from the recording medium and may be installed in the storage part 520. The present invention may be understood as the invention for the computer readable recording medium in which the packaging control program is recorded.

### [Barcode reader 506]

The barcode reader 506 may read codes for identifying the medicines from a container reservoir of the tablets (box, bottle and the like) disposed at a medicine shelf of a pharmacy or JAN codes, RSS codes, or QR codes described on PTC sheet. Furthermore, the barcode reader 506 may be used to read the code information indicating the formulation identification information printed on the medicine package 451A. The information read by the barcode reader is input to the formulation control unit 501 via. the wireless communication from the barcode reader 506. Here, the barcode reader 506 may be, for example, a mobile peripheral such as a PDA or a smart phone.

### [Medicine dispensing processing and packaging control processing]

Hereunder, with referring to Fig. 15, one example of process procedures of the medicine dispensing processing executed in the medicine dispensing apparatus 4 executed by the controller part 510 of the formulation control unit 501 and the packaging control processing executed by the controller part 551 of the dispensing unit 505 will be explained. Here, the process procedures (steps) executed by the controller part 510 is referred as the step S1, S2,,, and the process procedures (step) executed by the controller part 551 is referred as the step S11, S12, ... Now, it is contemplated that a sequential process obtaining similar processing results with the processing results of the medicine dispensing processing and the packaging control processing may be performed by any one of the controller part 510 and the controller part 551.

### (Formulation control unit 501 side: Step S1)

First, in the step S1, the controller part 510 determines whether or not there has been an issuing request for the formulation data. Particularly, the controller part 510 determines that the issuing request of the formulation data has been made when an issuing operation for issuing the formulation data registered beforehand is performed to the operation part 540.

Here, the controller part 510 is waited in the step S1 until the issuing request for the formulation data is done (in S1; No). On the other hand, when the controller part 510 determines that the issuing request for the formulation data has been made (S1; Yes), the processing is passed to the step S2. Here, it is contemplated as another embodiment that when the controller part 510 receives the formulation data from the host system such as the judgement supporting apparatus 2, the controller part 510 determines that the issuing request of the formulation data is done without requiring the issuing operation and passes the processing to the step S2.

### (Formulation control unit 501 side: Step S2)

Next, in the step S2, the controller part 510 determines whether or not the fixed cassettes 41A are present corresponding to all of the medicine information which is input as the medicine information indicating the tablets subjected to packaging by the formulation data. Particularly, the controller part 510, based on the cassette master stored in the storage part 520, determines whether or not the tablets, to which the cassette 41A corresponds is not present, are included in the formulation data. Here, when the fixed cassette 41A corresponding to at least one of the medicine information subjected to the packaging is not present (S2; No), the controller part 510 passes the processing to the step S3.

On the other hand, when the determination that the fixed cassettes 41A corresponding to all of the medicine information to be subjected to the packaging are present (S2; Yes), the controller part 510 passes the processing to the step S7, In this case, in the step S7, a start request for dispensing operation using each of the fixed cassettes 41A is sent to the controller part 551 and the processing for executing the dispensing operation is performed. Here, in the construction that the medicine dispensing apparatus 4 does not include the fixed cassettes 41A, the controller 510 may omit the processing of the step S2 and the controller part 510 may pass the processing to the step S3 when the determination is made that the issuing request for the formulation data is done.

### (Formulation control unit 501 side: Step S3)

In the step S3, the controller part 510 allocates the medicine information of which corresponding fixed cassette 41 A is not present, among the medicine information input by the formulation data to the variable cassettes 41B. Here, the controller part 510, when a plurality of medicine information to which the corresponding fixed cassettes 41A are not present is included in the formulation data, allocates the variable cassettes 41B with respect to each the medicine information. As described above, the controller part 510 is one example of the allocating mean when the medicine information for subjecting to the packaging is input by the formulation data and the processing for allocating the medicine information to the variable cassette 41B (allocation step) is executed.

Particularly, in the storage part 520, an allocation information 521 for indicating an allocation state between the variable cassette 41B and the medicine information is stored. Now, Fig. 11 is a drawing of one example of the allocation information 521.

As shown in Fig. 11, in the allocation information 521, the medicine IDs indicating kinds of tablets currently allocated to each variable cassette 41B are stored as the medicine information. Besides, instead of the medicine ID, the medicine information such as the medicine codes, JAN codes, (or RSS codes) may be stored. Furthermore, it is assumed that cassette numbers C1, C2, ... are set beforehand to the variable cassettes 41B. The cassette identification information is also stored un the RFID tag 575A of each variable cassette 41B. Now, in the allocation information 521, to the variable cassette 41B to which the medicine information is currently not allocated or unallocated is stored. Particularly, in the allocation information shown in Fig. 11, the allocations in which the medicine ID "M1" is allocated to the cassette number "C1" and the medicine ID "M2" is allocated to the cassette number "C3" and the cassette numbers "C2" and "C4" are not yet allocated with the medicine information, is illustrated. Now, the data structure of the allocation information shown in Fig. 11 is mere one example, and the allocation information 521 may be stored, for example, in the storage part 520 as one item of the medicament master. In this case, the cassette identification information of the variable cassette 41B allocated to the medicine may be stored in association to each medicine included in the medicament master.

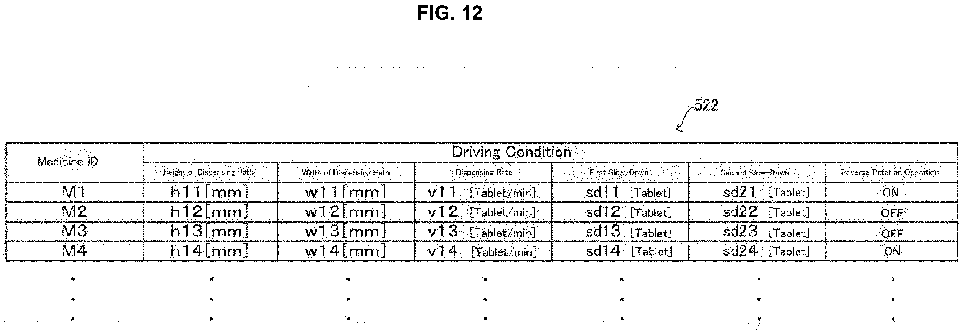

In addition, in the storage part 520, drive correspondence information 522, which indicates a corresponding relation between the medicine information and a drive condition of the variable cassette 41B, is stored. Here, Fig. 12 is a drawing showing one example of the drive correspondence information 522.

As shown in Fig. 12, in the drive correspondence information, the drive condition being set beforehand corresponding to each medicine information is stored. In the drive condition, three kinds of conditions such as a pre-drive condition relating to adjustment of the variable cassette 41B prior to starting the dispensation of the tablets from the variable cassette 41B, an on-drive condition relating to drive control during the dispensation of tablets from the variable cassette 41B, and a drive-stop condition relating to the drive control when stopping the dispensation of the tablets from the variable cassette 41B may be included.

Particularly, in the example of the drive correspondence information 522 shown in Fig. 12, as the drive conditions to each of the tablets of which medicine IDs are "M1", "M2", "M3", and "M4", information for each item such as the height of a dispensing path, a width of the dispensing path, a dispensing rate, first slow-down, second slow-down and a reverse rotation operation is stored. Here, the driving condition is mere one example and it is contemplated that when the variable cassette 41B is one that dispenses for every one tablet by vibration, a vibration frequency or an amplitude of the vibration may be set as the drive condition. Furthermore, the data structure of the drive correspondence information shown in Fig. 12 is mere one example and the drive condition determined by the drive correspondence information 522 may be one that stored in the storage part 520 as one item of the medicament master.

The height of the dispensing path and the width of the dispensing path may be mere one example of the pre-drive condition and may indicate the values of the height h1 and the width w1 set beforehand (refer to Fig. 7) as the values which allow the tablets to be dispensed for every one tablet from the dispensing port 704 by the second rotor 703 of the variable cassette 41B.

The dispensing rate is one example of the on-drive condition and represents a rotation speed of the second rotor 703 suitable to each tablet when the tablets are dispensed from the variable cassette 41B. For example, when the size of the tablets is small and the rotation speed of the driving motor 572 is high, it becomes easy to dispense excess tablets before the driving motor 572 is stopped. On the other hand, when the size of the tablets is large excess tablets are not dispensed before the driving motor 572 is stopped even when the rotation speed of the driving motor 572 is high. Thus, it is contemplated that, for example, the dispensing rate of the tablets being set as the drive condition, i.e., transferring rate of the tablets by the second rotor 703 may be different depending on the sizes of the tablets. Particularly, it is contemplated that the dispensing rate at the case for larger size tablets may be set low when compared to the dispensing rate in the case for smaller size tablets.

The first slow-down and the second slow-down are one example of the condition when the drive is stopped, and relate to information for an execution timing of the slow-down for the rotation speed of the second rotor 703 gradually when stopping the dispensation of the tablets from the variable cassette 41B. The first slow-down defines the timing for decreasing the rotation speed of the second rotor 703 to a first rotation speed being set beforehand. Furthermore, the second slow-down defines the timing for further decreasing the rotation speed of the second rotor 703 to a second rotation speed being slower than the first rotation speed. For example, when a shape of the tablet contained in the variable cassette 41B is roundish and is easy to roll over, there is a fear that the tablets are dispensed with rolling after stopping the drive of the second rotor 703. Therefore, for example, each start timing for the first slow-down and the second slow-down is set faster with respect to the tablets having shapes being easy to roll over such as, for example, spherical. In the present embodiment, each start timing for the first slow-down and the second slow-down may be set depending on residual tablet number to be dispensed from the variable cassette 41B. Thereby, the excess dispensation of the tablets may be protected upon stopping the dispensation of the tablets from the variable cassette 41B. Alternatively, since each timing for the first slow-down and the second slow-down is set slower with respect to the tablets being hard to roll over upon stopping the drive of the second rotor 703, a time delay of dispensation due to unnecessary slow-don may be suppressed.

Besides, the item of the reverse rotation operation is one example for the stop-drive condition and is information about presence or absence of execution for the reverse rotation operation, which changes the transferring direction to the reverse direction by the second rotor 703 upon stopping the dispensation of the tablets from the variable cassette 41B. For example, with respect to the tablets surviving on the second rotor 703 with the shape being easy to roll over such as the spherical shape and providing a fear for the excess dispensation by merely stopping the drive of the second rotor 703, the reverse rotation operation is set to "ON". Thereby, the excess dispensation of the tablets from the variable cassette 41B upon stopping the dispensation of the tablet may be protected. Here, with respect to the tablets with the shape being hard to roll over upon stopping the drive of the second rotor 703, the reverse rotation operation is set to "OFF" and unnecessary reverse rotation operation may not be performed.

Particularly, the controller part 510 performs the processing for specifying the variable cassette 41B currently being able to communicate (controllable) in each variable cassette 41B. For example, the controller part 510 determines that the variable cassette 41B is in the state being able to communicate if reading the information from the RFID tag 575A by the RFID reader writer 575 has been succeeded within the variable cassettes 41B.

In addition, the controller part 510 determines presence or absence of the current allocation of the medicine information to each variable cassette 41B currently being able to communicate based on the allocation information 521 (refer to Fig. 11) and allocates the medicine information to be subjected to the dispensation to the unallocated variable cassette 41B. In this case, when the controller part 510 determines the variable cassette 41B for allocating the medicine information, the controller part 510 updates the contents of the allocation information in response to the allocation results.

Here, it is contemplated that there is a plurality of candidates for the variable cassette 41B to which the medicine information is allocated. In this case, it is contemplated that the controller part 510 determines presence or absence of the allocation of the medicine information based on a priority order set beforehand to each variable cassette 41B and allocates the medicine information to the variable cassette 41B that has been determined as unallocated first. Now, when the unallocated variable cassette 41B is not present, the controller part 510 reports to a user by displaying the acknowledgement on the monitor 530.

Furthermore, in the step S3, the controller part 510 records by controlling the RFID reader writer 575 the medicine information allocated to the variable cassette 41B to the RFID tag 575A of each variable cassette 41B to which the medicine information has been allocated. In this case, it is contemplated that the controller part 510 records various information such as a dispensation amounts, patient names, allocation dates, names of responsible pharmacists and identification information of the medical prescription together with the medicine information depending on the formulation data.

On the other hand, it is contemplated that the medicine information is not recorded to the RFID tag 585A of the variable cassette 41B as another embodiment. Particularly, it is contemplated that the cassette identification information is recorded beforehand in the RFID tag 575A and the RFID reader writer 575 is the RFID reader which can only read the information. Even when this is the case, the controller part 510 may recognize the medicine information allocated to the variable cassette 41B based on the cassette identification information read from the RFID tag 575A and the allocation information (refer to Fig. 9).

### (Formulation control unit 501 side: Step S4)

In the step S4, the controller part 510 specifies the drive condition corresponding to the medicine information to be subjected to the dispensation based on the drive correspondence information 522 (refer to Fig. 12) and sends to the controller part 551 the drive condition and the cassette identification information to which the medicine information is allocated. Thereby, the controller part 551 may make the variable cassette 41B drive in accordance with the drive condition.

### (Packaging control unit 505 side: Step S11)

On the other hand, in the packaging control unit 505, the controller part 551 in the step S11 determines presence or absence of the reception of the drive condition from the controller unit 510. Here, the controller unit 551, when the drive condition is received (S11: Yes), passes the processing to the step S12 and until the drive condition is not received (S11: No) passes the processing to the step S13. Besides, the controller part 551 records the received drive condition to the storage part 552 in association to the cassette identification information of the variable cassette 41B to which the medicine information is allocated.

### (Packaging control unit 505 side: Step S12)

In the step S12, the controller part 551 makes the variable cassette 41B corresponding to the cassette identification information received together with the drive condition drive according to the pre-drive condition among the drive conditions and changes the height h1 of the dispensing path and the width w1 of the dispensing path. As described above, in the medicine dispensing apparatus 4, when the pre-drive condition is included in the drive condition, the controller part 551 makes the variable cassette 41B in accordance with the pre-drive condition (h1 and w1 of the dispensing path) drive and then performs the dispensation of the tablets from the variable cassette 41B (S14).

Particularly, the controller part 551, by controlling the height regulation part 705A and the width regulation part 706A according to the drive condition, changes the kinds of the tablets being able to dispense from the variable cassette 41B for one tablet unit to the tablet indicated by the medicine information allocated in the step S3. First, the controller part 551 returns the position of the height regulation member 705 and the width regulation member 706 to initial states by driving the driving motor 573 and the driving motor 574. Then, the controller part 551 drives the height regulation part 705A by the driving motor 573 to change the height h1 to be regulated by the height regulating member 705 of the variable cassette 41B to the height of the dispensing path determined by the drive condition. Furthermore, the controller part 551 drives the width regulating part 706A to change the width w1 to be regulated by the width regulating member 706 of the variable cassette 41B to the width of the dispensing path determined by the drive condition. Of course, when a construction which is able to detect the current state of the height regulating member 705 and the width regulating member 706 is adopted, positions may not be returned to the initial states first.

As described above, when the height h1 and the width w1 of the dispensing path are changed according to the drive condition, in the variable cassette 41B, the dispensation of the tablets indicated by the medicine information allocated in the step S3 in one tablet unit becomes possible and the dispensing amounts of the tablets may become controllable.

Furthermore, it is contemplated as another embodiment the construction that the pre-drive condition is not included in the driving condition and the height regulating part 705A and the width regulating part 706A of the variable cassette 41B may be actuated manually such that the height h1 and the width w1 of the dispensing path may be adjusted optionally. In this case, the user adjusts the height h1 and the width w1 of the dispensing path of the variable cassette 41B and then, mounts the variable cassette 41B to the mounting part 412 of the tablet supply unit 502. Here, it is contemplated that the height regulating part 705A and the width regulating part 706A have the constructions allowing their actuation, for example, with a screwing operation using a tool such as a driver and the like.

### (Formulation control unit 501 side: Step S5)

Next, in the step S5, the controller part 501 makes the display part 707 of the variable cassette 41B to which the medicine information is allocated in the step S3 display the medicine information.

For example, the controller part 510 makes the display part 707 display by extracting the information of the display item set beforehand from the formulation data. Particularly, on the display part 707, the medicine name, the medicine ID, the dispensing amount and the JAN code (barcode) of the tablet to which the variable cassette 41B is allocated are displayed. Here, various information such as the patient name, the allocation date, or an allocation staff may also be displayed on the display part 707.

### (Formulation control unit 501 side: Step S6)

Thereafter, in the step S6, the controller part 510 determines whether or not a charge completion operation indicating completion for charging the tablet to the variable cassette 41B has been done to the operation part 540. Particularly, the user removes the variable cassette 41B from the tablet supply unit 502 after the medicine information is allocated to the variable cassette 41B in the step S3 and also after the medicine information is displayed on the display part 707. Then, the user charges the tablets of a necessary tablet number with referring to the medicine information displayed on the medical prescription or the display part 707 corresponding to the formulation data to the variable cassette 41B. Further then, the user mounts the variable cassette 41B to the tablet supply unit 502 and then performs the charge completion operation to the operation part 540.

Now, during the charge completion operation is performed (S6; No.), the controller part 510 makes the processing wait in the step S6. On the other hand, when the determination that the charge completion operation has been performed (S6; Yes), the controller part 510 passes the processing to the step S7. Now, when a plurality of medicine information is allocated to a plurality of variable cassettes 41B, respectively, in the step S6, it is determined whether or not the charge completion operation of the tablets is done for all of the variable cassette 41B corresponding to each of the medicine information.

### (Formulation control unit 501 side: Step S7)

In the step S7, the controller part 510 sends a start request for the dispensing operation based on the formulation data to the controller part 551. Particularly, with respect to the dispensing operation for the tablets not present in the fixed cassette 41A among the tablets indicated by the medicine information included as the dispensation object in the formulation data, the controller part 510 sends the start request, for example, as the following procedure.

First, the controller part 510 retrieves the cassette identification information of the variable cassette 41B mounted to each of the mounting part 412 and then, specifies the variable cassette 41B currently mounted to each of the mounting part 412. Then, the controller part, based on the formulation data, specifies each of the variable cassette 41B containing the tablets corresponding to the medicine information indicated by the formulation data in the variable cassette 41B. Further then, the controller 510 sends to the controller part 551 the cassette identification information, identification information of the mounting mart 412 to which the variable cassette 41B is mounted and necessary information for the dispensing operation such as the dispensing amount of the tablets and the like with respect to each medicine information indicated by the formulation data.

### (Packaging control unit 505 side: Step S13)

On the other hand, in the packaging control unit 505, the controller unit 551 determines in the step S13 presence or absence of the start request from the controller part 510. Here, the controller part 551, when the start request for the dispensing operation is received (S13: Yes), the processing is passed to the step S14 and when the start request for the dispensing operation is not received (S13: No), the processing is passed to the step S11.

### (Packaging control unit 505 side: Step S14)

In the step S14, according to the start request for the dispensing operation, the controller part 551 executes the dispensing operation for dispensing the necessary medicines from the medicine cassette 41 and the hand distribution unit 45 of the tablet supply unit 502 and for packaging the tablets in the dispensing unit such as the administration timing and the like by the packaging unit 504. Now, in the above packaging operation, the tablet number dispensed from the variable cassette 41B is counted by a counter having an optical sensor not shown in the figure disposed to the dispensing port 704 of the variable cassette 41B and is input as the dispensed number to the controller part 551. Thereby, the controller part 551 controls the drive of the variable cassette 41B based on the dispensed number input by the counter to dispense only preset dispensing amounts (formulation amounts) from the variable cassette 41B.

### (Packaging control unit 505 side: Step S15)

Thereafter, the controller part 551, when the dispensing operation in the step S14 is completed, in the subsequent step S15, sends a completion acknowledgement of the dispensing operation to the control unit 510.

### (Packaging control unit 510 side: Step S8)

In response to the above in the formulation control unit 501, the controller part 510 waits the completion acknowledgement of the dispensing operation from the controller part 551 (S8: No). Then, upon receiving the completion acknowledgement of the dispensing operation (S8: Yes), the controller part 510 passes the processing to the step S9.

### (Packaging control unit 501 side: Step S9)

In the subsequent step S9, the controller part 510 displays the acknowledgement for the completion of the dispensation on the display part 707 of the variable cassette 41B. For example, in the step S9, it is contemplated that the letters of "dispensation completed" is displayed on the display part 707 of the variable cassette 41B or that the display of the medicine information on the 707 is erased and the like.

Now, in the medical institute such as hospitals and pharmacies and the like, the judgement work is performed by a pharmacist to confirm the propriety of the packaged tablets by the medicine dispensing apparatus 4 corresponding to the formulation data. To this, in the judgement supporting system 1, the judgement work of the pharmacist will be supported by performing the judgement supporting processing described later (referring to Fig. 16). Here, the judgement supporting processing may also be performed in the controller part 41 of the medicine dispensing apparatus 4.

### [Judgement supporting processing]

Hereafter with referring to Fig. 16, one example of the judgement supporting processing executed by the controller part 21 of the judgement supporting apparatus 2 in the judgement supporting system 1 will be explained. The judgement supporting processing may be executed in the cases that a log-in operation to the judgement supporting apparatus 2 by the pharmacist having the final judgement authority set beforehand is done, or a judgement initiation operation for performing the final judgement processing after the log-in operation is done and the like. Besides, a log-in authentication to the judgement supporting apparatus 2 is performed by the user master stored in the storage part 22. Such as the "display" and "operation" in the judgement supporting processing described hereunder are made by using the display part 33 and the operation part 34 of the client peripheral 3 to which the log-in operation is done. Of course, a similar operation and display may be done in the judgement supporting apparatus 2.

### <Step 21>

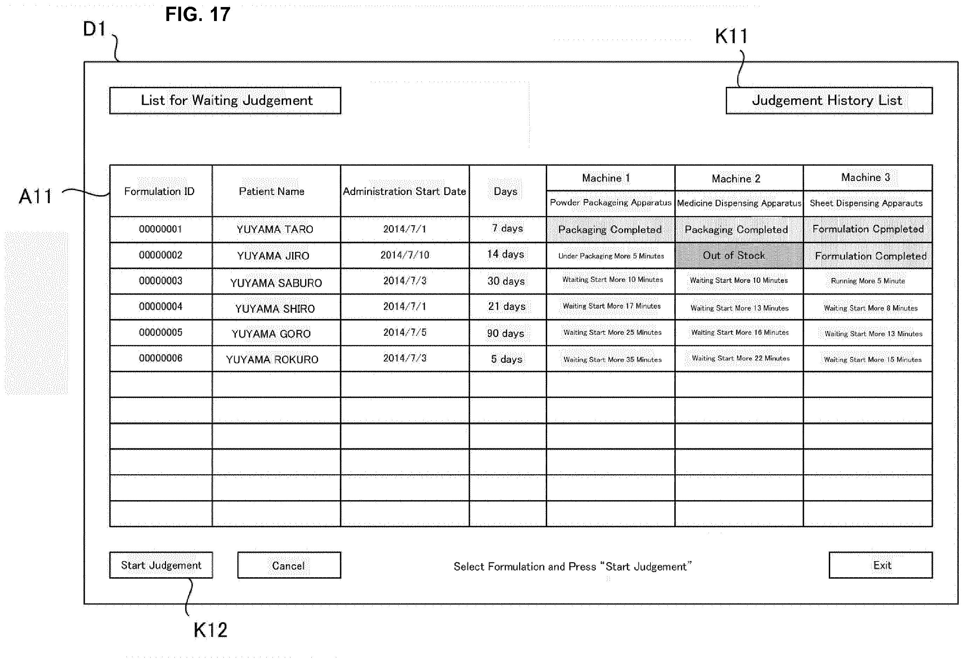

First in the step S21, the controller part 21 makes the client peripheral 3 display a list screen D1 for waiting the judgement on which a list of the formulation data to be candidate for the judgment object is displayed. Now, Fig. 17 illustrates one example of the list screen D1 for waiting the judgement.

As shown in Fig. 17, in the list screen D1 for waiting the judgement, a list display region A11 for displaying the list of the formulation data to be subjected to the judgement. Particularly, in the list display region A11, the formulation ID included in the formulation data (formulation identification information), the patient name (patient identification information), an initial date of administration starts and days as well as status information of a plurality of prescription devices such as the medicine dispensing apparatus 4 and the prescription device 5 connected to the judgement supporting system 1 are displayed.

Furthermore, in the list screen D1 for waiting the judgement, operation keys K11, K12 and the like are displayed for receiving user operations. The operation key K11 is an operation key for displaying a judgement history screen D2 to display judgement histories in the judgement supporting apparatus 2, and the operation key K12 is an operation part for displaying a judgement screen D3 to execute the judgement processing in the judgement supporting apparatus 2. That is to say, the controller part 21 may start the judgement about the prescriptions made in a plurality of devices such as the medicine dispensing apparatus 4 and the proscription device 5 and the like.

### <Step S22>

In the step S22, the controller part 21 determines whether or not the display operation of the judgement history screen D2 is done. Particularly, the controller part 21 determines that the display operation for the judgement history screen D2 has been done when the operation key K11 in the list screen D1 for waiting the judgement is operated. Then, when the controller part 21 determines that the display operation for the judgement history screen D2 has done, (S22: Yes), the controller part 21 passes the processing to the step S23, and when the display operation for the judgement history screen D2 is not done (S22: No), the processing is passed to the step S24.

### <Step S23>

In the step S23, the controller part 21 makes the client peripheral 3 display the judgement history screen D2. As described above, the controller part 21 may display the judgement history about the prescriptions performed by a plurality of the prescription devices such as the medicine dispensing apparatus 4 and the prescription device 5 and the like.

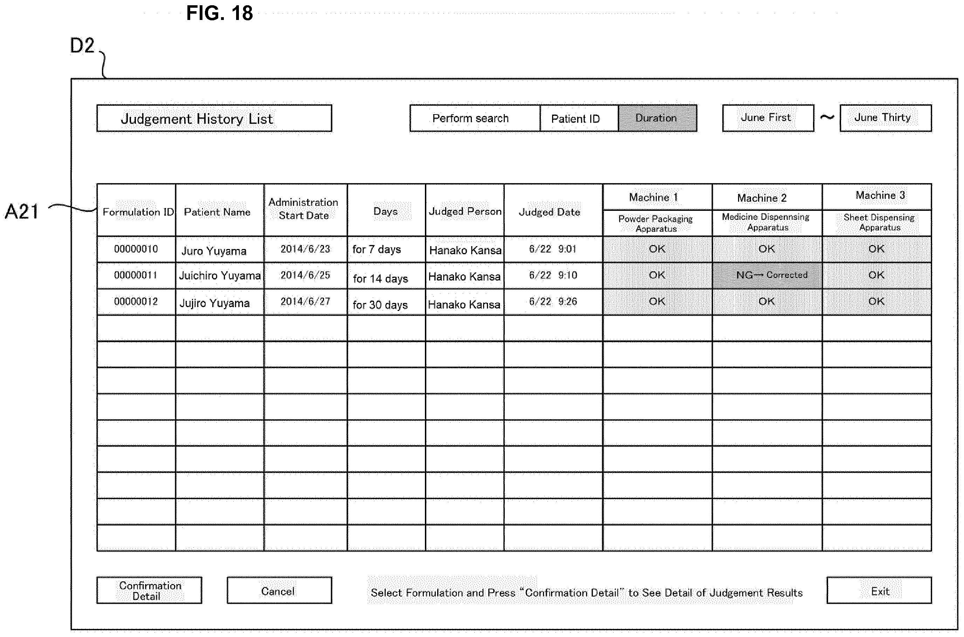

Here, Fig. 18 illustrates one example of the judgment history screen D2. As shown in Fig. 18, in the judgement history screen D2, a list display region A21 for displaying the list of the formulation data of which judgement has been completed is displayed. In the list display region A21, information such as the prescription ID (prescription identification information) included in the formulation data, the patient name (patient identification information), an initial date of administration start, a judged person and a judged date and the like is displayed. Furthermore, in the list display region A21, the display "OK", which indicates the judgement result of the medicines prescribed in each of the medicine dispensing apparatus 4 and the prescription device 5 is proper, or the display "NO", which indicates the judgement result is error, is displayed. Particularly, the result corresponding to the medicine dispensing apparatus 4 is the result of the automatic judgement processing. Here, for the display "OK", a background or the letters may be displayed in a first specified color such as blue or white set beforehand and for the display "NG", the background color or the letter may be displayed in a second color such as red set beforehand. In addition, when the display "NG" is displayed, contents of a counter measure addressed to the acknowledgement of the error (for example, "correction completed") may be displayed.

### <Step S24>

In the step S24, the controller part 21 determines whether or not the judgement start operation is done. Particularly, the controller part 21 determines that the judgement start operation is done when the operation key K12 is operated under the condition that the formulation data is selected in the list screen D1 for the waiting judgement. When the controller part 21 determines that the judgement start operation is done (S24: Yes), the controller part 21 passes the processing to the step S25 and when the judgement start operation is not done, (S24: No), the controller part 21 passes the processing to the step S26. Incidentally, the controller 21 may also determine that the judgement start operation for the formulation data to be subjected to the judgement is done, for example, when the code information described on the medicine package 451 is read by the code reader part 27 and the formulation data is specified based on the code information.

### <Step S25>

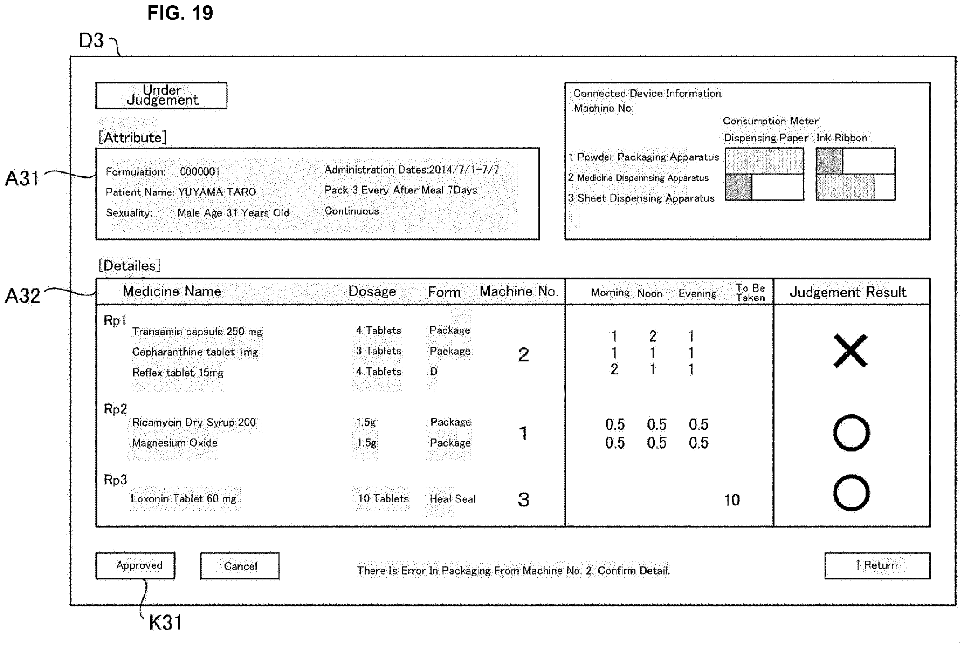

In the step S25, the controller part makes the client peripheral 3 display the judgement screen D3 for performing the judgement about the formulation data selected in the list screen D1 for waiting the judgement. Here, Fig. 19 is a drawing illustrating one example of the judgement screen D3. As shown in Fig. 19, in the judgement screen D3, a basic information region A31 and a judgement result region A32 are displayed. In the basic information region A31, information such as formulation ID (formulation identification information) included in the formulation data, patient name, sexuality, age, administration date and usage and the like is displayed.

In the judgement result region A32, the contents of the formulation data selected in the list screen D1 for waiting the judgement and the judgment results and the like about the formulation data are displayed for every record (Rp1-Rp3). Here, when a plurality of records is included in the formulation data, each of the records may sometimes be referred as the formulation data.

Furthermore, in the judgement result region A32, medicine names, dosages, medicine forms, device numbers, dosages and results of the judgements are displayed. Here, the judgement results are, for example, the judgement results for each of the formulation data by the automatic judgement processing performed in the medicine dispensing apparatus 4 and are timely input to the judgement supporting apparatus 2 from each of the medicine dispensing apparatus 4 and the prescription device 5. Furthermore, the automatic judgement processing may be executed by the controller part 21 of the judgement supporting apparatus 2 which has obtained various information such as images and the like from the medicine dispensing apparatus 4 and the prescription device 5.

### <Step S26>

In the step S26, the controller part 21 determines whether or not a detail confirmation operation has been done about the judgement results displayed on the judgement screen D3. Particularly, in the judgement screen D3, it is determined that the detail confirmation operation has been done when display positions of the judgement results corresponding to any one of the records included in the formulation data displayed on the judgement screen D3 are selected. Here, when the controller part 21 determines that the detail confirmation operation has been done (S26: Yes), the controller 21 passes the processing to the step S27 and when the detail confirmation operation is not done (S26: No), the controller 21 passes the processing to the step S28.

### <Step S27>

In the step S27, the controller part 21 executes a judgement display control processing for displaying a judgement detail screen D303 to which the details of the judgement results displayed on the judgement screen D3 is displayed. Here, the judgement display control processing is executed by the display processing part 211 of the controller part 21.

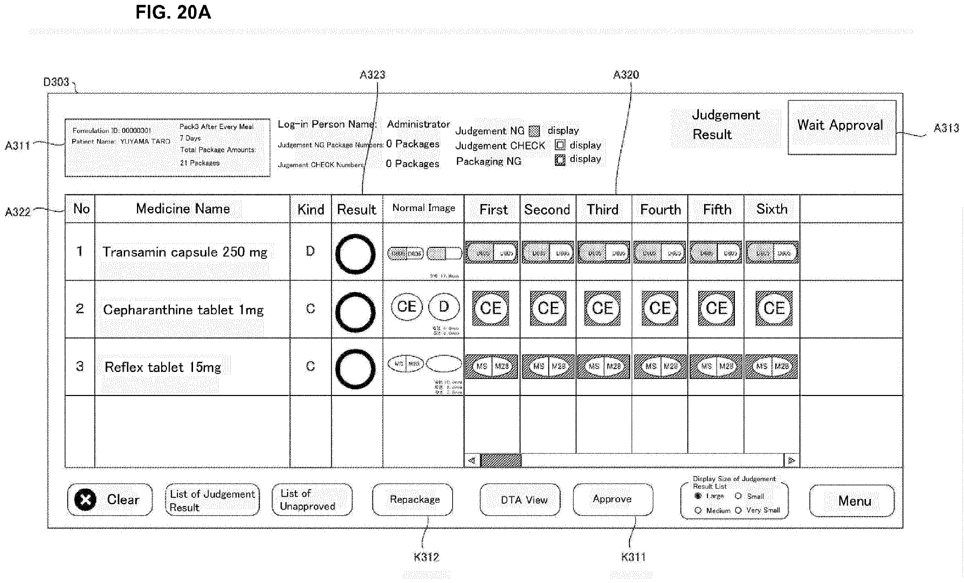

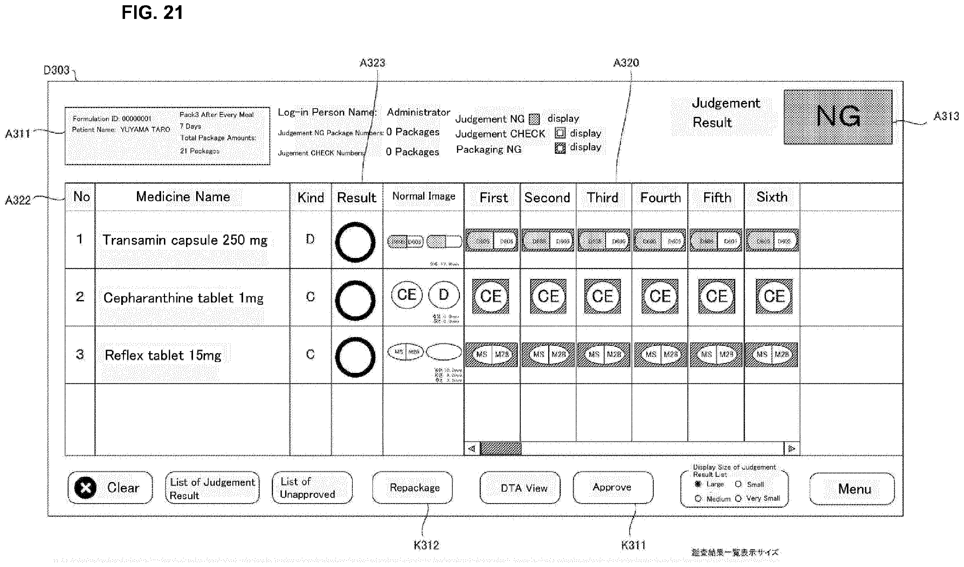

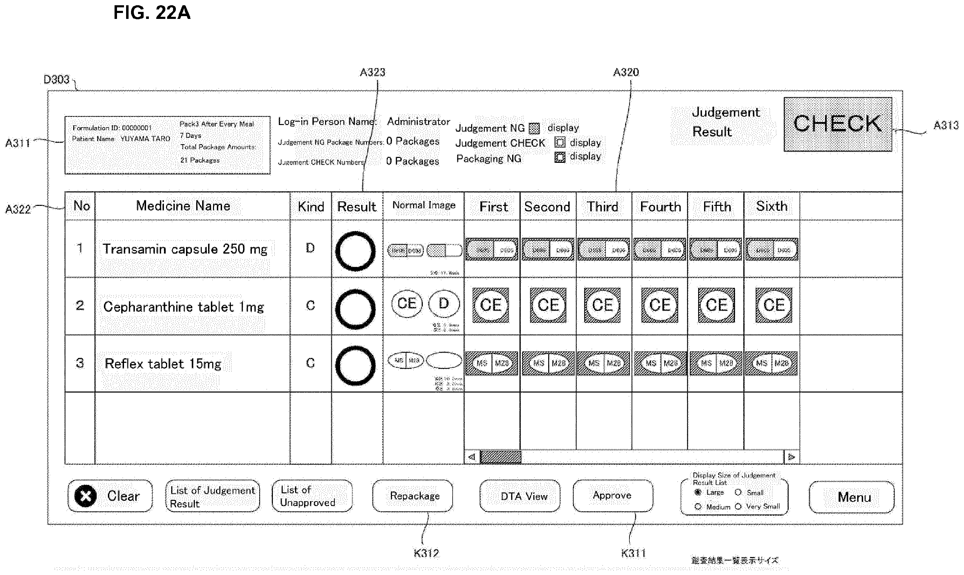

Here, Fig. 20A, Fig. 21 and Fig. 22A are drawings of the judgement detail screen. Particularly, Fig. 20A is a display example when the the judgement result by the automatic judgement processing is proper; Fig. 21 is a display example when the judgement result by the automatic judgement is error; and Fig. 22A is a display example when the judgement result by the automatic judgement processing is to be check-required.

In the judgement detail screen D303, a basic information region A311, a prescription detail region A322 and a judgement result region A313 are displayed. The basic information region A311 is the region in which the patient information and the formulation data are displayed likely to the basic information region A31. Particularly, in the basic information region A31, formulation related information such as the formulation IDs, patient names, and usages and the like are displayed. Here, other information such as dosage and the like in the formulation data may be displayed as the formulation related information in the basic information region A31. In the prescription detail region A322, the contents of the tablets dispensed in the medicine package 451 in the unit for the medicine package 451 (unit for administration timing) are displayed in a direction set beforehand side by side.

Here, in the prescription detail region A322 and in a display region A320 for displaying package number information such as a first package, a second package,... that indicates package numbers of the medicine package 451, it is contemplated that the administration timing (taking date and administration timing) may be displayed together with the package number information or instead of the package number information.

Furthermore, it is contemplated that in the medicine dispensing apparatus 4, when the tablet numbers corresponding to every administration timing are counted by the pass-through detection sensor 474, the controller part 21 displays the tablet number on the judgement detail screen D303. Thereby, the user may understand easily that there is no problem in the tablet number. Furthermore, when the medicine dispensing apparatus 4 comprises a photographing part for taking photographs of the medicine package 451 after the dispensation at the dispensing unit, it is contemplated that the controller part 21 displays the photographed images after the packaging taken by the camera on the detail screen D303.

In the prescription detail region A322, medicine names, classifications, results and normal images are displayed. Particularly, in the classifications, it is displayed so as to be able to identify that the tablets were dispensed from any one of the medicine cassettes 41 and the hand distribution unit 45. For example, when a source of the dispensation is the medicine cassette 41, an identification sign such as "C" or "Ka in Japanese Katakana" letter may be displayed and when the source of the dispensation is the hand distribution unit 45, the identification sign such as "D" or "Te in Japanese Kanji" letter may be displayed. Here, in the results, it may be individually displayed whether or not the judgement result is proper. The normal image is the registered image which has been registered in the medicament master beforehand as the verified image of the tablet. For example, in the medicament master, two images of the tablet corresponding to the front and back faces are registered in association to each of the tablets. Here, the registered images may also be one photographed image in which the region of tablet including the identification information thereof within the outer peripheral face of the tablet is photographed.

Besides, in the prescription detail region A322, in association to each of the tablets, the photographed images of the tablets taken by the photographing part 46 before the dispensing to each of the medicine packages 451 are displayed as dispensing result information. Here, when only the counting judgement processing is performed in the automatic judgement processing, the display of the photographed images may be omitted.

Besides, the controller part 21, when the image judgement processing is performed as the automatic judgement processing, displays at least an original image from which the identification information of the tablet was read in the medicine dispensing apparatus 4 or the original image compared with the normal image. On the other hand, the controller part 21 may further display a plurality of images corresponding to different outer peripheral images of the tablet photographed for each tablet in the medicine dispensing apparatus 4. Thereby, the pharmacist may determine the propriety with referring to the photographed images of a plurality of sheets when determination of the propriety of the tablet is difficult only with one photographed image of the tablet.

For example, in the prescription detail region A322 shown in Fig. 20A, as the photographed image of the tablet dispensed within the first package of the medicine package 451, each of the photographed images of three tablets of "Transamin capsule 250 mg", "Cepharanthine tablet 1mg", and "Reflex tablet 15mg" are displayed in one sheet. Here, when the tablet is a flat tablet, two images of the front and back faces of the tablet may be displayed and when the tablet has a shape of a capsule, it may be possible to display two images including the face on which the identification information of the tablet is included and the face on which the identification information of the tablet is not included.

Besides, as shown in Fig. 21, in the prescription detail region A322, when the result of the automatic judgement processing for the medicine included in the formulation data displayed on the judgement detail screen D303 is error, backgrounds of one or more regions just corresponding to the medicine such as the region A314 and the region A315 are displayed in the second specified color such as red determined beforehand. Thereby, the user understands easily the tablets of which results of the automatic judgement processing are error by confirming positions of the region A314 and A315.

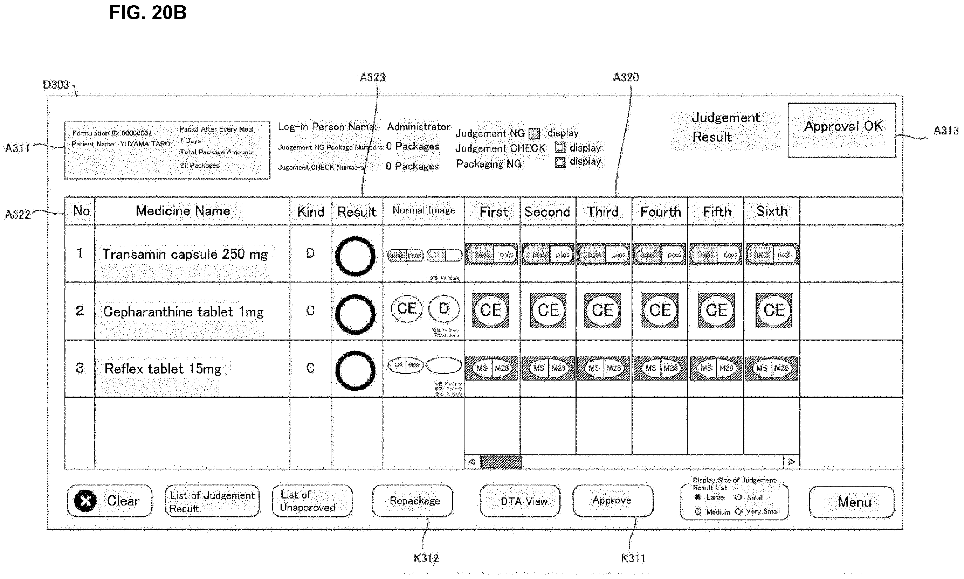

Furthermore, in the judgement result region A313, the results of all of the automatic judgement processing about all of the medicine included in the formulation data displayed on the judgement detail screen D303 are displayed. Here, the result of the automatic judgement processing displayed on the judgement result region A313 is one example of the dispensing result information. Particularly, in the example shown in Fig. 20A, "waiting approval", which acknowledges the judgement result of the automatic judgement processing is proper, is displayed on the judgement result region A313 together with the background color of the first specified color. Furthermore, in the judgement detail screen D303, an approval key K311 is displayed for performing the operation that the judgement results currently displayed are to be confirmed. Besides, the controller part 21 changes, when the approval key K311 is operated, the judgement result region A313 to "Approval OK" as shown in Fig. 20B.

Furthermore, in the example shown in Fig. 21, "NG", which acknowledges the judgement results of the automatic judgement processing is error, is displayed on the judgement result region A313 together with the background with the second specified color. Furthermore, in the example shown in Fig, 22A, the mark "CHECK", which acknowledges the judgement results of the automatic judgement processing requires the check, is displayed on the judgement result region A313 together with the background of a third specified color such as yellow which is determined beforehand and is different from the first specified color and the second specified color.

Particularly, in the prescription detail region A322, an individual result display region A323 is displayed individually, which indicates the judgement results of the automatic judgement processing for every tablet included in the formulation data. Particularly, in the example shown in Fig. 21, with respect to "Transamin capsule 250 mg" and "Cepharanthine tablet 1mg", the mark "Circle", which indicates the results of the judgement processing are proper, is displayed while with respect to "Reflex tablet 15mg", the mark "X", which acknowledges that the error occurs in the result of the judgement processing, is displayed. Thereby, the pharmacist can understand easily the tablets which causes the error of the result in the judgement processing. The information indicating whether or not the result of the judgement processing is proper is also one example of the dispensing result information. Here, as shown in Fig. 22A, when the result of the judgement is check-required, the mark "Triangle", which acknowledges requirement of the check, is displayed on the individual result display region A323.

Furthermore, it is contemplated that the controller part 21 displays, when the unfigured medicine is included as the proscribed medicine in the formulation data, the check-required (unfigured) is displayed on the judgment result region A313 of the judgement detail screen D303. For example, in the judgement detail screen D303, within the judgement result region A313, the mark "CHECK (unfigured)" is displayed on the judgement result region A313 with the background color of a fourth specified color such as orange different from the first -third colors. Furthermore, the controller part 21 also displays in a display field corresponding to the unfigured medicine with the fourth specified color in the precipitation detail region S322. Thereby, the user can specify the unfigured medicine easily.

Likely to the above, it is contemplated that the controller part 21 displays, when the non-registered medicine is included as the proscribed medicine in the formulation data, the acknowledgement for the check-required (non-registered) on the judgment result region A313 of the judgement detail screen D303. For example, in the judgement detail screen D303, within the judgement result region A313, the mark "CHECK (non-registered)" is displayed on the judgement result region A313 with the background color of a fifth specified color such as green different from the first-fourth colors. Furthermore, the controller part 21 also provides a display column corresponding to the non-registered medicine with the fifth specified color in the precipitation detail region S322. Thereby, the user can specify the non-registered medicine easily.

That is to say, the controller part 21 expresses, when the judgement result of the automatic judgement processing is to be the check-required, the categories of the check-required (Check-required (image), Check-required (unfigured) and Check-required (non-registered) in the display colors in the judgement result region A313. Therefore, the user can understand the categories of the check-required, when the judgement result of the automatic judgement processing is the check-required, by checking color arrangements in the judgement result region A313. Thus, when the judgement result of the automatic judgement processing is to be check-required, the same letter "CHECK" may be displayed in the judgement result region A313.

Now, as described earlier, in the automatic judgement processing, not limited to the image judgement processing, there are cases in that the shape judgement processing and the counting judgment processing and the like may be performed. Here, the categories of the judgement processing performed in the automatic judgement processing (image judgement processing, shape judgement processing, and counting judgment processing) may be displayed at the different region from the above judgement result region A313. On the other hand, it is contemplated that the controller part 21 may display the categories performed in the automatic judgement processing by using the judgement result region A313.

For example, the controller part 21 displays, when all of the judgement results of the image judgement processing, the shape judgement processing and the counting judgement processing is proper, the letter "waiting approval", or the letter "OK" and the like as shown in Fig. 20A on the judgement result region A313. However, when the image judgement processing is not performed and only shape judgement processing or the counting judgement processing has been performed, it is contemplated that the controller part 21 makes the judgement result region A313 display the category of the automatic judgement processing actually performed. For example, when the shape judgement processing is performed and the judgement result is proper, the letter "waiting approval (shape)" is displayed; when the counting judgement processing is performed and the judgement result is proper, the letter "waiting approval (counting)" is displayed. Here, when the image judgement processing is performed and the judgement result is proper, the letter "waiting approval (image)" may be displayed. Furthermore, it is contemplated that the letters displayed on the judgement result region A313 are the same with each other and the background colors of the judgement result region A313, which acknowledges that the judgement result is proper, are changed dependent on the categories of the automatic judgement processing.

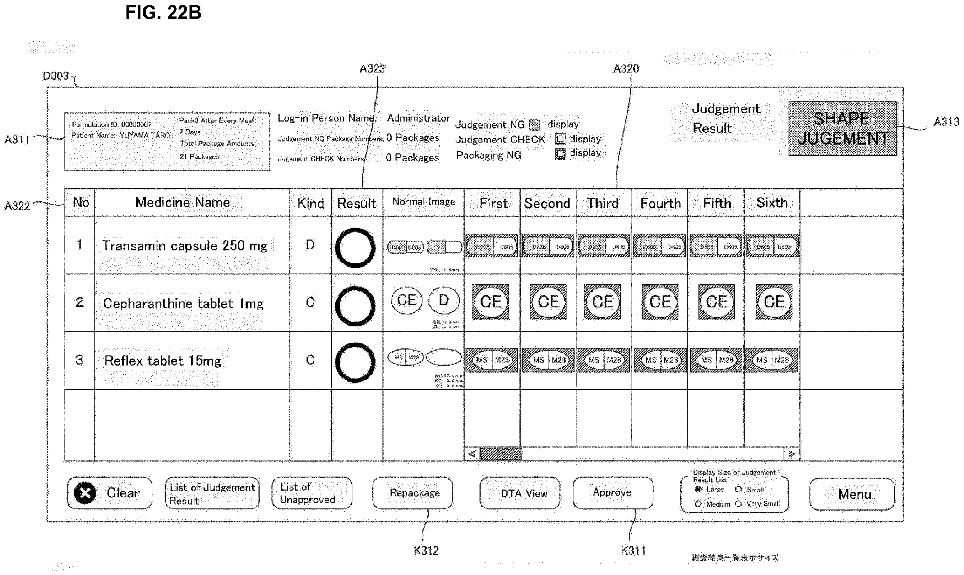

Furthermore, as shown in Fig. 22B, the image judgement processing is performed to a part of the tablets and the shape judgement processing is performed to another part of the tablets, and furthermore the judgement results thereof are proper altogether, the letter "shape judgement" is displayed on the judgement result region A313 and the background of the display region corresponding to the tablets subjected to the shape judgement processing may be displayed in the color arrangement determined beforehand.

Furthermore, it is contemplated that the controller part 21 displays, when only the judgement result of the shape judgement processing is error, the letter "NG (shape)" on the judgement result region A313 and when only the judgement result of the counting judgement processing is error, the letter "NG (counting)" on the judgement result region A313. Furthermore, it is contemplated that the letters displayed on the judgement result region A313 are the same with each other and the background colors of the judgement result region A313, which indicates that the judgement result is error, are changed dependent on the categories of the automatic judgement processing.

Furthermore, when a plurality of states such as the error or the check-required occurs at the same time in the result of the judgement for every administration timing in the formulation data, it is contemplated that the controller part 21 displays the state with higher priority on the judgement result region A313 by selecting the state according to priority conditions determined beforehand for each state. For example, when the error and the check-required occur as the judgement result, the error is given priority to others and the letter "NG" is displayed. Besides, in the automatic judgement processing, when a plurality of categories for the judgement processing is performed, the same will be applied. For example, the check-required (image) occurs in the judgement result of the image judgement processing and the check-required (shape) or the check-required (counting) occurs in the shape judgement processing or in the counting judgement processing, the image judgement processing is given priority and the letter "CHECK (image)" corresponding to the check-required (image) is displayed on the judgement result region A313.

Furthermore, in the judgement detail screen D303, an operation key K313 is displayed. The operation key K312 is the operation key for starting repackaging operation in which the dispensing operation about the administration timing corresponding one or a plurality of medicine packages 451 is reperformed. When a reissuing key K312 is operated, the controller part 21 displays a reissue operation screen D304 for setting a method of reperforming the package processing. Here, Fig. 22 is a drawing illustrating one example of the reissuing operation screen D304.

As shown in Fig. 23, in the reissuing operation screen D304, as an object for reperforming of the package processing, it may be allowed to select "Whole package", "Only CHECK package", "Only NG package", "CHECK, NG package" and "Designated package". When the "Whole package" is selected, the whole of the medicine package 451 is set to be the objects and when "only CHECK package" is selected, only the medicine packages 451 of which judgement results are the check-required is set to be the objects (Check-required (image), Check-required (shape), and Check-required (counting). Besides, when "Only NG package" is selected, the medicine packages 451 of which judgement results are error are set to be the objects (error (image), error (shape) and error (counting); when "CHECK, NG package" is selected, the medicine packages 451 of which judgement results are error and the check-required are set to be the objects. Furthermore, in the "Designated package" may optionally designate the medicine package 451 to be the object for reperforming, and, for example, sequential designation or individual designation may be allowed. Here, when the medicine packages 451 to be the objects for reperforming the package processing are already designated in the other screen, the identification information such as the medicine package numbers of already designated medicine package 451 are displayed in an input field for the designated package.

Furthermore, in the reissuing operation screen D304, a reissuing key K315 for starting execution of the reissuing is displayed and the controller part 21, in response to the operation of the reissuing key K315, sends a control instruction including such as reperforming data for reperforming the package processing of the medicine package 451 designated and the reperforming instruction to the medicine dispensing apparatus 4. Here, related processing is executed by the re-execution processing part 213 of the controller part 21. Thereby, in the medicine dispensing apparatus 4, according to the control instruction, the package processing is reperformed about the administration timing corresponding to the designated medicine package 451 on the reissuing operation screen D304.

### <Step S39>

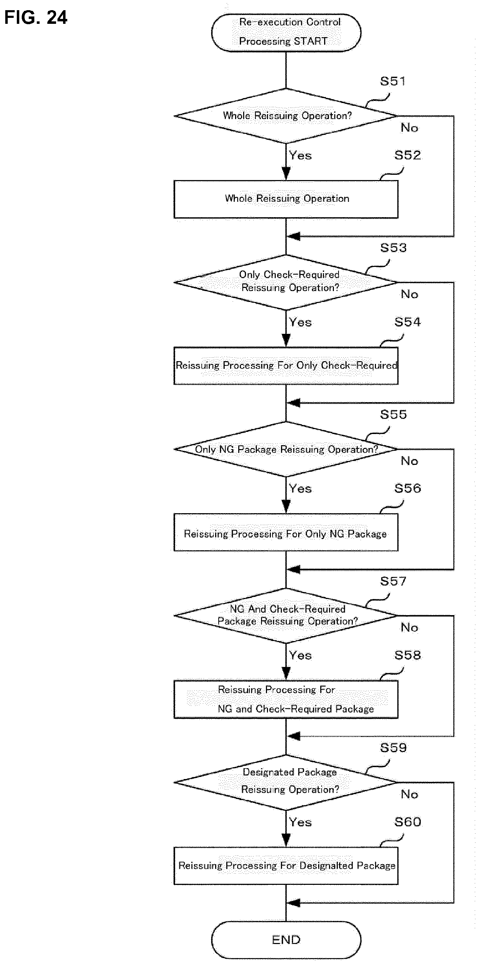

Thereafter, in the step S39, the controller part 21 executes the re-execution control processing for making the medicine dispensing apparatus 4 reperform a part or the whole of the package processing. Here, Fig. 24 is a drawing of one example of the re-execution control processing.

### [Re-execution control processing]

Here, with referring to Fig. 24, the re-execution control processing executed by the controller part 21 will be explained.

### <Step S51-S60>

In the step S51-S60, the processing for reperforming a part or the whole of the package processing executed in the medicine dispensing apparatus 4 with respect to the formulation data is executed. Here, the case that the package processing is reperformed in the just medicine dispensing apparatus 4 in which the package processing has been performed will be explained. On the other hand, it is contemplated that the controller part 21 makes in response to the user operation or automatically the other medicine dispensing apparatus 4 being different from the medicine dispensing apparatus 4 that the package processing was performed reperform the package processing with respect to the formulation data about one or plural administration timing. Thereby, since the package processing is reperformed by the different medicine dispensing apparatus 4, the occurrence of the error or the check-required may be avoided in the package processing. Here, the controller part 510 may execute a part or the whole of the package processing in response to the user operation in the medicine dispensing apparatus 4. In this case, the judgement detail screen D303 and the reissuing operation screen D304 are also displayed on the monitor 530 and the controller part 510 re-executes the package processing about one or a plurality of administration timings in response to the reperforming operation from the user to the monitor 530.

### <Step S51>

In the step S51, the controller part 21 determines whether or not the whole reissuing operation is done in the reissuing operation screen D304. More particularly, the controller part 21, in the reissuing operation screen D304, when the letter "whole package" is selected and then the operation key K315 is operated, determines that the whole reissuing operation is done. Here, when the controller part 21 determines that the whole reissuing operation is done (S51 :Yes), the processing is passed to the step S52 and when the whole reissuing operation is not done, (S51: No), the processing is passed to the step S53.

### <Step S52>

In the step S52, the controller part 21 executes the reissuing processing for sending a control instruction for which the package processing corresponding to the formulation data currently to be the judgement object is made entirely to reperform to the medicine dispensing apparatus 4 which performed the package processing. For example, as the control instruction, the formulation data is input again to the medicine dispensing apparatus 4. Thereby, in the medicine dispensing apparatus 4 the controller part 510 re-executes the package processing about all of the administration timing corresponding to the formulation data.

Besides, the controller part 510 of the medicine dispensing apparatus 4, as described above, executes the second photographing step and the second storing step. That is to say, the controller part 510, in the package processing being re-executed with respect to the formulation data, makes the camera 462 and the camera 463 take photographs of each tablet to be the dispensing object and stores them in association to the formulation data. Particularly, the controller part 510 stores the second photographed image each corresponding to the administration timing within the package processing in association to each of the administration timing. Furthermore, the controller part 510 executes, based on the second photographed images and the formulation data, the automatic judgement processing for determining the propriety of the package processing for each administration timing and stores the judgement results of the automatic judgement processing in association to the formulation data. Specifically, the controller part 510 stores the judgement results of the automatic judgement processing for every administration timing in association to each of the administration timing.

### <Step S53>

In the step S53, the controller part 21 determines whether or not the reissuing operation only to the medicine packages 451 occurring the check-required (hereinbelow, referred to "check-required package" in the reissuing operation screen D304 is done. More particularly, the controller part 21, in the reissuing operation screen D304, when the "Only CHECK package" is selected and then the operation key K315 is operated, determines that the reissuing operation only for the check-required package is done. Now, the controller part 21, when the reissuing operation only for the check-required package is done (S53: Yes), passes the processing to the step S54 and when the reissuing operation only for the check-required package is not done (S53: No), the controller 21 passes the processing to the step S55.

### <Step S54>

In the step S54, the controller part 21 executes the reissuing processing for sending the control instruction to the medicine dispensing apparatus 4 with which only the package processing occurring the check-required corresponding to the administration timing among the package processing corresponding to the formulation data currently to be the judgement object is made to reperform. For example, as the control instruction, the formulation data is input again to the medicine dispensing apparatus 4. Thereby, in the medicine dispensing apparatus 4, the controller part 510 reperforms the package processing about only a part of the administration timing occurring the check-required.

Now, the administration timing occurring the check-required to be the object for the reissuing processing is the administration timing in that any one of the judgement results in the image judgement processing, the shape judgement processing or the counting judgement processing is to be check-required. Furthermore, in the reissuing operation screen D304, it is contemplated that the image judgement processing, the shape judgement processing, and the counting judgement processing are able to be selected individually, and in this case, the administration timing in which the judgement result of selected any one or a plurality of judgement processings of the image judgement processing, the shape judgement processing or the counting judgement processing is the check-required becomes the object for the reissuing processing.

### <Step S55>

In the step S55, the controller part 21 determines whether or not the reissuing operation only to the medicine packages 451 occurring the NG package (hereinbelow, referred to "NG package" in the reissuing operation screen D304 is done. More particularly, the controller part 21, in the reissuing operation screen D304, when the "only NG package" is selected and then the operation key K315 is operated, determines that the reissuing operation only for the NG package is done. Now, the controller part 21, when the reissuing operation only for the NG package is done (S55: Yes), passes the processing to the step S56 and when the reissuing operation only for the NG package is not done (S55: No), the controller 21 passes the processing to the step S57.

### <Step S56>

In the step S56, the controller part 21 executes the reissuing processing for sending the control instruction to the medicine dispensing apparatus 4 for which only the package processing occurring the error corresponding to the administration timing among the package processing corresponding to the formulation data currently to be the judgement object is made to reperform. Thereby, in the medicine dispensing apparatus 4 the controller part 510 reperforms the package processing about only the package processing about a part of the administration timing occurring the error.

Now, the administration timing occurring the error to be the object for the reissuing processing is the administration timing in that any one of the judgement results in the image judgement processing, the shape judgement processing or the counting judgement processing is to be error. Furthermore, in the reissuing operation screen D304, it is contemplated that the image judgement processing, the shape judgement processing and the counting judgement processing are able to be selected individually, and in this case, the administration timing in which the judgement result of selected from any one or a plurality of judgement processing of the image judgement processing, the shape judgement processing or the counting judgement processing is error becomes the object for the reissuing processing.

### <Step S57>

In the step S57, the controller part 21 determines whether or not the reissuing operation for the NG package and the check-required package in the reissuing operation screen D304 is done. More particularly, the controller part 21, in the reissuing operation screen D304, when the "CHECK, NG package" is selected and then the operation key K315 is operated, determines that the reissuing operation only for the NG package is done. Now, the controller part 21, when the reissuing operation only for the NG package is done (S57: Yes), passes the processing to the step S58 and when the reissuing operation only for the NG package is not done (S57: No), the controller 21 passes the processing to the step S59.

### <Step S58>

In the step S58, the controller part 21 executes the reissuing processing for sending the control instruction to the medicine dispensing apparatus 4 for which only the package processing occurring the error or the check-required corresponding to the administration timing among the package processing corresponding to the formulation data currently to be the judgement object is made to reperform. Thereby, in the medicine dispensing apparatus 4 the controller part 510 reperforms the package processing about only a part of the administration timing occurring the error or the check-required.

### <Step S59>

In the step S59, the controller part 21 determines whether or not the reissuing operation for the designated package in the reissuing operation screen D304 is done. More particularly, the controller part 21 in the reissuing operation screen D304, when the "designated package" is selected and then the operation key K315 is operated, determines that the reissuing operation only for the designated package is done. Now, the controller part 21, when the reissuing operation only for the designated package is done (S59: Yes), passes the processing to the step S40 and when the reissuing operation only for the designated package is not done (S59: No), the controller 21 passes the processing to the step S60.

### <Step S60>

The controller part 21 executes the reissuing processing for sending the control instruction to the medicine dispensing apparatus 4 for which only the package processing for the designated administration timing corresponding to the administration timing among the package processing corresponding to the formulation data currently to be the judgement object is made to reperform. Thereby, in the medicine dispensing apparatus 4 the controller part 510 reperforms the package processing about only the package processing about the designated administration timing.

### <Step S28>

Now, return to the explanation of Fig. 7, in the step S28, the controller 21 determines whether or not the approval operation is done about the results of the automatic judgement processing with respect to the formulation data. Particularly, the controller part 21 in the judgement detail screen D303 determines whether or not the approval key K311 is operated. Here, when the controller part 21 determines that the approval operation is done (S28: Yes), the processing is passed to the step S29, and when the approval operation is not done (S29: No), the processing is passed to the step S30.

### <Step S29>

In the step S29, the controller part 21 executes the approval processing for confirming the results of the automatic judgement processing of the formulation data. For example, in the approval processing, the controller part 21 stores in the storage part 22 the approval of the result of the automatic judgement processing of the formulation data and the identification information of the user (name or ID) made the approval operation, i.e., the pharmacist together with the results of the automatic judgement processing in association to the formulation data.

Particularly, the controller part 21, when the approval key K311 is operated on the judgement detail screen D303, receives the approval of the judgement results of the automatic judgement processing and stores them in association to the formulation data. In addition, the controller part 21, as shown in Fig. 12B, makes the judgement result region A313 in the judgement detail screen D303 display the letter "Approval OK" as the judgement results of the automatic judgement.

### <Step S30>

In the step S30, the controller part 21 determines whether or not a judgement completion operation for terminating the judgement supporting processing is done. For example, the controller part 21 determines that the judgement completion operation is done when an operation key corresponding to "completion" in the list screen D1 is operated. Here, the controller part 21, when determining that the judgement completion operation is done (S30: Yes), terminates the judgement supporting processing and when the judgement completion operation is not done, the processing is reverted to the step S22.

Hereinbelow, other functions having the medicine dispensing apparatus 4 will be described.

### [Shifted-back detection function]

As described earlier, in the medicine dispensing apparatus 4, based on the formulation data, the package processing for packaging one or a plurality of tablets into the medicine package 451 for every administration timing is performed in the packaging unit 504. In this time, there are fears of a shifted-back defect that at least a part of the charged tablets to the medicine package 451 in the package processing moves to the next medicine package 451 and is packaged therein. To addressing this, the controller part 510 of the medicine dispensing apparatus 4 has the shifted-back defect detection function for detecting the shifted-back defect.

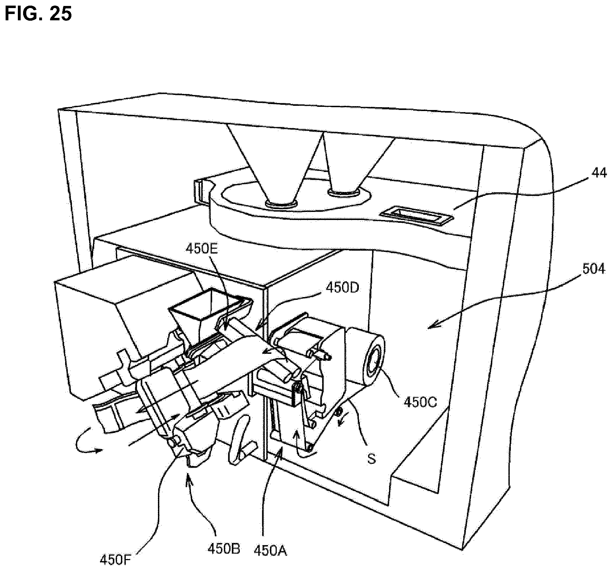

Particularly, in the medicine dispensing apparatus 4, a detector part 476 for detecting whether or not the tablet to be contained in the medicine package 451 is present before enclosing the medicine package 451 at an upstream side of the medicine package 451 to be enclosed. In addition, when the presence is detected at the upstream side by the detection part 476, the controller part 510 detects occurrence of the shifted-back defect with respect to the first medical package 451 (corresponding to the first wrapping material) and the next medicine package 451 (corresponding to the second wrapping material), since the tablet to be charged to the medicine package 451 should be enclosed in the next medicine package 451. Here, the controller part 510 when executing processing for detecting the shifted-back defect is one example for a shifted-back detention part. Hereinbelow, with referring to Fig. 25-Fig. 39, the construction in relation to the shifted-back detection function will be described.

As shown in Fig. 25, the packaging unit 504 is disposed below the rotation unit 44 and may package the tablets dispensed from the rotation unit 44. The packaging unit 504 comprises a dispensing paper supply part 450A and a wrapping mechanism 450B. The dispensing paper supply part 450A is a mechanism for drawing out the dispensing paper S wound about a roller axis 450C and for sending it to the wrapping mechanism 450B. The dispensing paper S is a thermal bonding sheet and is wound about the roller axis 450C with the state being twice folded along to a shorter direction. The wrapping mechanism 450B comprises a sheet support part 450D, a guide member 450E and a sealing apparatus 450F. The wrapping mechanism 450B may package the medicine M supplied from the rotation unit side 44 by bonding under pressure the dispensing paper S sent from the dispensing paler supply unit 450A to form a bag-like state.

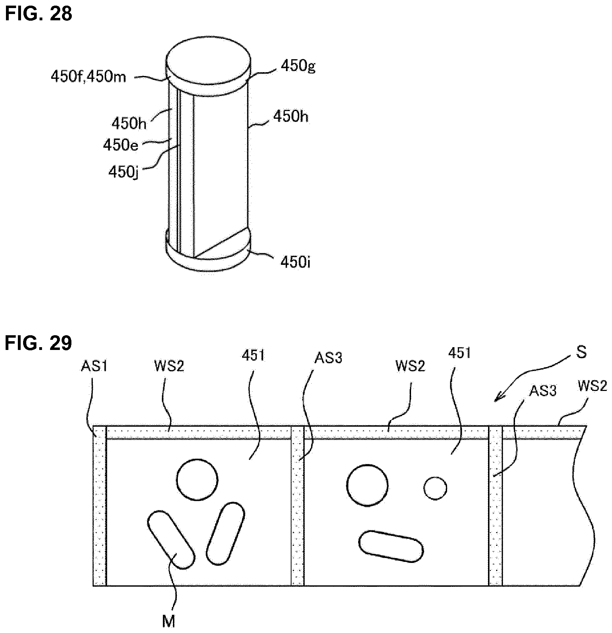

Further particularly, the guide member 450E has a function as a guide for guiding the dispensing paper S sent from the dispensing paper supply part 42. The seal apparatus 450F may form a half bag-like state by bonding under pressure a position such as one end of a longitudinal direction (downstream side) side and the like supplied and guided by the guide member 44b and may form a bag-like state by bonding and closing under pressure an open side of the half bag-like dispensing paper S. Further particularly, by bonding under pressure the dispensing paper S by the sealing apparatus 450F, the medicine package 451 containing the medicine M may be formed as shown in Fig. 29. The sealing apparatus 450F forms a vertical seal for closing the part of the dispensing paper S at the downstream side along to a feed direction and along to the shorter direction (a first vertical seal AS1 or a second vertical seal AS3) while forming a lateral seal WS2. Thereby, the dispensing paper S with the half-bag like state having the opening part at the upstream side part of the feeding direction of the dispensing paper S (medical package 451) is formed. In this state, the medicine M is charged into the dispensing paper S having the half-bag like state (medical package 451) and then, the opened part is closed by the sealing apparatus 450F. That is to say, when a part of the lateral seal WS2 is unclosed, the unclosed part is closed by the sealing apparatus 450F and the vertical seal (the second seal AS3), which closes along to the shorter direction of the dispensing paper S at the upstream side along to the feeding direction of the dispensing paper S, is formed to enclose thereof.

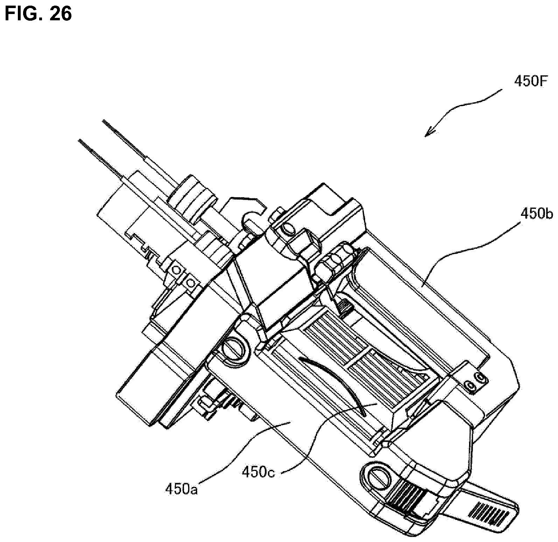

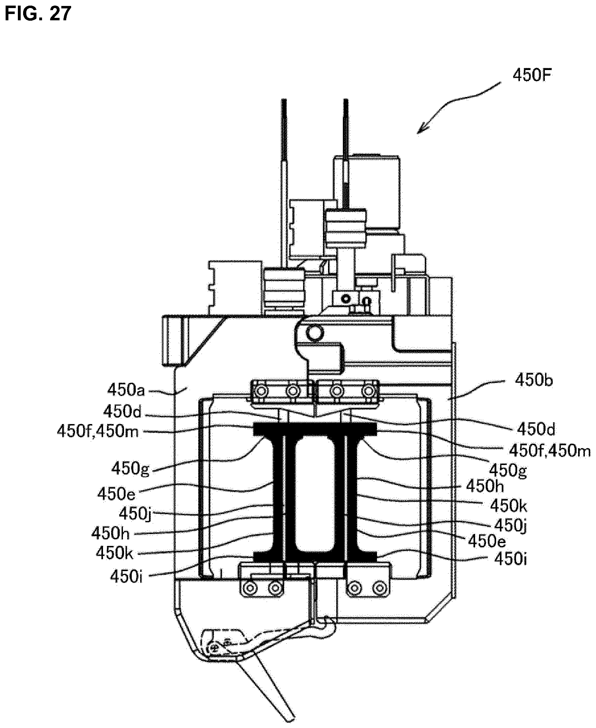

As shown in Fig. 26, a main part of the sealing apparatus 450F is composed from a pair of roller frames 450a, 450b. The sealing apparatus 450F is disposed with a protection cover 450c at the roller frame 450c side; however, in the state that the protection cover 450c is removed, as shown in Fig. 27, the roller frames 450a, 450b are almost symmetry in right and left in the state that they are contacted oppositely each other.

As shown in Fig. 27 and the like, the roller frames 450a, 450b are constructed by frames made from metal having an almost channel shape (gate shape) in a plane view. To the roller frames 450a, 450b, supporting axes 450d extending to an up-and-down direction are disposed and the vertical seal member 450e and the lateral seal member 450f are attached thereto. The vertical seal member 450e and the lateral seal member 450f are each attached rotatably to the supporting axes 450d. In addition, the vertical seal member 450e and the lateral seal member 450f are each connected to separate power supplies (not shown in the figure) through separate power transfer mechanisms (not shown in the figure) and are rotatable independently each other so that by changing a rotation speed of the vertical sealing member 450e and a rotation speed of the lateral sealing member 450f a bag length of the medicine package 451 may be changed.

The vertical sealing member 450e is made from metal and as shown in Fig. 27 has an almost linear shape. The vertical sealing member 450e has, as shown in Fig. 28, a lower end 450i having a disk shape and a heater part 450k having a plate shape. The heater part 450k is positioned between an upper end part 450g forming a lateral sealing member 450f described later and the lower end part 450i and is almost vertical to both. At lateral sides of the heater part 450k, a heater 450h and a cut-off line forming part 450j are disposed in line from the upper end parts 450g side to the lower end part 450i side. The heaters 450h, 450h are ones that are able to thermally bond the dispensing paper S. Thus, by rotating the vertical sealing members 450e, 450e positioned parallel and by passing the dispensing paper S in two-folded state between both, the seal (vertical seal) extending to the shorter direction of the dispensing paper S may be formed.

Furthermore, the cut-off line forming part 450j is one that may form perforations to the second vertical sealing AS3 of the dispensing paper S. In the present embodiment, the cut-offline forming part 450j at the roller frame 450b side is formed by a cutter for forming the perforations and the cut-off line forming part at the roller frame 450a side is formed from a blade receiver disposed corresponding to the cutter.

As shown in Fig. 27, the lateral sealing member 450f comprises the upper end parts 450g described above and the heater 450m. The upper end part 450g is a disk-shaped member disposed at the upper side of the heater part 450k of the vertical sealing member 450e. At the outer periphery of the upper end part 450g, the heater 450m is disposed around the whole periphery thereof. Thus, by rotating the lateral sealing members 450f, 450f disposed parallel and by passing the two-folded dispensing paper S between the upper end parts 450g, 450g, the sealing extending to the longitudinal direction of the dispensing paper S (vertical seal) may be formed.

As shown in Fig. 27, in the sealing apparatus 450F, within almost "square" shaped (rectangular) region enclosed by the roller frames 450a, 450b, the vertical sealing members 450e, 450e and the lateral sealing members 450f, 450f are disposed almost parallel with predetermined clearances. The sealing apparatus 450F may make the vertical sealing members 450e, 450e and the lateral sealing members 450f, 450f rotate to pass the dispensing paper S between these clearances and to form the lateral seal and the vertical seal, thereby it can form the medical package 451.

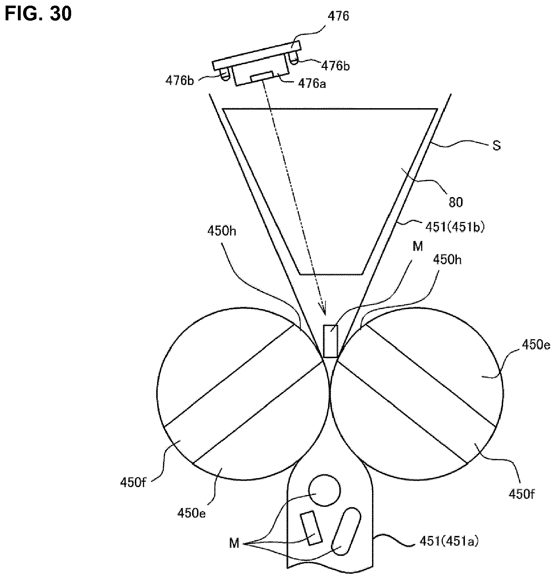

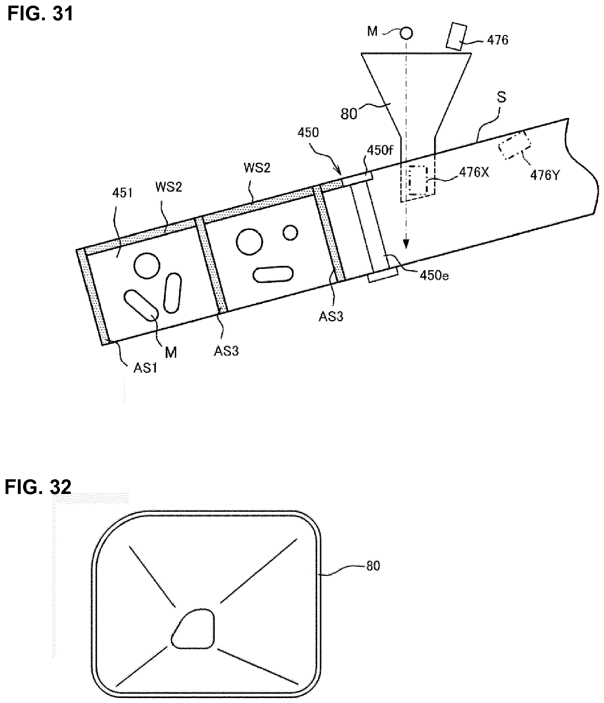

As shown in Fig. 28, to the rotation unit 44, a medicine introduction part 80 for supplying the tablet M dispensed individually from the rotation unit 44 to the packaging unit 504 is disposed. Any medicine introduction part 80 may be used so far as it may supply the tablet M into the dispensing paper S, in the present embodiment, it is constructed by a hopper. As shown in Fig. 30, 31, the medicine introduction part 80 is inserted by the sealing apparatus 450F into the opened part of the medicine package 451 in a non-closed state formed by the dispensing paper S and may introduce the tablet M into the medical package 451. Particularly, the medicine introduction part 80 is placed such that a base end part faces to the rotation unit 44 side and a tip part is inserted to the medical package 451 in the unenclosed state under the formation process with the sealing apparatus 450F. That is to say, the medicine introduction part 80 is inserted into the inside of the two-folded dispensing paper S at the upstream side of the feeding direction of the dispensing paper S with respect to the sealing apparatus 450F. Here, although omitting illustration, as described above, the pass-through detection sensor 474 is disposed at a lower position from a lower end of the medicine introduction part 80 and may detect the tablet M falling down into the dispensing paper S from the medicine introduction part 80.

Furthermore, as shown in Fig. 30 and Fig. 31, the detection part 476 is disposed to the packaging unit 504 for detecting the presence of the tablet M in an introduction path of the tablet M with the medicine introduction part 80. As shown in Fig. 30, the detection part 476 comprises a camera 476a being able to take photographs of the introduction path of the tablet M with the medicine introduction part 80 and a lighting device 476b. The camera 476a is placed such that the inside of the dispensing paper S at an upstream side from the sealing apparatus 450F along to the feeding direction of the dispensing paper S may be photographed (detection). In the present embodiment, the camera 476a is arranged to face the tip-part side from the base end part of the medicine introduction part 80. In addition, as described above, the medicine introduction part 80 is positioned at the upstream side from the sealing apparatus 450F. Thus, the camera 476a is arranged so that it may take the photographs (detection) of regions at the upstream side from the sealing apparatus 450F along to the feeding direction of the dispensing paper S. Furthermore, the lighting apparatus 476b comprises a light source such as a light emitting diode or a light bulb and the like. The lighting apparatus 476b is arranged likely to the camera 476a to face the tip-part side from the base end part of the medicine introduction part 80 such that it may project the inside region of the medicine introduction part 80. Here, the controller part 510 stores wrapping photograph images by the camera 476a in the storage part 22 and sends them to the judgement supporting apparatus 2.

Furthermore, in the medicine dispensing apparatus 4, the controller part 510, based on detection data input from the detection part 476, may determine occurrence of defected dispensing due to leak-out from the medicine package into which the medicine M should be packaged.

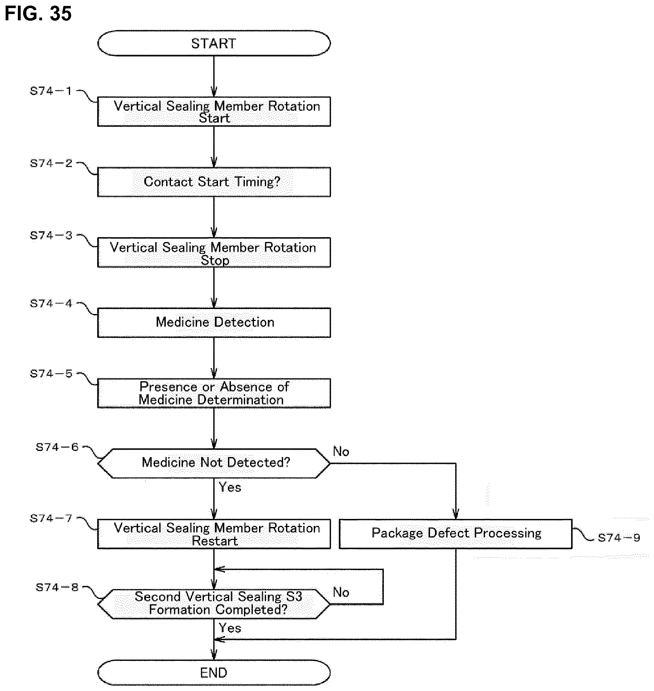

Next, a formation method of the medicine package 451 by the sealing apparatus 450F and a determination method for the defected packaging under the formation process of the medicine package 451 performed in the medicine dispensing apparatus 4 will be described. Now, in the description hereunder, first the method for forming the medicine package 451 will be briefly described, and thereafter, will be explained based on Fig. 35 a subroutine relating to formation steps of the second vertical seal.

### [With respect to formation method of medicine package 451]

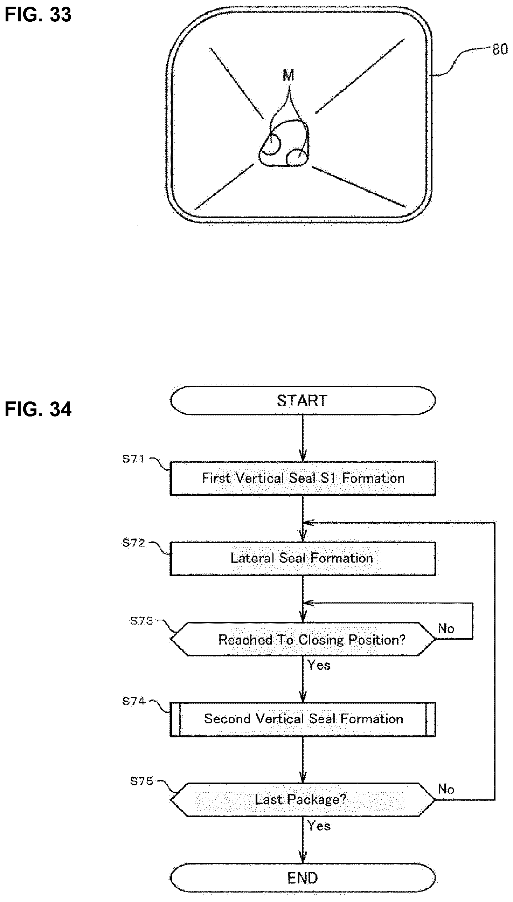

The controller part 510 forms the medicine package 451 in accordance with a control flow shown in Fig. 34. Hereunder, practical movement and control will be described according to Fig. 34.

### <Step S71>

When the medicine package 451 is formed, first in the step S71 the vertical seal (hereafter also referred to "first vertical seal AS1") for closing the downstream end of the medicine package 451 is formed by the vertical searing members 450e, 450e at a top position along to the feeding direction of the dispensing paper S (refer to Fig. 29). Then the control flow will be proceeded to the step S72.

### <Step S72>

In the step S72, the lateral seal WS2 (refer to Fig. 29) is formed for closing the end opposite to the fold of the dispensing paper S supplied in two-folded state. Particularly, the lateral seal is formed by rotating the lateral sealing members 450f, 450f and by passing the dispensing paper S between both.

### <Step S73>

In the step S73, it is confirmed whether or not the lateral seal WS2 is formed to reach to the necessary position for closing the medicine package 451 (closing position). When it is determined that the lateral seal WS2 reaches to the closing position (the step S73: Yes), the control flow is proceeded to the step S74, and when it does not reach to the closing position (the step S73: No), the control flow is reverted to the step S72.

### <Step S74>

In the step S74, the vertical seal for closing the end at the upstream side along to the feeding direction of the dispensing paper S in the medicine package 451 (hereunder, also referred to "second seal AS3") is formed in accordance with the subroutine of Fig. 35 described in detail hereinafter. Here, the second vertical seal AS3 is also functions as the first vertical seal AS1 of the medicine package 451 which will be formed just next. Thus, the second vertical seal AS3 functions as the seal for forming a border of the medicine package 451 formed continuously along to the longitudinal direction of the dispensing paper S. When the second vertical seal AS3 is formed, the control flow will be proceeded to the step S75.

In the step S75, it is confirmed whether or not the medicine package 451 enclosed by the second vertical seal AS3 in the step S74 is the last one. When the medicine package enclosed in the step S74 is not the last one (the step S75: NO), the control flow is reverted to the step S72 and when it is the last one (the step S75: YES), the sequential control flow will be terminated.

### [With respect to forming steps of second vertical seal]

Next, a subroutine for forming steps of the second vertical seal relating to the above described step S74 will be described in detail with referring to Fig. 35.

### <Step S74-1>

In the step S74-1, in order to form the second vertical seal AS3, the rotation of the heater parts 450k, 450k are made to started such that the heater parts 450k, 450k become to the positional relation opposite each other. Then, the control flow is proceeded to the step S74-2.

### <Step S74-2>

In the step S74-2, as shown in Fig. 27 and Fig. 28, it is confirmed whether or not the heater parts 450k, 450k of the vertical sealing members 450e, 450e reach to a timing for just staring contact with the dispensing paper S (contact starting point). Here, there are many methods for confirming whether or not the contact start timing has come. Particularly, for example, it is contemplated that in the step S74-1, there is a method in which a timer is disposed for starting the clocking from the timing when the rotation of the vertical sealing members 450e,450e and the approval is performed by passage of a predetermined time by the timer; there is a method in which a rotation detection device that can detect rotation amounts of the vertical seal members 450e, 450e is disposed and the approval is performed by checking that detected rotation amounts reach to the predetermined amount; or there is a method in which a detection device that can detect angles or postures of the vertical sealing members 450e, 450e of the heater part 450k, 450k is disposed and the confirmation is performed by determining whether or not the vertical sealing members 450k, 450k of the heater part 450k, 450k become the angles or postures for starting the contact and the like. By these methods, when it is confirmed that it is to be the contact start timing (the step S74-2: YES), the control flow will be proceeded to the step S74-3, and when it is not confirmed (the step S74-3: NO), the control flow is kept at the step S74-2 as is.

### <Step S74-3>

In the step S74-3, a control is performed for stopping the rotation of the vertical sealing members 450e, 450e temporarily. Thereby, the vertical sealing members 450e, 450e become to the state being temporarily stopped in the postures just contacting with the heater part 450k, 450k. Then, the control flow will be proceeded to the step S74-4.

### <Step S74-4>

In the step S74-4, presence or absence of the tablet M is detected by the sensor part 476 in the inside region of the medicine introduction part 80 and the region inside of the dispensing paper S and also at the upstream side along to the feeding direction from the vertical seal (the first seal AS1 or the second vertical seal AS3) formed already by the sealing apparatus 450F (packaging detection). In this case, the lighting device 476b is turned on and the inside region of the medicine introduction part 80 is lit up. The detection data by the detection part 476 is input to the controller part 510. Any detection data may be used so far as it is effective for determining presence or absence of the tablet M and in the present embodiment, the image data of the wrapping photographed image taken by the camera 476a are input to the controller part 510 as the detection data. Particularly, when the tablet M is not present, as shown in Fig. 32, the wrapping photograph image only of the medicine introduction part 80 is taken and when the tablet M is present, as shown in Fig. 33, the wrapping photograph image with the tablet M is taken. Such image data of the wrapping photograph images are input to the controller part 510 as the detection data. Then, the control flow will be proceeded to the step S74-5.

### <Step S74-5>

In the step S74-5, by the controller part 510, the determination for presence or absence of the tablet M is performed based on the detection data (image data) obtained by the packaging detection performed at the step S74-4. In the present embodiment, because the image data are obtained as the detection data, the determination may be performed about presence and absence of the tablet M by the methods such as image analysis utilizing the image data. Although any method may be used for the determination of presence or absence of the tablet M may be used, for example, the determination of presence or absence of the tablet M may be performed by preparing the wrapping photograph image obtained by the camera 476a under the condition that the tablet M is not present as a master image and by using the wrapping photograph image actually taken by the camera 476a and the master image. Now, when the determination is made by using the master image as described above and when the dispensing paper S is photographed in the wrapping photographed image taken by the camera 476a, it may be desired that different master images may be prepared in response to the kinds of the dispensing paper S. Particularly, although the dispensing paper S is supplied in the state with overlapping two faces by being folded twice, there are dispensing papers S with both faces being transparent or with one face being transparent while having an opaque part on the other face (for example, a band with a color such as white) and the like. Depending on the cases that the former one is used as the dispensing paper S or that one having the opaque part as the latter one is used as the dispensing paper S, it is obvious that difference between the wrapping photographed images taken by the camera 476a occurs. Therefore, for addressing to the difference of the dispensing papers S, it is desirable to prepare the master images depending on the kinds of the dispensing paper S. After the determination about the tablet M is performed as described above, the control step is proceeded to the step S74-6.

### <Step S74-6>

In the step S74-6, the confirmation is made whether or not the tablet M is detected as the result of the determination in the step S74-5. Here, when the tablet M is not detected (the step S74-6: YES), it is considered that the tablet M to be dispensed does not leak out from the medicine package 451. In this case, the determination is made that the dispensation of the tablet M is performed normally and the control flow is proceeded to the step S74-7. On the other hand, when the tablet M is detected (the step S74-6: NO), as shown in Fig. 30, there is high possibility for the occurrence of the shifted-back defect in which the tablet M to be packaged in the former medicine package 451 (the medicine package 451a at the lower side in the figure, one example of the first wrapping material) was leaked out from the medicine package 451 and moved to the medicine package 451 to be formed later (the medicine package 451b under the formation thereof at the upper side in the figure, one example for the second wrapping material). In this case, the determination that the shifted-back defect has occur and the control flow is proceeded to the step S74-9.

Particularly, it is contemplated that the controller 510 determines, when the tablet M in the next medicine package 451b is detected and the tablet M to be charged to the next medicine package 451b is not detected by the pass-through detection sensor 474, the determination for the occurrence of the shifted-back defect is made. That is to say, it is contemplated that the controller part 510 detects presence or absence of the shifted-back defect using not only the detection results of the detection part 476 but also using the detection result of the pass-through sensor 474. Thereby, the controller 510 may detect the shifted-back defect with distinguishing from a foreign matter mix defect in which, for example, foreign matters pass through the pass-through sensor 474 and are mixed into the medicine paper 451.

### <Step S74-7>

In the step S74-7, the control for restarting the rotation of the vertical seal members 450e, 450e temporarily stopped at the step S74-3 is executed. Thereby, the vertical sealing members 450e, 450e begin to contact with the surfaces of the heater part 450k, 450k to form the second vertical seal AS3.

### <Step S74-8>

In the step S74-8, the confirmation is made whether or not the formation of the second vertical seal AS3 is completed. When the determination is made that the formation of the second vertical seal AS3 is completed (the step S74-8:YES), the sequential control flow is completed. On the other hand, when the determination is made that the formation of the second vertical seal AS3 is not completed (the step S74-8: YES), the control of the step S74-8 are kept continued.

### <Step S74-9>

In the step S74-9, the processing for addressing to the fact that leakage of the medicine M is confirmed at the step S74-6 (packaging defect processing) is performed.

Particularly, the controller part 510 determines that the shifted-back defect has occurred with respect to the medicine package 451 in which the tablet M is detected by the determination at the step S74-5 and it is determined that the shifted-back defect has also occurred in the medicine package 451 one package before the present medicine package 451. For example, when the medicine M has been detected about the fourth medicine package 451 by the determination at the step S74-5, it is determined that the shifted-back defects have occurred in the third medicine package 451 (corresponding to the first wrapping material) and the fourth medicine package 451 (corresponding to the second wrapping material). Here, the controller part 510, when executing the related determination processing is one example for a determination processing part. Besides, the controller part 510, in the step S74-9, or after the completion of the package processing with respect to the formulation data, stores the acknowledgement for occurrence of the shifted-back defects for each of the medicine packages 451 as the judgement result of the automatic judgement processing in the storage part 520 and sends it to the judgement supporting apparatus 2.

As described above, in the medicine dispensing apparatus 4 of the present embodiment, it is determined that the packaging defect has occurred using the condition in that the presence of the tablet M is detected in the duration after the timing for starting the enclosure of the medicine package 451 introduced with the tablet M and before the timing for introducing the tablet M to be packaged in the next medicine package 451. By setting as described above, the packaging defect caused by leakage of the tablet M from the medicine package 451 normally to be packaged such that labor required for the judgement may be suppressed to the minimum extent.

In addition, in the above described medicine dispensing apparatus 4, the contact timing of the vertical sealing member 450e with the dispensing paper S for forming the second vertical seal AS3 is set to the start timing for enclosing the medicine package 451 to which the tablet M is introduced therein and at this timing, the bonding of the dispensing paper S is stopped temporarily to detect the tablet M by the detection part 476. Thereby, the packaging defect caused by the leakage of the tablet M when enclosing the medicine package 451 may be detected with a further excellent precision. Furthermore, the defects, in which the tablet M leaked from the medicine package 451, is bitten between the vertical sealing members 450e, 405e and the like may be suppressed.

Here, in the present embodiment, the example in which the contact timing of the vertical sealing member 450e for forming the second vertical seal AS3 with the dispensing paper S is regarded as the start timing of enclosing the medicine package 451 and detects the tablet M by the detection part 476, has been illustrated, but the present invention is not limited thereto, the similar processing may be possible by regarding the other timing as the start timing for enclosing the medicine package 451. In addition, in the present embodiment, the tablet M is detected by the detector part 476 at the start timing for enclosing the medicine package 451, but the present invention is not limited thereto, the presence of the tablet M may be detected at any optional timing within the duration after the start timing for enclosing the medicine package 451 and before the completion of enclosing (for example, after enclosing). Furthermore, in the present embodiment, the example of stopping temporarily the sealing by the vertical sealing member 450e at the detection timing of the tablet M by the detection part 476 has been explained, but the present invention is not limited thereto. Particularly, it is contemplated that the sealing by the vertical sealing member 405e is not stopped when detecting presence or absence of the tablet M by the detection part 476, or a seal formation speed by the vertical sealing member 450e is decreased.

Here, in the present embodiment, the example of the sealing apparatus 450F with the roller shaped vertical sealing members 450e, 405e and the lateral sealing member 450f, 450f being independently controllable has been explained, but the present invention is not limited thereto. That is to say, when a bag length of the medicine package 451 may be a constant, the sealing apparatus 450F may be constructed as one that the vertical sealing members 450e, 450e, and the lateral sealing members 450f, 450f are driven integrally.

In the present embodiment, the example forming the medicine package 451 by sandwiching the dispensing paper S to seal with the roller shaped vertical sealing members 450e, 405e and with the lateral sealing member 450f, 450f has been explained, but the present invention is not limited thereto and the medicine package 451 may be formed by sealing the dispensing paper S with other configurations and methods.

Here, in the present embodiment, the example for forming the medicine package by applying the seal to the twice overlapped part of the two folded dispensing paper S has been explained, but the present invention is not limited thereto. Particularly, it may be one that two sheets of the dispensing paper S are supplied and are juxtaposed while sealing each other to form the medicine package 451.

Furthermore, in the present embodiment, one which comprises the camera 476a has been illustrated as the detection device for detecting the tablet M in the detection part 476, but the present invention is not limited thereto and any one may be used so far as it may detect the presence of the tablet M. Particularly, as the detection part 476, an optical sensor or an infra-red sensor and the like being able to detect presence or absence of the tablet M at the upstream side along to the feeding direction of the dispensing paper S may be disposed inside the dispensing paper S. Here, when the optical sensor and the like is used as the detection part 476, it may be desired that counter measures may be provided considering characteristics of these sensors such that sufficient detection accuracy may be obtained. Particularly, when a detection distance of the optical sensor and the like is set short, there are fears that the detection accuracy becomes low about small sized tablet M. Therefore, when the size of the tablet M is small, a distance from the optical sensor to the surface of the tablet M becomes larger than that in the case for the tablet M with larger size. Thus, when the detection distance of the optical sensor and the like is set short, there are fears that the detection accuracy for the small tablet M decreases. On the other hand, when the detection distance of the optical sensor and the like is set long, there are fears that the vertical sealing member 450e and the like is detected as the tablet M. Therefore, when the optical sensor and the like is adopted as the detection part, it is preferred to provide beforehand the counter measures such as setting the detection distance and the like considering the size of the tablet M handled.

Furthermore, the detection part 476 may be positioned at any position inside of the dispensing paper S so far as it may detect the presence of the tablet M at the upstream side from the sealing apparatus 450 along to the feeding direction of the dispensing paper S. Particularly, in Fig. 31 as shown by double chain lines, it may be one that the detection part 476X similar to the detection part 476 is disposed at the tip side of the medicine introduction part 80 or may be one that the detection part 476Y is disposed at further upstream side from the medicine introduction part 80.

Besides, for improving the detection accuracy of the exact packaging of the tablet M, other sensor may be disposed in addition to the detection part 476. Particularly, a falling down sensor for detecting the falling down of the tablet M and the like may be disposed to the medicine introduction part 80. When adopting the condition that the tablet M may be detected by the falling down sensor and the leakage of the tablet M is not detected based on the detection result by the detection part 476 is adopted, it may be more precisely detected whether or not the tablet M dispensed for the packaging use is exactly packaged.

In the present embodiment, while the example has been illustrated using the detection part 476 for detecting the medicine M being packaged exactly at the timing for enclosing of the medicine package 451; however, the detection part 476 may be used to the other usage. Particularly, the presence of the tablet M may be detected by the detection part 476 at the timing when the tablet M for packaging is just introduced into the dispensing paper S from the introduction part 80. That is to say, the detection part 476 may be used not only for enclosure detection also but for introduction detection of the tablet M into the dispensing paper S through the introduction part 80. When constructed as above, the detection part 476 may be used advantageously to conformation whether or not the tablet M is supplied to the dispensing paper S. Therefore, both of the fact that there is no leakage of the tablet M when enclosing of the dispensing paper S (medicine package formation) by the above enclosure detection and that fact that the tablet M is exactly supplied by the introduction detection can be confirmed. Only when both are confirmed, the determination may be made that the tablet M has been packaged exactly, thereby the determination accuracy for exact dispensing of the tablet M may be further improved.

In the present embodiment, the example detecting presence or absence of the tablet M by the detection part 476 at the timing when starting the formation of the second vertical seal AS3 has been illustrated, but the present invention is not limited thereto. That is to say, the tablet M may be detected by the detection part 476 in any timing within the duration after the timing when starting the enclosure of the medicine package 451 introduced with the tablet M and before the timing when introducing through the medicine introduction part 80 the tablet M to be packaged in the medicine package 451 to be formed next (hereunder, also referred to "detectible duration"). Particularly, the detection of the tablet M by the detection part 476 may be performed in the duration after the enclosure completion of the medicine package 451 introduced with the tablet M (after formation of the second vertical seal AS3) and before the timing for introduction of the tablet M to be packaged in the medicine package 451 to be formed next. Furthermore, it is contemplated that the detection by the detection part 476 may be performed not only at the predetermined timing within the above detectible duration but may also be performed continuously within the predetermined duration included in the above detectible duration or may be performed intermittently within the detectible duration.

In the present embodiment, the example of the sealing apparatus 450F has been illustrated as one that is possible to bond the dispensing paper S with sandwiching thereof by the roller shaped members consisted of the vertical sealing members 450e, 450e and the lateral sealing members 450f, 450f, but the present invention is not limited thereto, and one that bonds the dispensing paper S by the other method may be adopted instead of the sealing apparatus 450F. Particularly, one that a pair of plate-shaped heaters having a planer shape such as a T-shape and the like may be disposed and may be able to bond by sandwiching the dispensing paper S between the heaters together may be used instead of the sealing apparatus 450F of the present embodiment.

### [Shifted-back defect display function]

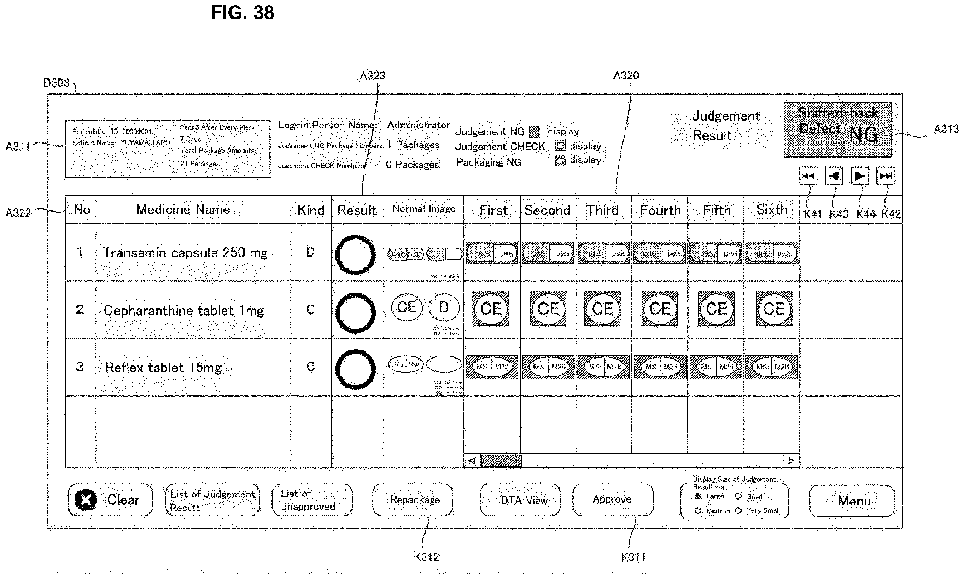

As described above, in the judgement supporting apparatus 1, when the shifted-back defect is detected by the shifted-back detection function, information of presence or absence of the shifted-back defect and the wrapping photograph images by the camera 476a is sent to the judgement supporting apparatus 2. Thereby, the controller 21 of the judgement supporting system 2 may display it on the judgement detail screen D303. Here, Fig. 38 is a drawing of one example of the judgement supporting screen D303.

As shown in Fig. 38, when the shifted-back defect is detected by the shifted-back detection function, the controller part 21 changes to the second specific color such as red and the like indicating the occurrence of the shifted-back defect as the background color of the display region A320 in a column corresponding to the administration timing of the medicine package 451 that the shifted-back defect occurred. Particularly, in Fig. 38, a display example when the wrapping has been performed with the tablet moved from the third medicine package 451 (corresponding to the first wrapping material) to the fourth medicine package 451 (corresponding to the second wrapping material) is shown. Furthermore, the controller part 21 displays the letter "shifted-back NG" with the background color of the second specific color acknowledging the occurrence of the shifted-back defect in the judgement result region A313. Here, because the tablet causing the shifted-back defect cannot be identified, in the judgement detail screen D303, it is contemplated that the display the mark "X" similar to the error or the mark "triangle" similar to the check-required is presented.

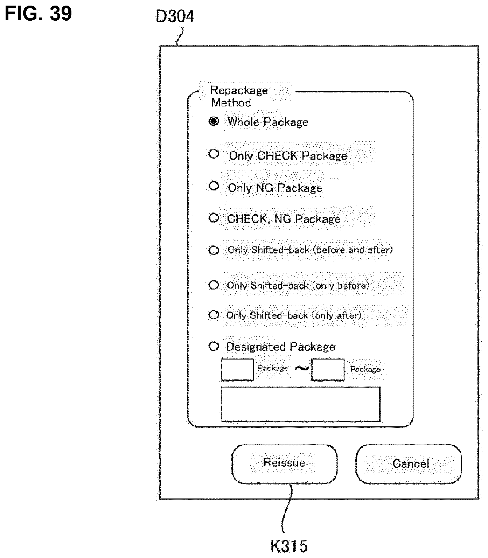

Furthermore, as described above, the controller part 21 displays, when the operation key K312 displayed on the judgement detail screen D303 is operated, the reissuing operation screen D304 (refer to Fig. 23). On the other hand, when the shifted-back defect occurs, it is contemplated that the controller part 21 displays the reissuing operation screen D304 shown in Fig. 39 when the operation key K312 is operated. Here, the controller part 21 may, in spite of presence or absence of the occurrence of the shifted-back defect, display the reissuing screen D304 shown in Fig. 39.

Particularly, the controller part 21 makes the reissuing operation screen D304 shown in Fig. 39 display selection operation parts corresponding to "Whole package", "Only CHECK package", "Only NG package", "CHECK, NG package", "Designated package" and in addition "Only Shifted-back (before and after)", "Only Shifted-back (only before)" and "Only Shifted-back (only after)". The controller part 21 upon executing the relating processing is one example of the operation display processing part. Here, in the reissuing operation screen D304, but not limited to the case in that all of "Only Shifted-back (before and after)", "Only Shifted-back (only before)", and "Only Shifted-back (only after)" are displayed altogether, and it is contemplated as another embodiment to display at least one or a plurality of them as another embodiment is displayed.

Here, the selection operation part corresponding to "Only shifted-back package (before and after)" is one example of a first re-execution operation part for receiving the operation for executing the package processing about both of two before and after medicine packages 451 in which the shifted-back defect is detected. The selection operation part corresponding to "Only shifted-back package (only before)" is one example of a first re-execution operation part for executing the package processing with respect to the before-medicine package 451 only about in two before and after medicine packages 451 in which the shifted-back defect is detected. The selection operation part corresponding to "Only shifted-back package (only after)" is one example of a first re-execution operation part for executing the package processing with respect to the after-medicine package 451 only about in two before and after medicine packages 451 in which the shifted-back defect is detected. On the other hand, the selection operation parts corresponding to "Only CHECK package", "Only NG package", and "CHECK, NG package" are each one example of the second re-execution operation part for receiving the operation for executing the dispensing operation about the non-proper wrapping material of which judgement results of the automatic judgement processing by the controller part 510 are not proper.

Here, as the reissuing operation screen D304 shown in Fig. 23, it is contemplated as another embodiment that "Only Shifted-back (before and after)", "Only Shifted-back (only before)", and "Only Shifted-back (only after)" are not displayed. In this case, it is contemplated that the controller part 21 selects the medicine package 451 which is determined that the shifted-back defect has occurred as the object for the reissue when the selection operation part corresponding to "Only CHECK package", "Only NG package", or "CHECK, NG package" for selecting the medicine package 451 (non-proper wrapping material) of which determination results of the automatic judgement processing are not proper is selected. That is to say, the selection operation parts corresponding to "Only CHECK package", "Only NG package", and "CHECK, NG package" may be the first reissuing operation part.

Furthermore, it is contemplated as another embodiment that the selection operation parts corresponding to "Only NG package", "NG package (medicine category)", "NG package (counting)", "NG package (medicine category and shifted-back)", and "NG package (medicine category and shifted-back)" are displayed on the reissuing operation screen D304. The selection operation part "Only NG package" is one example of the first re-execution operation part for receiving the operation for executing the package processing with respect to the medicine packages 451 of which judgment results of any one of the image judgement processing, the shape judgement processing and the counting judgement processing are error and the medicine packages 451 that the occurrence of the shifted-back defect has been detected. The selection operation part "NG package (medicine category)" is one example of the first re-execution operation part for receiving the operation for executing the package processing with respect to the medicine packages 451 of which judgment results of the image judgement processing or the shape judgement processing are error. The selection operation part "NG package (counting)" is one example of the first re-execution operation part for receiving the operation for executing the package processing with respect to the medicine packages 451 of which judgment results of the counting judgement processing are error. The selection operation part "NG package (medicine category and shift back)" is one example of the first re-execution operation part for receiving the operation for executing the package processing with respect to the medicine packages 451 of which judgment results of the image judgement processing or the shape judgement processing are error and the medicine packages 451 that the occurrence of the shifted-back defect has been detected. The selection operation part "NG package (counting and shift back)" is one example of the first re-execution operation part for receiving the operation for executing the package processing with respect to the medicine packages 451 of which judgment results of the counting judgement processing are error and the medicine packages 451 that the occurrence of the shifted-back defect has been detected.

In addition, in the reissuing operation screen D304, a reissuing key K315 is displayed for starting the execution of the reissue and the controller part 21 sends, in the state that the object for the reissue is selected by the operation of the selection operation part and also when the reissuing key K315 is operated, the control instruction including the reissuing data and the reissuing instruction for executing the package processing of the selected medicine package 451 to the medicine dispensing apparatus 4. Here, the relating processing is executed in the re-execution processing part 213. Thereby, in the medicine dispensing apparatus 4, according to the control instruction, the package processing is re-executed with respect to the administration timing corresponding to the medicine package 451 designated by the reissuing operation screen D304.

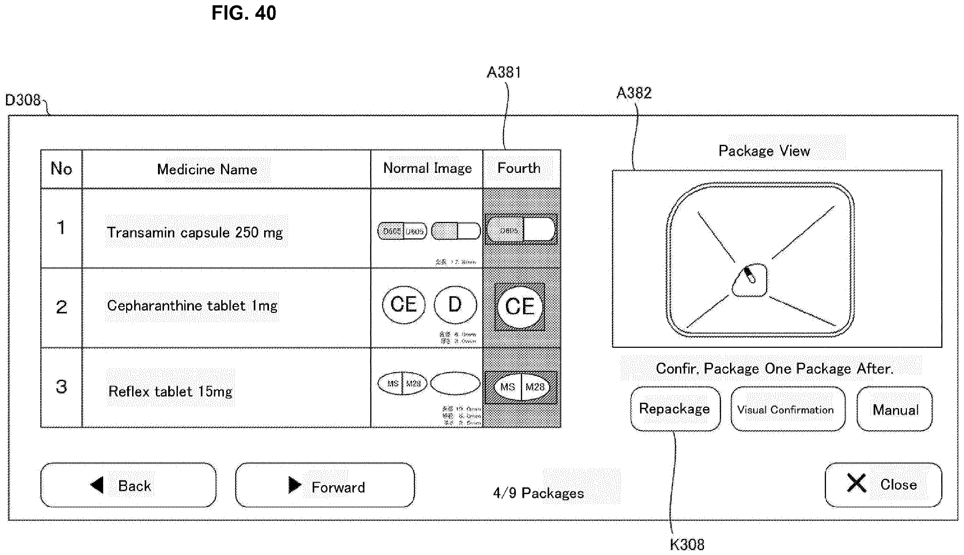

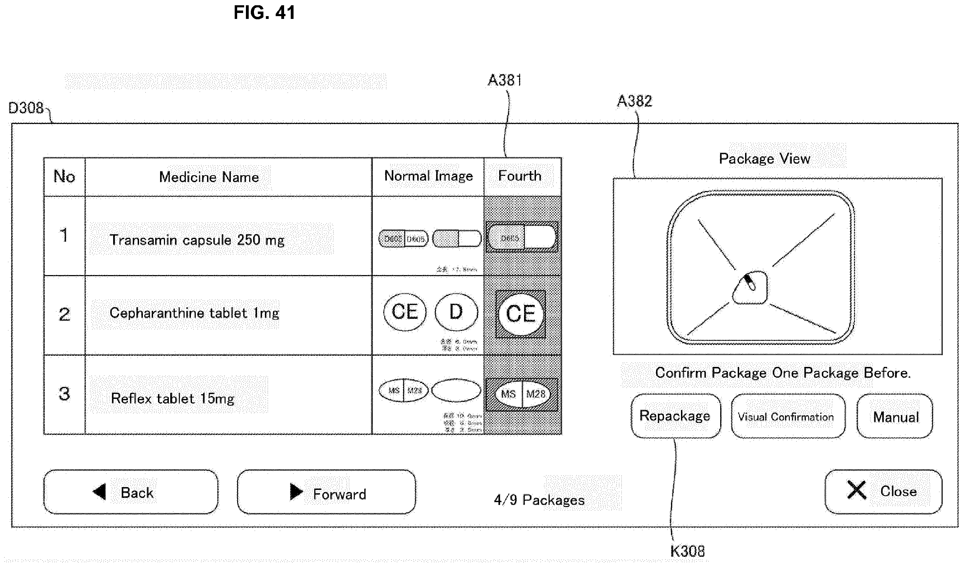

In addition, the controller part 21 displays, in the judgement detail screen D303, when the display region A320 corresponding to the third medicine package 451 or the fourth medicine package is selected, the medicine package individual information screen D308 for displaying the judgement results of the medicine package corresponding to the display region A320. Here, Fig. 40 is a drawing of one example of the medicine package individual information screen D308 when the display region A320 of the third medicine package 451 is selected and Fig. 41 is a drawing of one example of the medicine package individual information screen D308 when the display region A320 of the fourth medicine package 451 is selected. Besides, it is contemplated that the controller part 21 displays, when the selection of the display region A320 and also when the shifted-back defect occurs, the medicine package individual information screen D308 and when the shifted-back defect does not occur, displays a medicine package individual information screen D306 described later.

As shown in Fig. 41, in the medicine package individual information screen D308 displayed when the display region A320 corresponding to the fourth medicine package 451 is operated, for each of the tablets, medicine names, normal images, and photographed image of medicine package number (fourth) are displayed. Besides, the background part is displayed with the predetermined color acknowledging the occurrence of the shifted-back defect. Furthermore, in the medicine package individual information screen D308, a package view region A382 displaying the wrapping photograph image corresponding to the fourth medicine package 451 taken by the camera 476a of the detection part 476 and used in the determination for presence or absence of the shifted-back is displayed. Thereby, the user may confirm the occurrence of the shifted-back defect with referring to the package view region A382.

Besides, in the wrapping individual information screen D308, the information of the fourth medicine package 451 is displayed, however, when the shifted-back defect occurs, there is high probability that the tablet number is short due to the shifted-back defect with respect to the third medicine package 451 one package before the fourth medicine package 451. Thus, the controller part 21 displays, when displaying the wrapping individual information screen D308 corresponding to any one of two medicine packages 451 determined that the shifted-back defect occurs, a message on the wrapping individual information screen D308 that demands the confirmation for the other medicine package 451. Particularly, the controller part 21 displays, as shown in Fig. 41 in the wrapping individual information screen D308, corresponding to the fourth package, the message "also confirm one package before" and also displays, in the wrapping individual information screen D308 corresponding to the third package, display as shown in Fig. 40 the message "also confirm one package after".

Besides, in the medicine package individual information screen D308, an operation key 308 is displayed as the first re-execution operation part for receiving the operation for reperforming the package processing to at least one of the third medicine package 451 and the fourth medicine package 451 determined that the shifted-back defect occurs.

In addition, it is contemplated that the controller part 21 displays, when the operation key K308 is operated, likely to the operation key K312 (refer to Fig. 38), the reissuing operation screen D304 (refer to Fig. 39). That is to say, the user may perform the operation for reperforming the package processing about the medicine package 451 that the shifted-back defect occurs from the medicine package individual information screen D308. Besides, it is contemplated that the controller part 21 without displaying the reissuing operation screen D304 sends to the medicine dispensing apparatus 4 the control instruction including the re-execution data and re-execution instruction for reperforming the package processing to at least one of the third medicine package 451 and the fourth medicine package 451. Here, the relating processing is executed by the re-execution processing part 213 of the controller part 21

On the other hand, as shown in Fig. 40 in the medicine package individual information screen D308 displayed when the display region A320 corresponding to the third medicine package 451 is operated, for each of the tablets, medicine names, normal images, and a photographed image of medicine package number (third) are displayed. Besides, the background part is displayed with the predetermined color acknowledging the occurrence of the shifted-back defect. On the other hand, in the medicine package individual information screen D308, the wrapping photograph image corresponding to the fourth medicine package 451 taken by the camera 476a of the detection part 476 rather than the wrapping photograph image corresponding to the third medicine package 451 taken by the camera 476a of the detection part 476 is displayed in the package view region A382. That is to say, also in the medicine package individual information screens D308 corresponding to any one of the third medicine package 451 and the fourth medicine package 451, the wrapping photograph image corresponding to the fourth package may be displayed.

Here, in the present embodiment, when the tablet to be charged to the medicine package 451 is charged to the next medicine package 451, it is determined that the errors occur in both of the medicine packages 451. Contradictory to the above, it is contemplated as another embodiment that, for example, even when the tablet to be charged to the medicine package 451 will be wrapped to the other medicine package 451 after the subjected medicine package 451, and if the both of the medicine packages 451 are identified, it may be possible to determine that the errors occur in both of the medicine packages 451. For example, as the detection method of the shifted-back, it is contemplated that when the fact that the medicine package 451 is already charged with the tablet before charging the tablet to be charged to the medicine package 451 is detected by the detection part 476, the determination that the shifted-back defect occurs is made. On the other hand, it is contemplated that the construction that can determine whether or not the tablet number is short from the photographed image of the medicine package 451 formed with containing the tablets after the packaging. In this case, when the fact that the tablet is charged into the medicine package 451 before charging the tablet into the medicine package 451 is detected by the detection part 476 and also when the medicine package 451 being short in the tablet number before the subjected medicine package 451 is detected, it is contemplated that the errors occur in both of the medicine package 451. Thereby, the user may become possible to understand that the shortage of the tablet number of the medicine package 451 is caused by the shifted-back.

### [Wrapping view display function]

Now, in the shifted-back detection processing (refer to Fig. 35) executed for realizing the shifted-back detection function, the photographing is performed by the camera 476a when the heater parts 450k, 450k of the vertical sealing members 450e, 450e come to the timing just contacting with the dispensing paper S. Contradictory to this, it is contemplated that, separately to the start timing for the contact, the controller part 510 makes the camera 476a take the photograph when charging the tablets to the medicine package 451 formed in the packaging unit 504 through the medicine introduction part 80 from the rotation unit 44.

Here, it is contemplated that charging timing for charging the tablet is to be the detection timing of the tablet by the pass-through detection sensor 474. Besides, the charging timing is to be the timing set beforehand as the timing for charging of the tablet discharged from the medicine cassette 41 or the hand distribution unit 45 to the medicine introduction part 80 from the rotation unit 44. Thereby, in a photographed area of the camera 476a, the medicine introduction part 80, the tablet, and the medicine package 451 are included. Here, when the shifted-back defect does not occur, in the photographed image taken at the charging timing, the tablets charged to the medicine introduction part 80 or the medicine package 451 are photographed; however, since in the photographed image taken at the contact start timing, the tablets move toward the downstream side from the vertical sealing member 450e, the tablets is not photographed.

In addition, the controller part 510 stores the photographed image by the camera 476a in the storage part 22 and send it to the judgement supporting apparatus 2, Thereby, in the medicine dispensing apparatus 4 or the judgement supporting apparatus 2, the controller part 510 or the controller part 21 may realize the wrapping view function for displaying the photographed image.

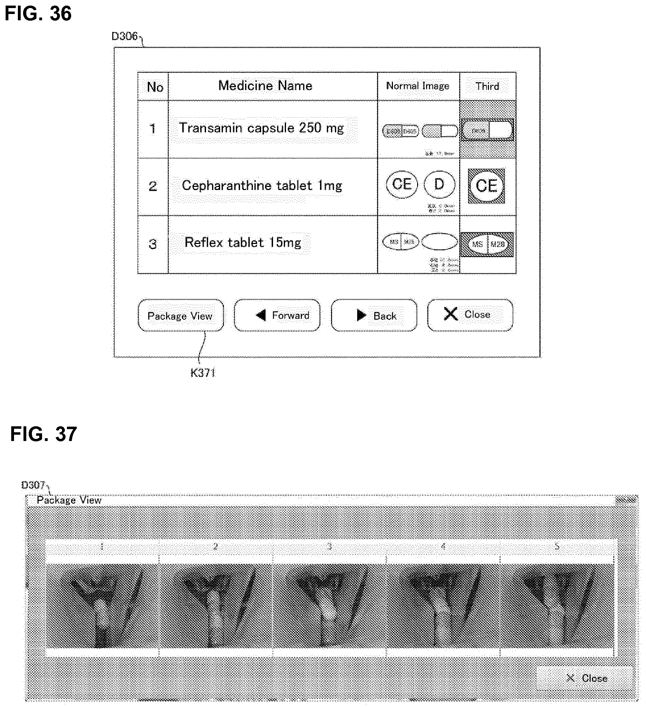

For example, in the judgement supporting apparatus 2, the controller part 21 displays on the judgement detail screens D301-D303 and the like, when the medicine identification information such as the medicine package number for identifying the medicine package 451 is selected, the medicine package information screen D306 for showing a list of the tablets contained in the medicine package 451. Here, Fig. 36 is a drawing of one example of the medicine package individual information screen D306.

As shown in Fig. 36, in the medicine individual information screen D306, medicine names, normal images, and photographed images and the like of each tablet contained in the third medicine package 451 in the package processing are displayed. Furthermore, in the medicine package individual information screen D306, with respect to the tablets of which judgement result of the automatic judgement is error among the tablets contained in the medicine package 451 now under displaying, the background of the display region for the photographed image is displayed in the sixth specific color such as red acknowledging that the result of the automatic judgement is error.

Furthermore, in the medicine package individual information screen D306, an operation key K371 is displayed and the controller part 21 may display, in response to the operation to the operation key K371, the wrapping screen D307 for displaying the photographed images of tablets contained in the medicine package 451 taken by the detection part.

Here, Fig. 37 is a drawing of one example of the wrapping screen D307. As shown in Fig. 37, in the wrapping screen D307, the photographed image of the tablets taken by the detection part 476 every time the tablets fall down toward the medicine package 451 from the rotation unit 44 through the medicine introduction part 80. As described earlier, in the photographed area photographed as the photographed image, the tablet introduction part 80, the tablet, and the medicine package are included. Thereby, the user, with referring to the wrapping screen D307, can understand easily the state of each tablet when falling down to the medicine package 451.

Now, the controller part 21 may display, instead of the photographed image taken at the charging timing, or together with the photographed image, the photographed image taken at the contact start timing in the wrapping view D307. Furthermore, it is contemplated that the controller part 21 displays on the wrapping view D307, when the occurrence of the shifted-back defect is detected by the shifted-back detection function, the message acknowledging the occurrence of the shifted-back defect and the photographed image taken at the starting timing for the contact display.

### [Print alone performing function]

As described earlier, in the medicine dispensing apparatus 4, a printer unit 48 is disposed to the packaging unit 504. In addition, to each medicine package 451 prior to the reception of the tablets in the medicine package 451 by the packaging unit 504, with using the printer unit 48, a part of the information such as the patient name and the administration timing and the like included in the formulation data may be printed. On the other hand, it may be the case that the construction is desired, which is able to output the medicine package 451 without containing the medicine therein and with the printing thereon a part of the information such as the patient name and the administration timing and the like included in the formulation data.

Thus, in the medicine dispensing apparatus 4, it is contemplated that the controller part 510 may be executable with switching the first printing mode in which when the package processing is performed the information is printed on the medicine package 451 by the printer unit 48 and the second printing mode in which without accompanied with the dispensation of the medicine by the tablet supply unit 502 while printing the information on the medicine package 451 by the printer unit 48 to form an empty medicine package 451 by the packaging unit 504. Particularly, the controller unit 510 comprises a selection processing part for selecting any one of the first printing mode and the second print mode depending on the user operation to the operation part 540 and may perform printing by the printer unit 48 while switching the printing mode to the first printing mode or to the second printing mode selected by the selection processing part. Furthermore, it is contemplated that the controller part 510 automatically select the first printing mode when the issuing operation of the formulation data for starting the package processing is performed. Now, the dispensing action by the tablet supply unit 502 comprises, for example, dispensing the tablet from the medicine cassette 41, supplying the tablet to the individual dispensing part 43 by opening the bottom face of DTA of the hand distribution unit 45, or supplying the tablet in each of the measure of the individual dispensing part 43 to the rotation unit 44 and the like. Particularly, the controller part 510 executes the processing according to the packaging control program for realizing the print individual performing function. In addition, the case that the medicine dispensing apparatus 4 is a tablet packaging apparatus for packaging the tablets will be explained as an example; however, for example, the similar explanation will be applied in the case that the medicine dispensing apparatus 4 is a powder packaging apparatus for packaging a powder drug.

Particularly, when the second printing mode is selected, the controller part 510 prints a part of the information of the formulation data or a part or all of the information input by the user operation on each of the medicine package 451 by the printer unit 48. For example, in the information to be printed on the medicine package 451, at least the administration timing (administration period) may be included. Thereby, continuous medicine packages 451 on which each administration timing is printed is output from the medicine dispensing apparatus 4 in the empty state. Here, the information printed on the medicine package 451 is not limited to the administration timing, and may be for example, any one or a plurality of administration timings, patient names, medicine names and the like.

For example, the controller part 510 makes the operation part 540 of the medicine dispensing apparatus 4, in response to the user operation for performing the print in the second printing mode, display a setting screen for setting the information to be printed on the medicine package 451 on the display part such as the monitor 530 and the like. Then, the controller part 510 receives input operations of the information using the operation part such as the operation button, the keyboard, the mouse and the touch panel disposed to the operation part 540 or the selection operation of the formulation data. Then, the operation part 510 makes, when a print start operation is performed by the user, the print unit 48 print the information received by the input operation or a part of the formulation data selected in the selection operation on the medicine package 451. In other words, it is contemplated that the medicine dispensing apparatus 4 comprises a reception operation part (controller part 510) being able to receive the input operation of the information to be printed on the medicine package 451 and the printer unit 48 prints the information received by the reception operation part on the medicine package 451.

Now, it is contemplated that the controller part 510, in the second printing mode, when receiving in response to the user operation the information to be printed on the medicine package 451 and when the print start operation being performed without input the information of the administration timing (administration period), acknowledges an error. For example, it is contemplated that the controller part 510 is able to set allowance or inhibition for omission of the printing of the administration timing in the second printing mode as an initial setting of the medicine dispensing apparatus 5. Furthermore, it is contemplated that the controller part 510 acknowledges the error using the monitor 530 and the like when the omission of printing of the administration timing is set to inhibition and when the print star operation is made without inputting the administration timing.

Furthermore, the controller part 510 may execute not only the package processing based on the formulation data but also may execute the package processing based on medicine names (medicine ID), dosages, usages and the like input by the user to the medicine dispensing apparatus 4. Here, when the first printing mode is executed, i.e., the package processing is executed, the input of the medicine identification such as the medicine name for identifying the medicine to be subjected to wrapping of the medicine package 451 is necessary; however, when the second printing mode is executed, printing of the medicine name is not necessary. Thus, it is contemplated that the controller part 510, in the case that the first printing mode is selected and when the the issuing operation of the formulation data for starting the package processing without inputting the medicine name (one example of the package processing starting operation), acknowledges the error using the monitor 530 and the like and does not start the dispensing operation. On the other hand, it is contemplated that the controller part 510, when the second printing mode is selected and even when the print starting operation is performed without inputting the medicine name, does not acknowledge the error and dispenses the empty medicine package 451 by the printer unit 48. Now, it is contemplated that the controller part 510 is able to set allowance or inhibition of the omission of the printing of the medicine name in the second printing mode. In this case, the controller part 510, in the second printing mode, when the print starting operation is performed without inputting the medicine name and when the omission of the printing of the medicine name is set to be inhibition, acknowledges the error by using the monitor 530 and the like and when the omission of the printing of the medicine name is set to be allowance, does not acknowledge the error.

Here, in the packaging unit 504, considering time when the tablets are charged to the medicine package 451, a supply motion of the sheet by the dispensing paper supply part 450A is intermittently performed. Thus, in the first printing mode, the printing motion also performed intermittently. Contradictory to this, in the second printing mode the tablet is not charged into the medicine package 451 such that it is contemplated that the controller part 510 in the packaging unit 504, sets a speed of the supply motion with the dispensing paper supply part 450A higher than that of the supply motion in the first mode. More particularly, with respect to the intermittent supply of the sheet in the first printing mode and the second printing mode, it is contemplated that termination time of the feeding motion of the sheet is set shorter in the second printing mode than that of the first printing mode.

Now, for example, the sealing apparatus 450F has a construction being disposed with a pair of planer heaters having a plane shape such as T -shape and being able to bond the dispensing paper S with sandwiching thereof between the heaters each other, because the time for bonding will be required and then the feeding motion by the dispensing paper supply part 450A is required to perform intermittently. However, in the medicine dispensing apparatus 4, the sealing apparatus 450F may bond the dispensing paper S with sandwiching by the roller shaped members formed from the vertical sealing members 450e, 450e and the lateral sealing members 450f, 450f. Thus, in the medicine dispensing apparatus 4, it is contemplated that the controller 510 in the second printing mode perform the feeding motion of the sheet by the dispensing paper supply part 450A and the printing motion by the printer unit 48 continuously. Thereby, in the medicine dispensing apparatus 4, since the printing to the medicine package 451 may be performed quickly, required time may be shortened when compared to the case that, for example, printing to the medicine package 451 is realized by performing the package processing in the state without the formulation medicine.

### [Erroneous charging protection function]

Incidentally, in the medicine dispensing apparatus 4, when a pharmacist charges the tablets to the medicine cassette 41 such as the variable cassette 41 B or the fixed cassette 41A, there are fears that the tablets are erroneously charged into the medicine cassette 41 different from the medicine cassette 41 associated beforehand to the tablet by human errors. Contradictory to the above, it is contemplated that the medicine dispensing apparatus 4 comprises a function for protecting the erroneous charging to the medicine cassette 41. Now, the case that the medicine dispensing apparatus 4 is a tablet packaging apparatus for packaging the tablets will be explained as an example, however, for example, the similar explanation will be applied in the case that the medicine dispensing apparatus 4 is a powder packaging apparatus for packaging the powder or in the case that a liquid agent distributing apparatus for packaging the liquid agent.

Particularly, in the medicine dispensing apparatus 4, a mount/unmount detection part for detecting mount/unmount of each medicine cassette 41 such as an optical sensor or a mechanical sensor and the like is disposed. In addition, the controller part 510 may detect the mount/unmount of the medicine cassette 41 depending on the mount/unmount detection part.

Furthermore, the controller of the medicine dispensing apparatus 4, by executing the processing according to the packaging control program functions as a detection processing part 511, a specification part 512, a determination part 513, and a report processing part 514.

The detection processing part 511 may detect removal of each medicine cassette 41. The specification part 512 specifies, when the information of the subjected medicine to be replenished to the medicine cassette 41 is input, the medicine cassette 41 corresponding to the subjected medicine. The determination processing part 513 determines, when the information of the subjected medicine is input and then removal of the medicine cassette 41 is detected by the detection processing part 511, whether or not the removed medicine cassette 41 and the medicine cassette 41 specified by the specification part 512 are identical each other. The report processing part 514 may report a determination result by the determination processing part 514.

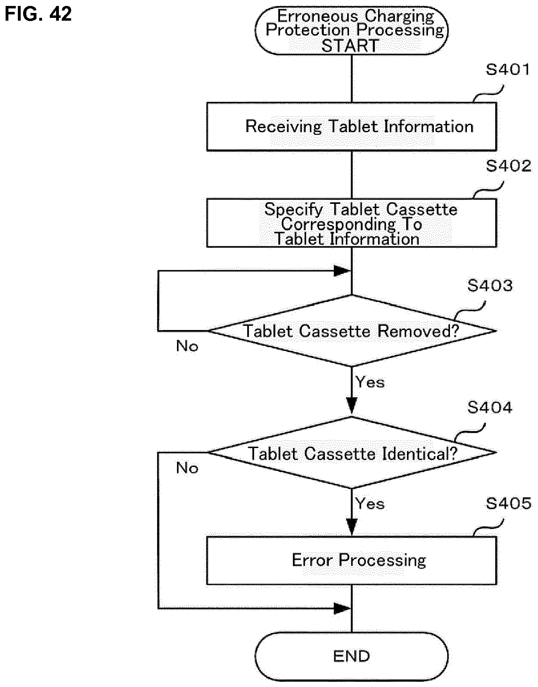

Here, Fig. 42 is a flowchart for showing one example of the erroneous charging protection processing executed by the controller part 510. The erroneous charging protection processing may be executed when a predetermined replenishment start operation is done to the medicine dispensing apparatus 4. Now, it is contemplated that the replenishment start operation is done not only when the tablets are replenished to the medicine cassette 41 but also when excess residual tablets charged to the variable cassette 41B after their usage are returned to the fixed cassette 41 A. Furthermore, the erroneous charging protection processing may be executed when the tablet is allocated to the variable cassette 41B.

### <Step S401>

First in the step S401, the controller part 510 executes processing for receiving input of the information of the tablets charged to the medicine cassette 41. For example, as the information of the tablets, names of the tablets, letters stamped or printed on the tablets, shapes of the tablets, or colors of the tablets may be included. Furthermore, the controller part 510 may display an operation screen such as a list screen of the tablets or a search screen of the tablets and the like on the monitor 530 and may receive the input of the information of the tablets according to selection operations onto the operation screen. Now, the controller part 510 may receive, when the code information such as the barcode or the two-dimensional code provided to a container box or a container bottle containing the tablets and the like are read, the input of the information of the tablets from the code information. Furthermore, when the tablets are allocated to the variable cassette 41B, it may be possible to specify the tablets as the information of the tablets to be charged in the medicine cassette 41.

### <Step S402>

In the step S402, the controller part 510 specifies, depending on the information of the tablets received in the step S401, the medicine cassette 41 corresponding to the tablets. Here, relating processing is executed by the specification processing part 512 of the controller 510. Particularly, the controller part 510 may displays candidates of the tablets corresponding to the information of the tablet received in the step S401 and when a user operation for selecting the specific tablet from the candidates of the tablets is performed, the controller part 501 specifies the medicine cassette 41 corresponding to the selected tablet. Now, the specification of the medicine cassette 41 corresponding to the selected tablet may be, for example, done based on the cassette master.

### <Step S403>

In the step S403, the controller part 510 determines whether or not the removal of any one of the medicine cassettes 41 is detected by the mount/unmount detection part. Here, the relating processing is executed by the detection processing part 512 of the controller part 510. In addition, when the removal of the medicine cassette 41 is detected (S403: Yes), the processing is passed to the step S404 and when the removal of the medicine cassette 41 is not detected (S403: No), the processing is waited in the step S403.

### <Step S404>

In the step S404, the controller part 510 determines whether or not the medicine cassette 41 specified in the step S402 and the medicine cassette 41 detected the removal thereof in the step S403 are identical each other. Then, when the medicine cassettes 41 are identical each other (S404: Yes), the erroneous charging protection processing is terminated and when the medicine cassettes 41 are not identical each other (S404: Yes), the processing is passed to the step S405.

Now, in the medicine dispensing apparatus 4, it is contemplated that the tablets may be replenished parallel to a plurality of the medicine cassettes 41. In this case, the information of a plurality of the tablets are input in the step S401, and a plurality of the medicine cassettes may be specified in the step S402. Then, it is contemplated that the controller part 501 in the step S404 determines whether or not the medicine cassette 41 detected the removal thereof corresponds to any one of a plurality of the cassettes 41 specified in the step S402.

### <Step S405>

In the step S405, the controller part 510 executes an error processing for acknowledging to the user the fact that the medicine cassette 41 is erroneously removed. Now, in the error processing, for example, a predetermined message for acknowledging occasion of the error is displayed on the monitor 530 and the acknowledgement is stored in the storage part 520. Thereby, the user can aware the error of the medicine cassette 41 so that the erroneous charging to the medicine cassette 41 is protected. Now, in the error processing, the error may be acknowledged by error sounds or an error indication lamp.

As described above, in the medicine dispensing apparatus 4, when the medicine cassette 41 different from the medicine cassette 41 to be subjected to the charging of the tablets is removed, the acknowledgement is reported, and hence the erroneous charging of the tablets by the user may be protected.

### [Other example of erroneous charging protection processing]

Incidentally, in the erroneous charging protection processing, when the medicine cassette 41 is removed, the case, in which the propriety of the medicine cassette 41 is determined, will be described. On the other hand, as for the measure addressing to the protection of the erroneous charging to the medicine cassette 41 from another point of view, it is contemplated as another example that the protection of the removal of the erroneous medicine cassette 41 is protected.

Particularly, in the medicine dispensing apparatus 4, as shown in Fig. 3, a cassette lock part 410 arranged corresponding to each of the medicine cassette 41 and being able to lock the removal of the medicine cassette 41 is disposed. The cassette lock part 410 may, for example, include a driver part such as a solenoid or a motor and the like and a limiter part for limiting the removal of the medicine cassette 41 by driving the driver part. In addition, the controller part 510 may control presence or absence of the lock for each of the medicine cassettes 41 individually by controlling the driver part of the cassette lock part 410.

Furthermore, the controller part 510 of the medicine dispensing apparatus 4, by executing the processing according to the packaging control program, functions as the lock processing part 515 by performing the erroneous charging protection processing for realizing the erroneous charging protection function. The lock processing part 515 locks, when the medicine cassette 41 is mounted, the removal of the medicine cassette 41 by the cassette lock part 410 and releases the lock by the cassette lock part 410 with respect to the medicine cassette 41 specified by the specification processing part 512. Now, the lock processing part 515, when the mounting of the medicine cassette 41 is detected by the mount/unmount detection part, controls the cassette lock part 410 to lock the removal of the medicine cassette 41.

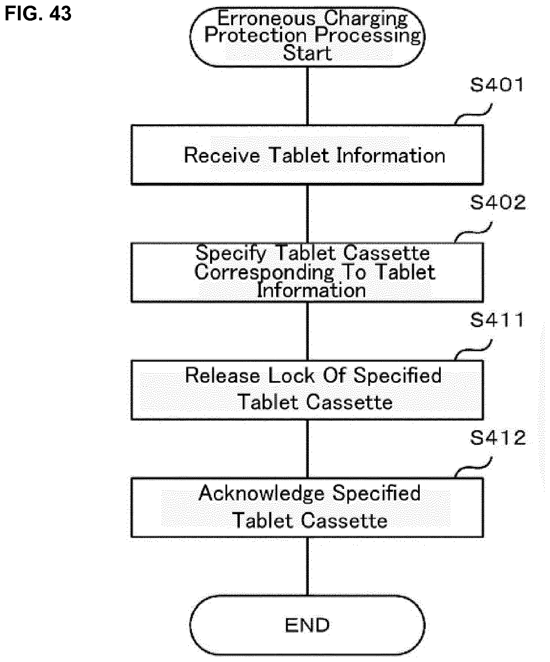

Here, Fig. 43 is a flowchart showing another example of the erroneous charging protection processing executed by the controller part 510. Particularly, in the erroneous charging protection part, instead of the steps 403-404, the steps S411-S412 are executed. Now, it is contemplated as another embodiment that the controller part 510 executes the step S411-S412 between the step S402 and the step S403.

### <Step S411>

As shown in Fig. 43, after the execution of the steps S401-S402, in the step S411, the controller part 510 release the lock for the removal only for the cassette lock part 410 corresponding to the medicine cassette 41 specified in the step S402. Thereby, in the medicine dispensing apparatus 4, only the cassette 41 specified in the step S402 becomes removable and the removal for the other medicine cassette 41 are kept locked.

Now, in the step S410, it is contemplated that information for a plurality of tablets is input sequentially and when this is the case, in the step S411, removal locks for each of the medicine cassettes 41 corresponding to a plurality of the tablets are released.

### <Step S412>

In the step S412, the controller part 510 acknowledges to the user the medicine cassette 41 unlocked in the step S411. For example, the controller part 510 makes the monitor 510 display the identification information such as the cassette number or the position and the like for identifying the medicine cassette 41. In addition, when a light source such as LED and the like is disposed corresponding to each of the medicine cassette 41, it is contemplated that the controller part 510 makes the light source turn on or flash corresponding to the medicine cassette of which lock is released.

As described so far, in the medicine dispensing apparatus 4, only the medicine cassette 41 subjected to charging of the tablets becomes removable such that the erroneous charging by the user may be protected.

### [Removal support function]

Incidentally, when the medicine information has been allocated to the variable cassette 41B, thereafter, the user will charge the tablets with drawing out the variable cassette 41B. With respect to the above, to the medicine dispensing apparatus 4, it is contemplated that a driving mechanism is disposed for moving the variable cassette 41B for a predetermined amount to the drawing out direction. For example, the driving mechanism comprises a driving source such as a solenoid or a motor and the like and an urging mechanism for urging the variable cassette 41B to the drawing out direction by driven with the driving source. Furthermore, the controller part 510 makes, when the medicine information has been allocated to the variable cassette 41B, the driving mechanism control to move the variable cassette 41B to the drawing out direction for the predetermined amount. Particularly, as described above, under the condition that the removal of the variable cassette 41B is locked and the medicine information is allocated to the variable cassette 41B, in synchronous to the release of the lock for the removal about the variable cassette 41B, the variable cassette 41b is moved to the drawing out direction for the predetermined amount. Thereby, it may become easy for user to do work for removing the variable cassette 41B into which the tablets are charged. In addition, it may become easy to identify the variable cassette 41B into which the tablets should be charged.

### [Automatic setting function]

In the medicine dispensing apparatus 4, an appearance shape of the tablet such as the height and the width corresponding to every tablet is stored in the medicament master and in the variable cassette 41B, the height regulating member 705 and the width regulating member 706 are adjusted in response to the height and the width of the tablet allocated to the variable cassette 41B. Thus, the tablet being not registered in the medicament master cannot be dispensed from the variable cassette 41B for every unit amount. Then, the controller part 510, when one or both of the height and the width of the tablet allocated to the variable cassette 41B is not registered in the medicament master, dispenses the tablet for every unit amount by executing the following processing. In addition, to the tablet packaging apparatus 4, a first tablet detection part is disposed for detecting the tablet passed through the height regulating member 705 in the second rotor 703. Now, the tablet detection part is an optical sensor and the like disposed at the position detectable the tablet between the height regulating member 705 and the width regulating member 706. Furthermore, as described above, to the dispensing port 704 of the variable cassette 41B, a second tablet detection part is disposed for detecting the passed through tablet.

First, the controller part 510 regulates the height regulating part 705A and the width regulating part 706A to adjust the height h1 to be regulated by the height regulating member 705 and the width w1 to be regulated by the width regulating member 706 to the predetermined minimum value. Then, the controller part 510 first drives the first rotor 702 and the second rotor 703 of the variable cassette 41B and drives the height regulating member 705 such that the height h1 with regulating by the height regulating member 705 becomes gradually higher. Then, when the tablet is detected by the first tablet detection part because the height h1 becomes higher than the height of the tablet, the controller 510 stops to drive the height regulating member 705. Next, the controller 510 gradually drives the width regulating member 706 such that the width w1 with regulating by the width regulating member 706 becomes wider gradually. Then, when the tablet is detected by the second tablet detection part because the width h1 becomes wider than the width of the tablet, the controller 510 stops to drive the width regulating member 706. Thereby, thereafter, the tablet may be dispensed for every unit amount.

### DESCRIPTION OF SIGNS

- 1:: judgement supporting system
- 2:: judgement supporting apparatus
- 3:: client peripheral
- 4:: prescription device
- 5:: host system

## Claims

1. A judgment supporting system comprising:
a packaging unit performing a package processing for packaging one or a plurality of tablets in a wrapping material for every administration timing based on formulation data; and
a shifted-back detection part for determining occasion of a shifted-back defect in a case that at least a part of the tablets to be charged in a first wrapping material in the package processing is wrapped in a second wrapping material being next to the first wrapping material.

2. The judgement supporting system of claim 1 further comprising:
an operation display processing part displaying a first re-execution operation part for receiving an operation to re-execute the package processing with respect to at least one of the first wrapping material and the second wrapping material determined that the shifted-back detect has been occurred by the shifted-back detection part; and
a re-execution part executing the package processing with respect to at least one of the first wrapping material and the second wrapping material when the first re-execution operation part is operated.

3. The judgement supporting system of claim 1, wherein the first re-execution part receives an operation for executing the package processing with respect to the first wrapping material and the second wrapping material determined that the shifted-back detect has been occurred by the shifted-back detection part; and
the re-execution processing part executes the package processing with respect to the first wrapping material and the second wrapping material when the re-execution operation part is operated.

4. The judgement supporting system of claims 2 or 3, wherein the judgement supporting system further comprises a judgement processing part for determining propriety of a result of the package processing based on the formulation data;
the operation display processing part displays the first re-execution operation part and a second re-execution operation part receiving an operation for executing the package processing with respect to a non-proper wrapping material of which result by the judgement processing part is not proper; and
the re-execution processing part executes the package processing with respect to the first wrapping material and the second wrapping material when the first re-execution operation part is operated and executes the package processing with respect to the non-proper wrapping material when the second operation part is operated.

5. The judgement supporting system of claim 4, wherein;
the judgement processing part may execute at least one of an image judgement processing for determining propriety of a result of the package processing based on identification information of the tablet included in a photographed image photographing the tablet; a shape judgement processing for determining propriety of a result of the package processing based on an appearance of the tablet included in a photographed image photographing the tablet; and a counting judgement processing or determining propriety of a result of the package processing based on packaging amounts of the tablets, and
the operation display processing part displays one or a plurality of the second re-execution operation part for receiving the operation to execute the package processing with respect to the non-proper wrapping material of which determination result in any one of the image judgement processing, the shape judgement processing and the counting judgement processing is not proper.

6. A medicine dispensing apparatus comprising:
a medicine supply unit for dispensing one or a plurality of medicines based on formulation data;
a packaging unit for performing a package processing to package the tablet dispensed by the medicine supply unit for every administration timing into a wrapping material;
a printer unit for printing information on the wrapping material; and
a controller part being able to execute with switching between a first printing mode and a second printing mode, the first printing mode printing the information on the wrapping material by the printer unit upon executing the package processing and the second printing mode printing the information on the wrapping material by the printer unit without accompanied with the dispensation of the medicine by the medicine supply unit to form the wrapping material in an empty state.

7. The medicine dispensing apparatus of claim 6, wherein the controller part, when the first printing mode is selected and also when a start operation for packaging is done without input of medicine designation information for identifying a medicine acknowledges an error, and the controller part when the second printing mode is selected and even when a start operation for packaging is done without input of medicine designation information of identifying a medicine, does not acknowledge an error.

8. A medicine dispensing apparatus comprising:
a plurality of medicine cassette being able to dispense a predetermined medicine for every unit amount;
a detection processing part being able to detect removal of each of the medicine cassettes;
a specification processing part for specifying the medicine cassette corresponding to the subjected medicine when inputted information of a subjected medicine to be replenished to the medicine cassette;
a determination processing part for determining whether or not the removed medicine cassette and the medicine cassette specified with the specification processing part is identical each other after inputting information of the subjected medicine and when detecting the removal of the medicine cassette by the detection processing part; and
a report processing part for acknowledging a determination result by the determination processing part.

9. A medicine dispensing apparatus comprising:
a plurality of medicine cassettes being able to dispense a predetermined medicine for every unit amount;
a cassette lock part being able to lock removal of each of the medicine cassettes;
a specification processing part for specifying the medicine cassette corresponding to the subjected medicine when inputted information of a subjected medicine to be replenished to the medicine cassette; and
a lock processing part for locking the removal of the medicine cassette by the cassette lock part upon mounting the medicine cassette and for releasing the lock by the cassette lock part with respect to the medicine cassette specified by the specification processing part.
